# EUROPEAN PATENT APPLICATION

(11) **EP 2 594 280 A1**
(43) Date of publication of application: **22.05.2013**
(21) Application number: 12007751.6
(22) Date of filing: 16.11.2012
(51) Int. Cl.: A61K 38/17, A61P 29/00, A61P 37/00, A61P 11/00, A61K 45/06, A61K 39/395

(54) **Activin neutralisers and uses thereof for treatment of diseases associated with aberrant "host defence response" activation**

(30) Priority: 21.11.2011 GR 20110100649
(71) Applicant: Biomedical Research Foundation of the Academy of Athens, 115 27 Athens (GR); Sideras, Paschalis, 15126 Marousi (GR); Apostolou, Eirini, 15561 Cholargos (GR); Stavropoulos, Athanasios, 15562 Cholargos (GR); Sountoulidis, Alexandros, 56626 Sikies Thessaloniki (GR); Xirakia, Charoula, 35100 Lamia (GR); Protopapadakis, Evdokia, 15562 Cholargos (GR); Giaglis, Stavros, 4057 Basel (CH); Ritvos, Olli, 00120 Helsinki (FI); Andreakos, Evangelos, 15451 Athens (GR)
(72) Inventor: Sideras, Paschalis, 15126 Marousi (GR); Apostolou, Eirini, 15561 Cholargos (GR); Stavropoulos, Athanasios, 15562 Cholargos (GR); Sountoulidis, Alexandros, 56626 Sikies Thessaloniki (GR); Xirakia, Charoula, 35100 Lamia (GR); Protopapadakis, Evdokia, 15562 Cholargos (GR); Giaglis, Stavros, CH-4057 Basel (CH); Ritvos, Olli, 00120 Helsinki (FI); Andreakos, Evangelos, 15451 Athens (GR)
(74) Representative: Lyberis, Nikolaos

(57) **Abstract**

The present invention is concerned with methods and compositions for use in the medical art and relates to the use of an activin neutraliser or a mixture thereof for the manufacture of a medicament for the prophylaxis and / or therapy of diseases associated with aberrant "Host Defence Response" activation in a subject. The present invention provides activin neutralisers in the form of soluble ActRII polypeptides, or a fragment(s) or analogue thereof; follistatin, follistatin-like 3 protein, or a fragment(s) or analogue thereof; low molecular weight organic compounds; antibodies raised against either activin-A, activin-B, or activin receptors or immunogenic portions thereof; and antisense nucleic acids, ribozymes, triplex agents, siRNAs and miRNAs. Also provided are pharmaceutical compositions, comprising a therapeutically effective or prophylactically effective amount of at least one activin neutraliser in admixture with pharmaceutically acceptable carriers, adjuvants and / or diluents. Also provided are methods for the prophylaxis and / or therapy of diseases (including a combination of therapies) associated with said aberrant Host Defence Response activation. Diseases that may be treated according to the method of the present invention include ALI/ARDS, sepsis and COPD.

## Description

### TECHNICAL FIELD OF THE INVENTION

The technical field of the present invention relates to the use of an activin neutraliser or a mixture thereof for the manufacture of a medicament for the prophylaxis and / or therapy of diseases associated with aberrant "Host Defence Response" activation in a subject. Said diseases include ALI/ARDS, sepsis and COPD. The present invention also contemplates methods for a combination of therapies for the prophylaxis and / or therapy of diseases associated with aberrant HDR activation in a subject. The activin neutraliser composition may be co-administered with at least one additional therapeutic agent which may facilitate the desired prophylactic or therapeutic outcome.

### BACKGROUND OF THE INVENTION

Bibliographic details of the publications referred to by author in this specification are collected alphabetically at the end of the description.

### Tissue host defence response (HDR) and development of self-inflicted pathology

Disturbance of the homeostatic balance either due to infection, injury or malfunction stereotypically activates a network of interactive biological processes that act in synergy to ensure restoration of homeostasis and secure the subject's chances of survival (Medzhitov and Janeway 2002; Medzhitov 2009; Iwasaki and Medzhitov 2010). Among the processes that comprise this stereotypic activation, of key importance, are: (a) the activation of the coagulation and complement cascades; (b) the activation of the innate and adaptive immunity; and (c) the activation of the tissue repair process.

Although the host defence response ("HDR") is a protective response; deviation from its normal function may turn otherwise protective processes into self-damaging ones and contribute greatly to the pathophysiology of several human diseases, examples of which are sepsis, acute respiratory distress syndrome (ARDS), ischaemia-reperfusion injury, gout, pseudo-gout, Alzheimer's disease, atherosclerosis, rheumatoid arthritis, colitis, vascular and abdominal surgery, trauma, burns and others (Meduri 1996).

Thus, whereas activation of the coagulation system is a key protective mechanism, its aberrant over-activation can lead to disseminated intra-vascular thrombosis (a process known as hyper-coagulability). Furthermore, over-activation can lead to consumption of coagulation factors and platelet dysfunction (a process known as hypo-coagulability).

Production of pro-inflammatory mediators initially, and anti-inflammatory mediators subsequently, allow the system to mount a protective inflammatory response and return to baseline once the initial infectious or toxic stimulant is contained. Exaggerated pro-inflammatory responses could lead to serious collateral tissue damage whereas exaggerated anti-inflammatory response could lead to total paralysis of the immune system leaving the host unprotected and thus exposed to any opportunistic infection. Similarly, appropriate activation of the tissue repair processes can remediate tissue integrity and restore homeostasis; however, exaggerated and/or inappropriate activation can cause excessive deposition of extracellular matrix components such a collagen and thus lead to development of fibrosis (Wynn 2007).

The mobilisation of similar processes during infection, tissue injury or tissue malfunction suggests that both infectious and sterile stimuli may function through common receptors and pathways; however, it wasn't until the description of "pathogen-" and "danger-" associated molecular patterns, and their receptors, that this behaviour of the host defence system could be explained mechanistically. Recognition of "pathogen-associated molecular patterns" (PAMPs) or "microbe-associated molecular patterns" (MAMPs) by pattern-recognition receptors (PRRs) present on APCs explained the capacity of the host to discriminate between "infectious non-self" and "non-infectious self" (Medzhitov and Janeway 2002; Medzhitov 2009; Iwasaki and Medzhitov 2010) and recognition of highly pro-inflammatory "danger-associated molecular patterns" (DAMPs) release in the microenvironment by dying or stressed cells, even under microbe-free conditions, by PRRs and other specialised receptors explained the capacity of the host to sense "danger" and mobilise the "Host Defence Response" accordingly.

### Pathogen-Associated Molecular Patterns (PAMPs)

Pathogen-associated molecular patterns (PAMPs) represent molecular components that are invariably present on groups of pathogens or microbes (entire molecules or, more often, part of molecules or polymeric assemblages) and are distinct from those found in the host. These conserved microbial components, largely carbohydrates and glycolipids in nature, are needed for their own survival.

Pattern recognition receptors (PRR) are germ-line encoded surface receptors that recognise PAMPs derived from microorganisms. Typical PAMPs include bacterial endotoxin (lipopolysaccharides, LPSs), peptidoglycan, microbial unmethylated CpG-DNA and viral double-stranded RNA (Hemmi, Takeuchi et al. 2000; Krieg 2002).

Five classes of PRRs have been identified to date: Toll-like receptors (TLRs); NOD-like receptors (NLRs); RIGI like receptors (RLRs); C-type lectin receptors (CLRs) and absence in melanoma 2 (AIM2) like receptors 8. Following ligand recognition these receptors activate downstream signalling pathways, such as the NFκβ, MAPK and type-I interferon pathways, which result in the up-regulation of pro-inflammatory cytokines and chemokines that are important in inflammatory and antimicrobial responses.

### Danger-Associated Molecular Patterns (DAMPs)

Danger-associated molecular patterns (DAMPs) are endogenous molecules that under normal physiological conditions are sequestered within the cells, thus hidden from recognition by the immune system (Matzinger 2002; Seong and Matzinger 2004).Under conditions of infection, cellular stress or injury these molecules are released in the extra-cellular environment by dying or stressed cells and trigger inflammation. Irrespective of whether or not there are signs of infection in a tissue DAMPs in the extra-cellular spaces are generic signals of potential danger to the host (Gordon 2002; Medzhitov and Janeway 2002; Beutler 2007; Bianchi and Manfredi 2007).

Many inflammatory and autoimmune diseases are characterised by extremely destructive tissue environments and thus in many of these conditions high levels of DAMPs are found locally and / or systemically sustaining a "vicious cycle" of injury, inflammation and repair. Prototypical DAMPs derived from necrotic or stressed cells include the chromatin-associated protein high-mobility group box-1 (HMGB1), heat shock proteins (HSPs) (Quintana and Cohen 2005) and purine metabolites, such as ATP (Bours, Swennen et al. 2006) and uric acid (Kono, Chen et al. 2010).

Increased levels of DAMPs have been associated with several human diseases. Thus, extranuclear expression of HMGB1 has been detected in sepsis (Yang, Ochani et al. 2004), arthritis (Kokkola, Li et al. 2003; Taniguchi, Kawahara et al. 2003), systemic lupus erythematosus (SLE) (Popovic, Ek et al. 2005), cancer (Taguchi, Blood et al. 2000), hepatitis (Sitia, Iannacone et al. 2007) and COPD (Ashitani, Mukae et al. 2002; Wouters, Groenewegen et al. 2007; Ferhani, Letuve et al. 2010; Borensztajn, von der Thusen et al. 2011; Kanazawa, Tochino et al. 2011).

Although it is apparent that PAMPs and DAMPs act in quite a different manner in order to stimulate an immune response, the existence of a dynamic cross-talk between the DAMP and PAMP activated processes has been recognised thus explaining the stereotypic nature of the host defence response. Pattern recognition receptors such as TLR2, TLR4 and TLR9 that recognise microbial products are also activated by HMGB1, the prototypical DAMP molecule (Park, Svetkauskaite et al. 2004; Kokkola, Andersson et al. 2005; Park, Gamboni-Robertson et al. 2006).

### Cell death and release of DAMPs

Although cell death invariably marks the course of many diseases, in immuno-inflammatory disorders such as infectious, autoimmunity and inflammatory syndromes, cell death is not simply an outcome but, rather, an integral part of their pathophysiology (Kono and Rock 2008; Chen and Nunez 2010).

Cell death is traditionally dichotomised into two forms: apoptosis and necrosis (although numerous other variations of cell death have been described such as pyroptosis, autophagy, aponecrosis and others).

Apoptosis corresponds to a form of cell death during which a programmed sequence of events leads to the cell elimination without releasing harmful substances into the surrounding area. This death process is therefore self-contained and, thus, cells dying by apoptosis are either devoid of immunological activity or may actually exert immunosuppressive properties (Morimoto, Janssen et al. 2006; Thorp and Tabas 2009).

Necrosis, on the other hand, usually occurs under conditions of extreme stress (such as toxin- or ischemia-induced injury) when removal of a cell through programmed death by apoptosis fails to occur. An important consequence of necrotic cell death is the escape of intracellular material from the cell due to rupture of the plasma membrane. In contrast to the silent death of apoptosis; cell death by necrosis appears to be immunologically very dangerous or alarming due to the release of numerous DAMPs in the micro-environment.

Thus, whereas apoptosis can be physiological, necrosis is essentially always pathological (Kalai, Van Loo et al. 2002; Silva, do Vale et al. 2008; Kroemer, Galluzzi et al. 2009; Antoine, Williams et al. 2010). However, it is worth noting that apoptosis can become inflammatory under conditions in which there is delayed clearance of apoptotic cells, as can occur with high levels of apoptosis, resulting in secondary necrosis (also known as aponecrosis or necroptosis) with loss of plasma membrane integrity (Silva, do Vale et al. 2008).

### Sepsis and Acute Respiratory Distress Syndrome (ARDS)

Inflammation is a self-limiting and protective response of the body in which cytokines production and leucocytes activation unfold properly and eliminate infectious agents, neutralise toxic substances or remediate tissue injury. However, dys-regulated inflammatory responses can lead to a hyper-inflammatory state and contribute to the development of severe pathology (Bone 1996; Bone 1996; Kox, Volk et al. 2000; Rice and Bernard 2005; Russell 2006). Sepsis and the Acute Respiratory Distress Syndrome (ARDS) are classical examples of severe diseases caused by typical aberrant HDR activation.

Sepsis is a systemic inflammatory response syndrome resulting from a microbial infection. Its pathogenesis is rather complex, but is partly attributable to dys-regulated inflammatory response. Severe sepsis is associated with organ dysfunction (i.e. hypoperfusion, tissue hypoxia, lung injury, etc.), while septic shock is a type of severe sepsis characterised by hypotension, despite fluid resuscitation (Rice and Bernard 2005; Russell 2006).

ARDS is a fulminant respiratory condition in which injury of the lung epithelium and endothelium triggers an uncontrolled inflammatory response and leads to often severe functional and structural deterioration of the lung (Tsushima, King et al. 2009). It usually requires mechanical ventilation and admission to an intensive care unit and is often fatal. A less severe form is termed "acute lung injury" (ALI) (Tsushima, King et al. 2009). ARDS is caused by a variety of pulmonary or extra-pulmonary factors. These include pneumonia, cryptogenic organising pneumonia, acute fibrinous organising pneumonia and diffuse alveolar damage (DAD) (Tsushima, King et al. 2009).

Although ALI/ARDS and sepsis are distinct disease entities there is an intimate interrelation among them (Meduri 1996; Khadaroo and Marshall 2002; Costa, Schettino et al. 2006; Tsushima, King et al. 2009). They both represent typical examples of severe pathology, eventually, associated with deregulated immune reactivity. A wide variety of precipitating causes are recognised for ARDS including pneumonia and aspiration of gastric contents; however, severe sepsis is the leading cause of ARDS (Bernard, Artigas et al. 1994; Tsushima, King et al. 2009). Conversely, a very common complication in sepsis is the development of ARDS. This "cause-effect" relationship could stem from the mobilisation of similar, potentially harmful, effector mechanisms during their development.

Originally, attention was focus primarily on the inflammatory component of ALI/ARDS and sepsis. However, anti-inflammatory therapies when tested in ARDS and sepsis patients under controlled clinical trials, failed to improve survival (at a significant level). Notably, although TNFα is highly up-regulated in both diseases, and was at one time considered as the prime therapeutic molecular target for the treatment of ARDS and sepsis, patients receiving a high dose of a TNFα-receptor-Fc fusion protein actually worsened, possibly due to induction of an iatrogenic immune defect that exacerbated infections (Fisher, Agosti et al. 1996).

Advances in ARDS and sepsis research have shifted the paradigm from an inflammatory primary pathology to a more complex pathophysiology, which involves all the components of the host defence response and is characterised by substantial tissue injury and extensive cell death, aberrant activation of coagulation, mobilisation of early and late inflammatory mediators and altered neuronal regulation of immune cells (Meduri 1996; Rittirsch, Flierl et al. 2008).

Furthermore, these apparently distinct biological processes do not unfold independently of each other, but rather constitute an integrated and highly interactive network. Consequently, deregulation of one of them can affect all the others amplifying potentially detrimental effects.

Thus PAMPs derived from microbes and / or DAMPs released in the tissues due to injury and necrotic cell-death creates a microenvironment which activates the coagulation, complement and inflammatory systems. These, in turn, cross-amplify each other (coagulation aggravates the inflammatory system and *visa-versa*), induce further tissue injury and establish a "vicious cycle" of activation and tissue injury that eventually compromises the functionality of the affected organ(s) and, finally, the survival of the subject.

The realisation in the art that other biological processes, besides inflammation, might play even more important roles in the pathophysiology of diseases such as sepsis and ARDS has created potential for the development of novel therapeutic approaches to treating these diseases.

### Chronic Obstructive Pulmonary Disease (COPD)

Chronic obstructive pulmonary disease (COPD) comprises two related diseases, namely, chronic bronchitis and emphysema. Both cause chronic obstruction of air flowing through the airways in and out of the lungs. The obstruction is generally permanent and worsens over time. The disease is associated with an abnormal inflammatory response of the lungs to noxious particles or gases such as tobacco smoke, passive inhalation of tobacco smoke, air pollution or toxic chemicals in the workplace. Tobacco smoking is the main risk factor for COPD. Emphysema develops when the alveoli, or air sacs of the lungs, become damaged causing them to enlarge and burst. The air sacs are the cells in the lungs where oxygen and carbon dioxide are exchanged and damage in this area makes it difficult for people with emphysema to expel air from their lungs. This causes a build-up of carbon dioxide in the body. Chronic bronchitis is caused by chronic inflammation of the lung airways. When the airways are inflamed and irritated thick mucus begins to form in them which, over time, can block the airways and makes breathing difficult (Han 2011).

Like sepsis and ARDS, COPD involves activation of the Host Defence Response; however, contrary to the acute nature of the activation in the former two diseases COPD involves chronic aberrant HDR activation. Pathology in COPD develops because the respiratory system reacts aberrantly to tissue injury caused by irritants such as cigarette smoke.

Although COPD is a chronic disease the functionality and tissue integrity of the lung deteriorates incrementally through acute exacerbations. Both the chronic component and the acute exacerbations of COPD involve activation of key processes of the HDR such as the coagulation, inflammatory, complement and tissue repair processes (Ashitani, Mukae et al. 2002; Wouters, Groenewegen et al. 2007; Ferhani, Letuve et al. 2010; Borensztajn, von der Thusen et al. 2011; Kanazawa, Tochino et al. 2011).

### Harmful central processes in ARDS, sepsis and COPD and therapeutic strategies relevant to the method of the present invention

### Local and systemic release of DAMPs

The prototype DAMP is a nuclear protein known as HMGB1 (high-mobility group box-1) (Scaffidi, Misteli et al. 2002). HMGB1 is a non-histone nuclear protein that binds to nucleosomes and promotes DNA bending (Agresti and Bianchi 2003). HMGB1 is present at variable levels in most cells (Muller, Ronfani et al. 2004) and is released from cells by two distinct mechanisms. It is liberated from cells undergoing necrosis (Scaffidi, Misteli et al. 2002) or it is actively secreted from stimulated cells. Myeloid and NK cells, when activated, can secrete their nuclear HMGB1 (Gardella, Andrei et al. 2002; Bonaldi, Talamo et al. 2003; Semino, Ceccarelli et al. 2007) after direct translocation to the cytoplasm and accumulation in secretory lysosomes. Although apoptotic cells do not release HMGB1; macrophages engulfing apoptotic cells are induced to secrete HMGB1 (Qin, Wang et al. 2006).

HMGB1 forms highly inflammatory complexes with DNA, LPS, IL-1β and nucleosomes; these complexes interact with the receptor for advanced glycation end products (RAGE) as well as with TLR2, TLR4, TLR9 and IL-1 receptors and lead to the recruitment of inflammatory cells and the release of pro-inflammatory cytokines including TNF-α, IL-1β, and IL-6 (Park, Svetkauskaite et al. 2004; Kokkola, Andersson et al. 2005; Park, Gamboni-Robertson et al. 2006).

Administration of polyclonal anti-HMGB1 antibodies or the DNA-binding "A" box fragment of the HMGB 1 protein, a competitive inhibitor of the pro-inflammatory "B" box fragment of the HMGB 1 protein, or inhibition of RAGE (one of the receptors for HMGB1), improved survival in endotoxaemia and experimental sepsis models (Wang, Bloom et al. 1999; Liliensiek, Weigand et al. 2004; Yang, Ochani et al. 2004; Qin, Wang et al. 2006).

In contrast to other sepsis-associated cytokines, the peak of HMGB 1 release occurs during later stages of the disease (Wang, Bloom et al. 1999) and, consistently, neutralisation of HMGB1 prevented death when treatment occurred after the onset of sepsis, indicating that HMGB1 is an important therapeutic target (Yang, Ochani et al. 2004).

Interestingly, elevated HMGB1 expression may sustain inflammation and remodelling in the airways of COPD patients. Elevated levels of HMGB1 have been detected in airways of cigarette smokers and these levels were further elevated in smokers with COPD. The levels of HMGB1 correlated with the severity of COPD. This suggests that HMGB1 contributes to COPD pathophysiology as well (Ferhani, Letuve et al. 2010; Kanazawa, Tochino et al. 2011).

### Activation of the Complement system and production of anaphylatoxins

As part of the innate immune response, the complement system is activated during the early stages of sepsis leading to the production of large amounts of the anaphylatoxins C3a and C5a (Nakae, Endo et al. 1994; Gerard 2003).

Although C3a appears to play a protective role in experimental sepsis models (Kildsgaard, Hollmann et al. 2000), C5a has numerous harmful effects (Rittirsch, Flierl et al. 2008). C5a acts as a central mediator in sepsis by modulating other systems, including the coagulation cascade, TLR4-mediated responses and the release of cytokines, such as macrophage migration-inhibitory factor (MIF) and HMGB1 (Riedemann, Guo et al. 2004; Hawlisch, Belkaid et al. 2005; Ritis, Doumas et al. 2006; Schreiber, Rittirsch et al. 2006; Rittirsch, Flierl et al. 2008). In clinical studies of sepsis, increased concentrations of C3a, C4a and C5a in the plasma have been linked to poor outcome and survival (Nakae, Endo et al. 1994; Gerard 2003). Interestingly, Complement inhibition decreases the pro-coagulant response and confers organ protection in a baboon model of *E. coli* sepsis (Silasi-Mansat, Zhu et al. 2010).

Complement activation has been linked to COPD pathophysiology as well. Significantly elevated levels of C5a have been found in the induced sputum of patients with COPD (Marc, Korosec et al. 2004; Marc, Kristan et al. 2010). C5a concentrations in patients with COPD correlate with the decline in diffusion capacity of the lung. Furthermore, C5a concentrations are even more increased in COPD patients during acute exacerbations.

### Activation of the coagulation system

The coagulation cascade is initiated when coagulation factors in the blood are exposed to sub-endothelial proteins following damage to the blood-vessel endothelium. In the clinical setting of sepsis and ARDS, dys-regulation of the coagulation cascade results in major complications (Levi and Ten Cate 1999).

Patients with ARDS and / or pneumonia exhibited elevated Tissue Factor (TF) activity in BAL fluid, together with decreased activity of the fibrinolytic system caused by enhanced release of plasminogen activator inhibitor-1 (Gunther, Mosavi et al. 2000). Furthermore, patients who developed ventilator-associated pneumonia, a common prelude to the development of ARDS, exhibited increased concentrations of soluble TF levels. This pro-coagulant change was detected several days prior to clinical and microbiological diagnosis of pneumonia (Schultz, Millo et al. 2004) indicating that aberrant activation of the coagulation system may be the primary instigating event in the pathophysiology of sepsis and ARDS.

The extent of activation of the coagulation cascade during sepsis and ARDS can range from a low level to the occurrence of disseminated intravascular coagulation (DIC). In the initial phase of DIC, thrombin activation results in intra- and extravascular fibrin formation (a process known as hyper-coagulability). However, prolonged activation of the system leads to consumption of coagulation factors and platelet dysfunction, known as hypo-coagulability (Abraham 2000).

In the late phase of DIC, microvascular fibrin deposition is often associated with the development of multi-organ failure owing to perturbations in the microcirculation (Abraham 2000). As DIC develops, inflammation and coagulation interact in a bidirectional manner (Esmon 2004).

Activated thrombin can promote activation of pro-inflammatory pathways and production of pro-inflammatory molecules such as TNF, IL-1β, IL-6 and C5a. These cytokines, in turn, can further stimulate coagulation (Bevilacqua, Pober et al. 1986; Stouthard, Levi et al. 1996; Bernard, Vincent et al. 2001).

Recombinant activated protein C is currently the only approved medicament for the treatment of sepsis (Bernard, Vincent et al. 2001). The administration of activated protein C in sepsis suppresses pro-inflammatory cytokine production and decreases the adhesion of phagocytes to injured endothelium (Bernard, Vincent et al. 2001; Cheng, Liu et al. 2003). However, the anticoagulant activity of activated protein C might exacerbate bleeding complications in patients who usually have a compromised clotting system (Bernard, Vincent et al. 2001), thus, stressing the need for further refinement for this beneficial treatment.

COPD is also associated with aberrant activation of the coagulation cascade. There is clinical and pathological evidence of enhanced pro-thrombotic processes in COPD patients which could potentially account for the increased thrombosis in pulmonary vessels in these patients (Ashitani, Mukae et al. 2002; Wouters, Groenewegen et al. 2007; Borensztajn, von der Thusen et al. 2011).

### Anti-inflammatory therapy with corticosteroids

Corticosteroids (CS) are potent anti-inflammatory agents which have been used with tremendous success for treating many life threatening diseases. However, despite the strong inflammatory component in the pathosphysiology of sepsis and ARDS the benefit of corticosteroid treatment for these diseases is still controversial. Whereas studies examining the effect of large doses CS did not show any benefit (Bernard 2002), studies using prolonged treatment with lower doses of CS (Meduri and Kanangat 1998) or early onset of CS therapy (Bone, Fisher et al. 1987) provided some evidence for improvement in patients with non-resolving acute respiratory distress syndrome (ARDS) (Meduri, Marik et al. 2007).

Inhaled corticosteroids have been widely tested in high doses for the management of COPD patients as well. In sharp contrast to the situation in asthma, corticosteroids provide little or no benefit in COPD patients and may have long-term detrimental effects. High doses of corticosteroids fail to reduce disease progression or mortality, even when combined with a long-acting beta (2)-agonist. The failure of corticosteroids as useful therapeutic agents in COPD therapy emanates, to a great extent, from an insensitivity that COPD patients often develop to corticosteroids (Barnes 2008; Barnes 2011).

It is evident that there is a need in the art for alternative anti-inflammatory or combination therapies comprising, for example, corticosteroids and other medicaments for the management of ARDS, sepsis, COPD and other inflammatory diseases where corticosteroids, surprisingly, do not work effectively.

### Surfactants replacement therapy in the treatment of ARDS

Normally, pulmonary surfactant phospholipids, acting in concert with surfactant protein C and surfactant protein B, reduce surface tension in the alveoli at end-expiration and prevent alveolar collapse (Jobe 1993; Enhorning, Duffy et al. 1995; Spragg, Lewis et al. 2003). Regardless of the cause, ARDS patients and sepsis patients with respiratory complications have dramatically reduced levels, and functionality, of their surfactant system. The overall defective function in these patients may contribute substantially to the characteristic gas-exchange abnormalities and may predispose such patients to injury from mechanical ventilation. Although exogenous surfactant therapy has proven to be effective in the treatment for neonatal respiratory distress syndrome; the value of surfactant replacement therapy for adults with ALI/ARDS has not yet been established. Whereas several small clinical trials have suggested that exogenous surfactant administration may provide some benefit in patients with ARDS (Spragg, Gilliard et al. 1994; Walmrath, Gunther et al. 1996; Gregory, Steinberg et al. 1997; Wiswell, Smith et al. 1999), more thorough studies have demonstrated that the use of exogenous surfactant in a heterogeneous population of patients with ARDS did not improve survival (Anzueto, Baughman et al. 1996; Spragg, Lewis et al. 2004). Still, patients who received surfactant administration had a greater improvement in gas exchange during the 24-hour treatment period than patients who received standard therapy alone, suggesting the potential benefit of a longer treatment course and stressing the need for refinement for this potentially beneficial treatment.

Although recognition that all the above mentioned processes are integral components of the Host Defence Response has raised hope for the development of effective agents for the prophylaxis and / or therapy of sepsis and / or ARDS, the attempts, so far, to target each of these components individually, as described above, has not produced any significant benefit in the clinical setting. Thus there is a need in the art for combined-targeting of the above processes or, ideally, identification and targeting of a central "master regulator(s)" of the HDR that will, in turn, provide useful therapeutic agents for prophylaxis and / or therapy of diseases associated with aberrant HDR activation.

### The transforming growth factor (TGF)-beta superfamily

The transforming growth factor-β (TGF-β) superfamily of proteins includes the sub-families of the transforming growth factors-β (TGF-β), the activins, the bone morphogenic proteins (BMPs), the brain-derived neurotrophic factor (BDNF) and the growth/differentiation factors (GDFs) (Massague 1990; Piek, Heldin et al. 1999; Moustakas and Heldin 2009). In general, CTGF-β family members bind to specific constitutively active type II serine/threonine kinase receptors which can subsequently recruit and phosphorylate specific type I serine/threonine kinase receptors. Deregulated expression or function of the TGF-β signalling system (i.e. changes in the levels of TGF-β cytokines or receptors) may result in pathophysiologic conditions such as autoimmune, neurodegenerative and fibrotic diseases, as well as carcinogenesis and chronic inflammation.

Activins comprise a distinct subfamily of the TGF-β superfamily. They are dimeric proteins, consisting of βA and βB subunits which are connected by disulfide linkages. Three different isoforms of activin, i.e. homodimeric activin-A (βA/βA) and activin-B (βB/βB), and heterodimeric activin-AB (βA/βB), have been described (Ling, Ying et al. 1986; Vale, Rivier et al. 1986). Additional βC, βD and βE chains have been discovered (for review see Yu *et al.* (Yu and Dolter 1997)); however, applicants results have demonstrated that overexpression of activin-βC or activin-βE in the lung of a mammal does not affect lung physiology in the way that over-expression of activin-A does, as described herein.

In most *in vitro* assays, activin-A, activin-AB and activin-B possess similar functional activities; however, some differences between the three isoforms have recently been described. An unexpected antagonism between activin-A and activin-B in the control of Ca²⁺ signalling in pancreatic beta-cells has been described (Bertolino, Holmberg et al. 2008) and the selective utilisation of the type-I receptor, ALK-7, by activin-B, and not activin-A, has been proposed as the mechanism accounting for this discrepancy (Bernard, Lee et al. 2006; Bertolino, Holmberg et al. 2008). Nevertheless, despite these discrepancies, activin-A, activin-B and activin-AB share a substantial amount of functional activities *in vitro* and *in vivo* and it is possible that in the context of different diseases any of the aforementioned activin-A, activin-B or activin-AB can set in motion the aberrant HDR activation and contribute to pathology development.

Activin-A was originally identified as a regulator of follicle stimulating hormone (FSH) secretion by the anterior pituitary gland (Vale, Wiater et al. 2004), and has been associated with numerous biological processes ranging from induction of mesoderm during early embryonic development (Thomsen, Woolf et al. 1990) to control of cell growth, differentiation, survival and apoptosis of various cell types, as well as regulation of hematopoiesis, angiogenesis, reproduction, metabolism and endocrine function (Woodruff and Mather 1995; Risbridger, Schmitt et al. 2001; Phillips, Jones et al. 2005).

Over the last several years, activin-A has attracted renewed attention largely due to accumulating evidence implicating it in several facets of immunity and tissue repair (Hubner, Alzheimer et al. 1999; Munz, Hubner et al. 1999; Sulyok, Wankell et al. 2004; Phillips, de Kretser et al. 2009). Functionally, activin-A is characterised by remarkable complexity and *numerous in vitro* studies have suggested often conflicting activities. A pro-inflammatory role has been supported by *in vitro* studies which showed that activin-A induces IL-6, TNFα, IL-1β and inducible nitric oxide synthase (iNOS) expression in macrophages (Nusing and Barsig 1999). Conversely, other *in vitro* studies described numerous anti-inflammatory properties such as suppression of IL-6 mediated signalling (Brosh, Sternberg et al. 1995), stimulation of IL-1 receptor antagonist secretion (Ohguchi, Yamato et al. 1998) and inhibition of NO synthesis in LPS-activated macrophages (Nusing and Barsig 1999; Wang, Tai et al. 2008). Consistent with an anti-inflammatory role for activin-A are reports describing its capacity to suppress *in vitro* T cell (Hedger, Drummond et al. 1989), B cell (Ogawa, Funaba et al. 2008) and human natural killer cell (Robson, Wei et al. 2009) activities, to inhibit maturation of dendritic cells (Segerer, Muller et al. 2008) and notably, to promote the development of Foxp3-/IL-10 producing regulatory CD4⁺T cells (Semitekolou, Alissafi et al. 2009), and to synergise with TGF-β to induce Foxp3⁺ regulatory cells (Huber, Stahl et al. 2009). Activin-A has also been implicated in the regulation of tissue repair and matrix remodelling related processes such as proliferation of lung fibroblasts and their differentiation into myofibroblasts (Ohga, Matsuse et al. 2000), induction of metalloprotease synthesis (Caniggia, Lye et al. 1997; Yoshinaga, Mimori et al. 2008), induction of the "alternative activated" phenotype of macrophages (Ogawa, Funaba et al. 2006), growth and contractility of vascular smooth muscle cells (Kojima, Mogami et al. 1993; Cho, Yao et al. 2003) and angiogenesis (Panopoulou, Murphy et al. 2005).

Not surprisingly, considering such wide spectrum of biological activities, and in the absence of adequate in-vivo substantiated information, both harmful and protective roles have been proposed for activin-A in the context of several immuno-inflammatory disorders. Thus, detection of high activin-A levels in patients and experimental animals adds support to the notion that activin-A may be involved in the pathogenesis of inflammatory bowel disease (Dohi, Ejima et al. 2005), experimental allergic airway inflammation and human asthma (Rosendahl, Checchin et al. 2001; Hardy, O'Connor et al. 2006; Karagiannidis, Hense et al. 2006), inflammatory arthropathies (Gribi, Tanaka et al. 2001), pulmonary (Matsuse, Fukuchi et al. 1995; Aoki, Kurabayashi et al. 2005), colonic, renal and hepatic fibrosis (Sulyok, Wankell et al. 2004; Werner and Alzheimer 2006) and sepsis (Phillips, Jones et al. 2001; Jones, Mansell et al. 2007).

Indirect evidence in favour of a pathogenic role of activin-A *in vivo* has been obtained from the utilisation of follistatin, an endogenous activin-inhibitor. Over-expression, or exogenous administration, of follistatin inhibited neutrophilic and mononuclear cell inflammation and fibrosis in the bleomycin-induced lung injury model (Aoki, Kurabayashi et al. 2005), in models of experimental colitis (Dohi, Ejima et al. 2005) and in an LPS-induced sepsis-like models in mice (Jones, Mansell et al. 2007). Although these findings were interpreted to suggest that activin-A is a good therapeutic target, in most of these studies follistatin was administered prophylactically and thus the observed protection could have originated from a reduction of bleomycin-toxicity through an interference with its metabolism or biodistribution rather than from modulating any subsequent injury-induced biological responses. Furthermore, prophylactic neutralisation of endogenous activin-A in the ovalbumin model, either with follistatin or follistatin-neutralising antibodies, has produced conflicting data suggesting protective (Semitekolou, Alissafi et al. 2009) or detrimental (Hardy, O'Connor et al. 2006) roles for this molecule.

Even though ALI/ARDS and sepsis are distinct disease entities there is an intimate interrelation among them (Meduri 1996; Khadaroo and Marshall 2002; Costa, Schettino et al. 2006; Tsushima, King et al. 2009). Both sepsis and ALI/ARDS represent typical examples of severe pathology associated with aberrant HDR activation. ALI and its most severe form ARDS are fulminant respiratory conditions in which injury of the lung epithelium and endothelium triggers an uncontrolled inflammatory response and leads to often severe functional and structural deterioration of the lung lung (Herridge and Angus 2005; Ware 2006; Matuschak and Lechner 2010). A wide variety of precipitating causes are recognised including pneumonia and aspiration of gastric contents; however, severe sepsis is the leading cause of ARDS (Bernard, Artigas et al. 1994; Tsushima, King et al. 2009). This "cause-effect" relationship could stem from the mobilisation of similar, potentially harmful, effector mechanisms during their development, with activin-A being one of them.

Sepsis and ARDS remain the leading cause of death in intensive care units (ICUs), claiming annually, only in the United States, hundreds of thousands of lives and constituting a major burden to the health care systems worldwide (Deans, Haley et al. 2005; Herridge and Angus 2005; Ware 2006; Matuschak and Lechner 2010). Identification of rate-limiting factors in the chaotic immune reactivity of these diseases is essential for development of effective treatment strategies.

COPD is another major cause of morbidity and mortality (Halpin and Miravitlles 2006). It represents a substantial economic and social burden throughout the world. Currently, COPD is the fifth leading cause of death worldwide and, despite advances in management; mortality is expected to increase in the coming decades, in marked contrast to other chronic diseases such as heart disease and stroke where there have been considerable decreases in mortality. The burden of COPD to patients and their families is high, both in terms of quality of life and health status. COPD exacerbations and hospitalisations (are a major financial burden) represent the major driver of the cost and morbidity of COPD.

Although COPD is clearly a disease different from sepsis and ARDS, because it is also associated with an aberrant host response to tissue damage, its pathophysiology, especially during acute exacerbations, incorporates mechanisms and processes that are involved in sepsis and ARDS and thus therapeutic agents targeting components of aberrant HDR activation will be useful for the management of COPD exacerbations.

Numerous pharmacologic therapies have been evaluated in Phase II and Phase III clinical trials for the treatment of ALI/ARDS. These treatments include glucocorticoids, surfactants, inhaled nitric oxide, antioxidants, protease inhibitors and a variety of other anti-inflammatory treatments. Unfortunately, to date, none of these pharmacologic treatments has proven to be effective (Cepkova and Matthay 2006; Matthay and Zemans 2011).

The similarly disappointing results in clinical trials for therapeutic agents targeting sepsis have led opinion leaders in the art to even question the existence of key rate-limiting factors in sepsis pathophysiology which could be targeted pharmacologically to treat sepsis successfully. As stated by Rittirsch *et al. "...it is now clear that the belief that a single key mediator causes sepsis, and that neutralization of such a factor could be a cure for all patients with sepsis, is erroneous. Instead, we now understand that sepsis is a complex, dynamic syndrome with great heterogeneity, and not a distinct disease.* " (Rittirsch, Flierl et al. 2008).

Thus there is a need in the art for new treatments for diseases caused by aberrant HDR activation, such as those described above, because these diseases are major causes of morbidity and / or loss of quality of life and constitute a tremendous burden on medical systems.

Applicants contemplate that, in contrast to the targeting of individual processes of the HDR (e.g. targeting only the coagulation, complement, inflammatory or tissue repair system) that so far has provided disappointing results, pharmacological neutralisation of key regulators of the HDR activation process can lead to simultaneous normalisation of many pathogenic pathways, and thus provides therapeutic benefit to patients where aberrant HDR activation drives pathology. Therefore, a therapeutic agent that pharmacologically neutralises a key HDR regulator will simultaneously affect pathogenic activation of the coagulation, complement, inflammatory and tissue repair processes thus acting as a suitable target for the treatment of aberrant HDR activation associated pathologies. Against predictions in the art, the current invention demonstrates that activin neutralisers are suitable for the prophylaxis and / or therapy of diseases associated with aberrant HDR activation in a subject.

In work leading up to the present invention, applicants have made the following discoveries: (a) applicants have discovered that up-regulation of activin-A is sufficient to activate all the processes of the HDR, thus demonstrating that activin-A is a "master mobiliser" of the HDR, and linking activin-A to the development of aberrant HDR activation in the context of diseases including, but not limited to, ALI/ARDS, sepsis and COPD; (b) applicants have discovered that in two independent mammal-disease models, one involving ectopic overexpression of activin-A with the use of adenovirus-mediated gene transfer in the lung (exogenous activin-A) and the other involving intra-tracheal instillation of lipopolysaccharite (LPS) (endogenous activin-A), activin-A levels have a rate-limiting function on the intensity of the HDR and the severity of pathology of the experimental animals; (c) applicants have discovered that, contrary to the prediction that pathologies such as sepsis and ARDS will not be attenuated by the targeting of a single pathogenic factor, prophylactic and, even more surprisingly, therapeutic neutralisation of activin-A in the above said two independent mammal models of ALI/ARDS protects mammals from the damaging effect of aberrant HDR activation; and (d) applicants have discovered that human subjects diagnosed with ARDS have substantially elevated levels of activin-A in their lungs, substantiating the relevance of applicants mammal-model discoveries to human disease.

Based on the prior art described above it is now disclosed herein that prophylactic and / or therapeutic pharmacological neutralisation of activin-A can attenuate the destructive effects of aberrant HDR activation and, thus, activin-A neutralisers are useful therapeutic agents both for the prophylaxis and / or therapy of diseases associated with aberrant HDR activation in a subject.

In the present study, which was initiated to delineate, *in vivo,* the consequences of deregulated activin-A expression, applicants demonstrate that ectopic expression of activin-A in the murine lung leads to a severe, sustained and continuously evolving respiratory pathology. Serial assessment of functional, histological and molecular alterations of the treated mice for a period of four months after viral instillation showed that many of the pro-inflammatory, anti-inflammatory and matrix remodelling activities that have been ascribed to activin-A by *in vitro* studies prevail in the complex lung environment as well, although at distinct phases of a dynamically evolving process. Moreover, applicants show for the first time that activin-A mediates regulation of surfactant biosynthesis, activation of the coagulation system and, more importantly, applicants provide evidence implicating activin-A in the pathophysiology of ARDS. Furthermore, applicants show that therapeutic administration of a recombinant soluble activin-A neutraliser (ActRIIB-Fc), at progressed stages of the disease, significantly attenuates the activin-A-induced pathology, demonstrating that activin-A neutralisers, as exemplified by ActRIIB-Fc, are useful therapeutic agents for the prophylaxis and / or therapy of diseases associated with aberrant HDR activation in a subject.

Finally, applicants show that the final outcome of a deregulated activin-A response in the lung tissue is the development of emphysema, the hallmark of COPD, thus linking activin-A with COPD pathophysiology and therefore activin-A neutralisers, as disclosed in the present invention, may be useful therapeutic agents for the treatment of COPD patients, in particular, during acute exacerbations.

Accordingly, the present invention describes a role for activin neutralisers that will be useful for the prophylaxis and / or therapy of diseases associated with aberrant HDR activation in a subj ect.

Therefore, the present invention provides useful therapeutic agents in the prophylaxis and /or therapy of diseases associated with aberrant HDR activation in a subject, in the form of activin neutralisers, as exemplified by ActRIIB-Fc.

### SUMMARY OF THE INVENTION

The specification contains nucleotide sequence information prepared using PatentIn, presented herein after the bibliography. Nucleotide sequences referred to in the specification are identified by the indicator SEQ ID NO: followed by the sequence identifier (e.g. SEQ ID NO:1, SEQ ID NO: 2, etc.). The sequence identifier referred to in the specification correlates to the information provided in numeric indicator field <400> in the sequence listing, which is followed by the sequence identifier (e.g. <400>1, <400>2, etc.). That is SEQ ID NO: 1, as detailed in the specification, correlates to the sequence indicated as <400> 1 in the sequence listing.

The present invention was made in view of the prior art described above and relates to the discovery that activin neutralisers, as exemplified herein by ActRIIB-Fc, are useful therapeutic agents for the prophylaxis and / or therapy of respiratory and systemic diseases associated with aberrant HDR activation in a subject. Typically, aberrant HDR activation may involve abnormally elevated expression of activin-A, activin-B or activin-AB. The results disclosed herein support the position that administration of an activin neutraliser, as exemplified by ActRIIB-Fc, attenuates pathology associated with aberrant HDR activation.

According to a first aspect of the present invention, there is provided activin neutralisers in the form of soluble ActRII polypeptides, or a fragment(s) or analogue thereof for the prophylaxis and / or therapy of diseases associated with aberrant HDR activation in a subject. Also provided are methods for producing and purifying activin neutralisers, where these are soluble ActRII polypeptides, or a fragment(s) or analogue thereof.

According to a second aspect of the present invention, there is provided activin neutralisers in the form of follistatin and follistatin-like 3 protein, or a fragment(s) or analogue thereof for the prophylaxis and / or therapy of diseases associated with aberrant HDR activation in a subject. Also provided are methods for producing and purifying activin neutralisers, where these are follistatin and follistatin-like 3 protein, or a fragment(s) or analogue thereof.

According to a third aspect of the present invention, there is provided activin neutralisers in the form of low molecular weight organic compounds for the prophylaxis and / or therapy of diseases associated with aberrant HDR activation in a subject. Also provided are methods for screening and purifying activin neutralisers, where these are low molecular weight organic compounds.

According to a fourth aspect of the present invention, there is provided activin neutralisers in the form of antibodies raised against either activin-A or activin-B, or activin receptors or immunogenic portions thereof, for the prophylaxis and / or therapy of diseases associated with aberrant HDR activation in a subject. Also provided are methods for producing and purifying activin neutralisers, where these are antibodies raised against activin-A or activin-B or activin receptors or immunogenic portions thereof.

According to a fifth aspect of the present invention, there is provided activin neutralisers in the form of antisense nucleic acids, ribozymes, triplex agents, siRNAs and miRNAs, for the prophylaxis and / or therapy of diseases associated with aberrant HDR activation in a subject. Also provided are methods for producing antisense nucleic acids, ribozymes, triplex agents, siRNAs and miRNAs.

According to a sixth aspect of the present invention, there is provided pharmaceutical compositions comprising at least one activin neutraliser and a pharmaceutically acceptable carrier, adjuvant and / or diluent for the prophylaxis and / or therapy of diseases associated with aberrant HDR activation in a subject. Also provided are modes and routes of administration and dosages of said activin neutralisers.

According to a seventh aspect of the present invention, there is provided methods for the prophylaxis and / or therapy of diseases associated with aberrant HDR activation in a subject by administering to said subject a pharmaceutical composition comprising at least one activin neutraliser and a pharmaceutically acceptable carrier, adjuvant and / or diluent. The diseases that can be treated according to the method of the present invention are selected from the group consisting of: (a) acute injury of tissues inflicted by infection, toxic substances or trauma, heart infarction, wound healing pursuant to surgery, severe burns or other tissue injury, meningitis, appendicitis, renal tubular necrosis, traumatic brain injury and sepsis; (b) autoimmune diseases including, but not limited to, Rheumatoid arthritis, multiple sclerosis, inflammatory bowel disease, vasculitis, anti-phospholipid Syndrome, sclerodema, Systemic lupus erythematosous and osteoarthritis; and (c) respiratory diseases including, but not limited to, severe asthma, interstitial fibrosis, organising peumonia, non-specific interstitial pneumonia, sarcoidosis, cystic fibrosis, lung transplantation, bronchiolitis obliterans, pulmonary hypertension, pneumonia, ALI/ARDS, COPD, acute responses against infectious agents such as pathogenic viruses (including, but not limited to, influenza A viruses H5N1 and H1N1, coronaviruses SARS, and human rhinoviruses C and D).

According to an eighth aspect of the present invention, there is provided methods for treatment of diseases associated with aberrant HDR activation in a subject by administering a pharmaceutical composition comprising at least one activin neutraliser in combination with other therapies. The diseases that can be treated by a combination of therapies according to the method of the present invention are selected from the group consisting of: (a) acute injury of tissues inflicted by infection, toxic substances or trauma, heart infarction, wound healing pursuant to surgery, severe bums or other tissue injury, meningitis, appendicitis, renal tubular necrosis, traumatic brain injury and sepsis; (b) autoimmune diseases including, but not limited to, Rheumatoid arthritis, multiple sclerosis, inflammatory bowel disease, vasculitis, anti-phospholipid Syndrome, scleroderma, Systemic lupus erythematosous and osteoarthritis; and (c) respiratory diseases including, but not limited to, severe asthma, interstitial fibrosis, organising peumonia, non-specific interstitial pneumonia, sarcoidosis, cystic fibrosis, lung transplantation, bronchiolitis obliterans, pulmonary hypertension, pneumonia, ALI/ARDS, COPD, acute responses against infectious agents such as pathogenic viruses (including, but not limited to, influenza A viruses H5N1 and H1N1, coronaviruses SARS, and human rhinoviruses C and D).

### BRIEF DESCRIPTION OF THE DRAWINGS

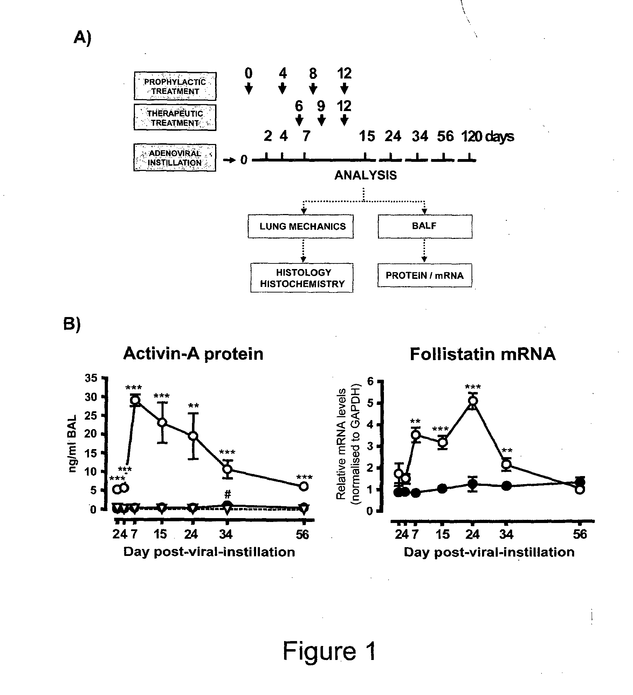

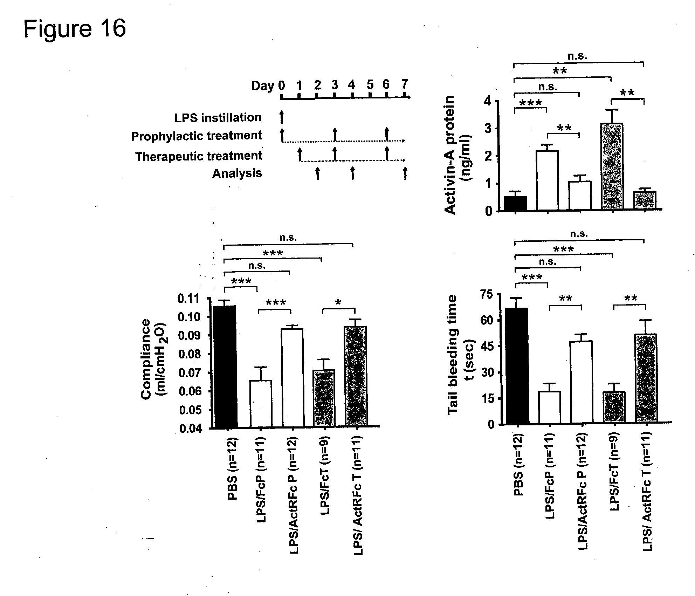

**Figure 1****.Adenovirus-mediated activin-A over-expression in the lung murine model**
   A) Experimental protocol for viral instillation and activin-A neutralisation using ActRIIB-Fc. Viruses were instilled in the trachea of C57BL/6 female mice which were analysed up to 120 days post-instillation. For prophylactic or therapeutic neutralisation of activin-A, 200 µg of recombinant ActRIIB-Fc were injected i.p. on the indicated days and the mice were terminated on day 15. B) Activin-A protein concentration in BAL fluids and follistatin mRNA levels in total lung extracts from Ad-ActA (n = 5, open circles), Ad-GFP (n = 3, dark circles) and untreated control mice (n = 3, open triangles). Virus-treated groups were compared with the untreated "pool" control group using one-way analysis of variance (ANOVA) with Bonferonni's post-hoc test analysis. *P<0.05, **P<0.01 and ***P<0.001.

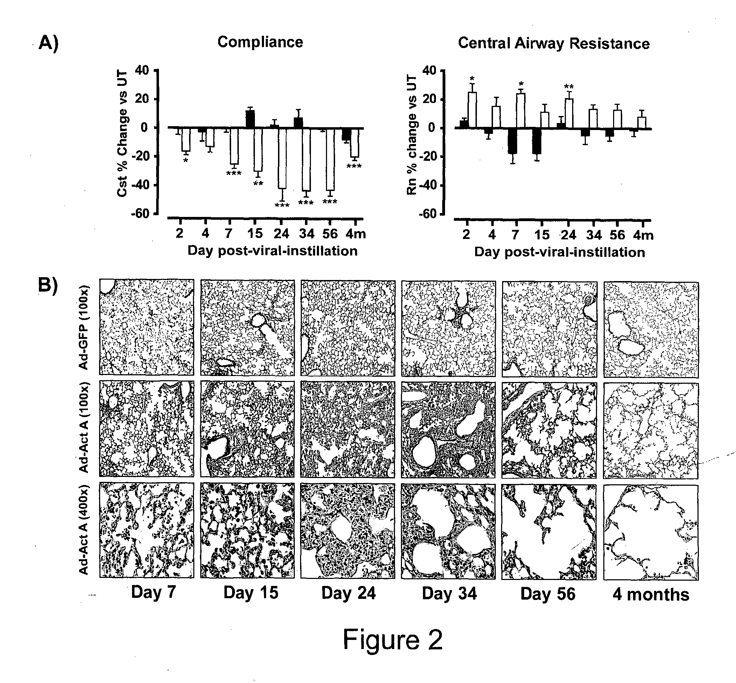

**Figure 2****. Activin-A over-expression in the airways leads to severe and chronic respiratory pathology in a murine model**
   A) Lung mechanics were analysed in anesthetised mice using the Flexivent system. Values are means ± SEM calculated of three replicate measurements for each mouse, shown as open bars for Ad-ActA (n = 5) and black bars for Ad-GFP-treated (n = 3) mice and normalised to the values of the untreated control group (n = 3). At each time point the Ad-ActA and Ad-GFP groups were compared with the untreated control group using one-way analysis of variance (ANOVA) with Bonferonni's post-hoc test analysis. *P<0.05, **P<0.01 and ***P<0.001. B) Representative H&E stained sections from Ad-GFP (100X) or Ad-ActA-treated (100X and 400X) mice.

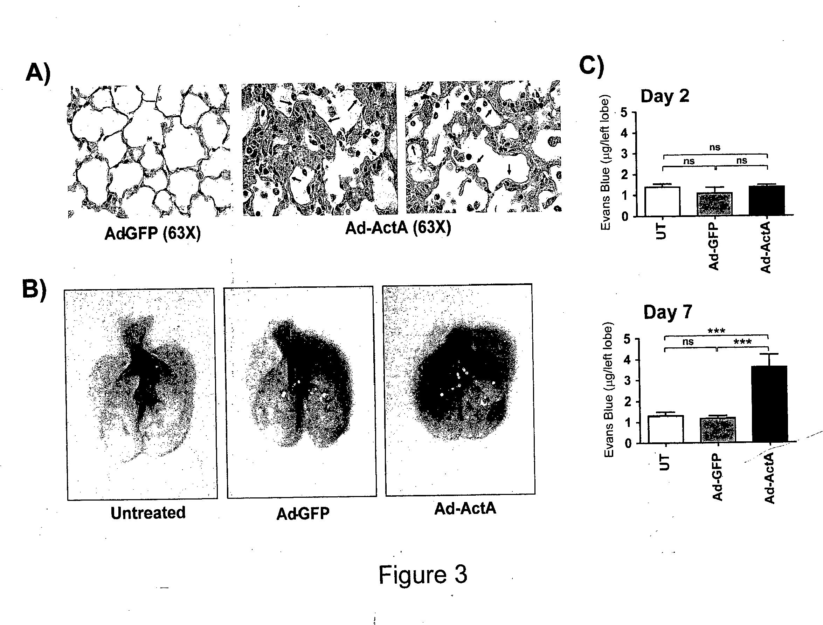

**Figure 3****. Activin-A over-expression in the lung causes micro-vascular injury and plasma extravasation in a murine model**
   A) H&E micrographs demonstrating the formation of hyaline membranes on the alveolar walls of Ad-ActA-treated mice. Hyalin membranes are indicated by arrows. B) Evan's Blue retention in the mesenchyma of Ad-ActA-treated mice. C) Quantitation of Evan's Blue retention in the left lung lobe of untreated and Ad-GFP or Ad-ActA-treated mice. Values are means ± SEM of 6 mice per group analysed 2 and 7 days post-viral-instillation.

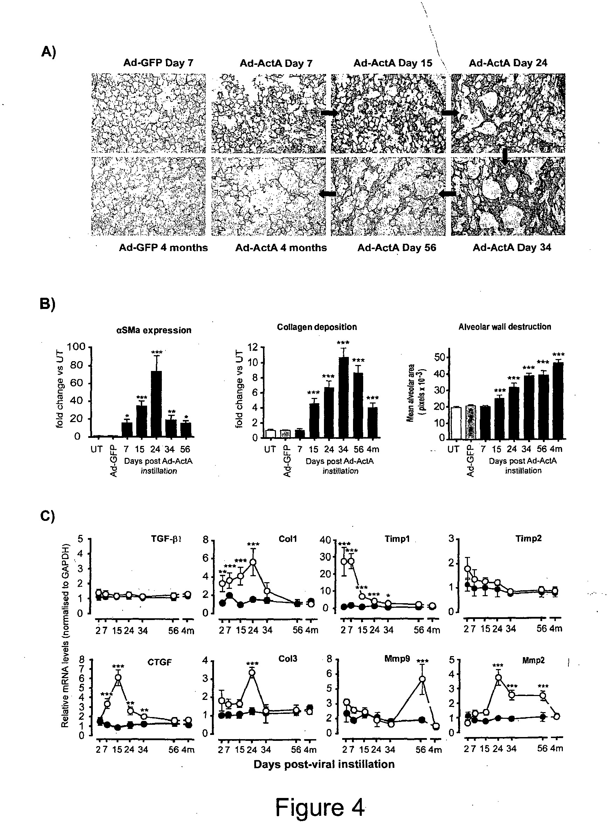

**Figure 4****. Activin-A over-expression leads to a transient fibrotic reaction which resolves and paves the way to the development of emphysematous lesions in a murine model**
   A) Representative Sirius Red stained tissue sections from Ad-GFP and Ad-ActA-treated mice demonstrating the transient fibrotic response induced by activin-A over-expression. B) Morphometric analysis of aSMA immuno-staining, Sirius Red staining and alveolar enlargement expressed as mean ± SEM of five mice per group. The virus-treated groups were compared with the untreated "pool" group using one-way analysis of variance (ANOVA) with Bonferonni's post-hoc test analysis. *P<0.05, **P<0.01 and ***P<0.001. C) Quantitative PCR analysis of TGFβ1, Collagens I & III, CTGF, TIMPs-1 &-2 and MMPs-2 & -9 mRNA expression in the Ad-ActA, Ad-GFP and untreated groups. The Ad-ActA (n = 5 per time point, open circles) and Ad-GFP (n = 3 per time point, dark circles) at each time point were compared with the untreated "pool" group using one-way analysis of variance (ANOVA) with Bonferonni's post-hoc test analysis. *P<0.05, **P<0.01 and ***P<0.001.

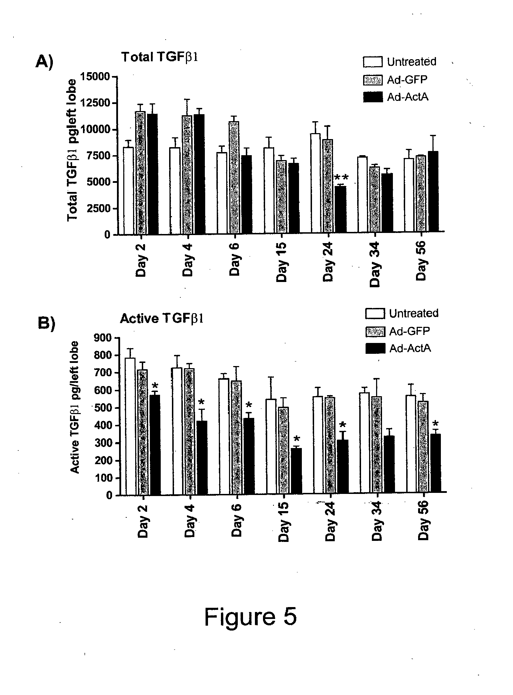

**Figure 5****. Total and active TGF-β levels in protein lung extracts from control and adenovirus-treated mice**
   Total (A) and active (B) CTGF-β protein levels were estimated by ELISA as described in the examples. The untreated (n = 3 per time point, white bars), Ad-GFP (n = 3 per time point, grey bars) and Ad-ActA-treated mice (n = 5 per time point, black bars) were compared at each time point using one-way analysis of variance (ANOVA) with Bonferonni's post-hoc test analysis. *P<0.05 and **P<0.01.

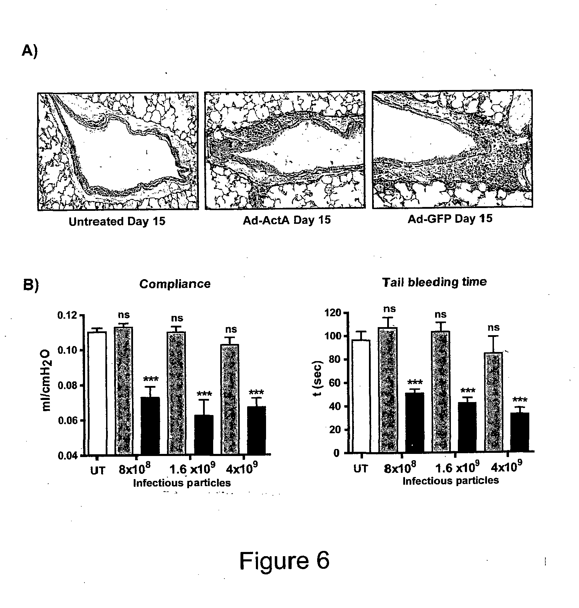

**Figure 6****: Adenovirus-induced peri-vascular inflammatory cell accumulation in a murine model**
   A) H&E stained lung sections from untreated and Ad-ActA or Ad-GFP-treated mice (8 × 10⁸ infectious particles) analysed 15 days post-viral-instillation. Similar levels of inflammatory cells accumulate in the peri-vascular region of large mascularised blood vessels suggesting that this effect is adenovector-dependent. B) The peri-vascular inflammation is not associated with any pathological change. In contrast to Ad-ActA-treated mice (black bars), even 4 × 10⁹ Ad-GFP infectious particles (grey bars) do not affect significantly lung compliance, tail bleeding time or any other parameter analysed. Lung mechanics and tail bleeding time were measured as described in the examples. Values are means ± SEM of 6 mice per group, analysed 25 days post-viral-instillation, compared with the untreated control group using one-way analysis of variance (ANOVA) with Bonferonni's post-hoc test analysis (***P<0.001).

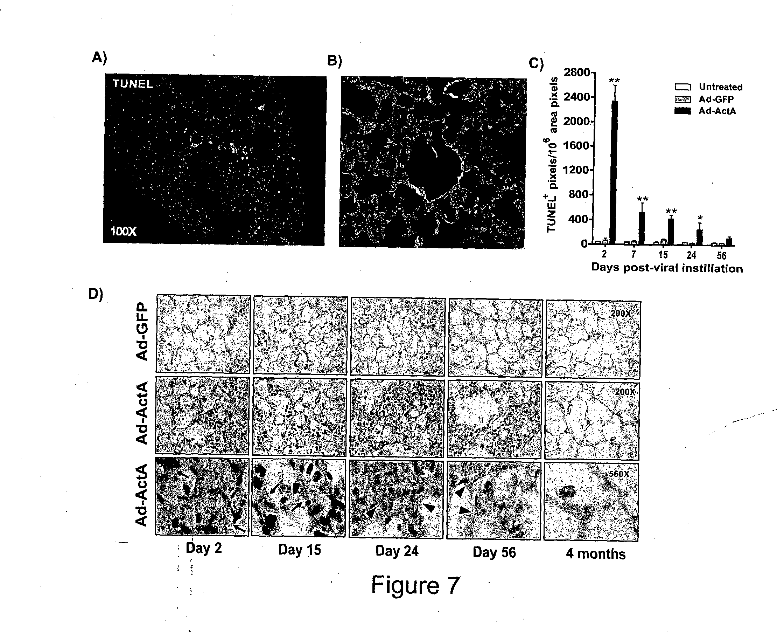

**Figure 7****. Activin-A induces an acute wave of alveolar cell death and a sustained increase of HMGB1 immuno-staining in a murine model**
   A) Low magnification of TUNEL-stained lung 48 hours post-viral-instillation. B) Representative confocal image of TUNEL and T1α double stained lung section from an Ad-ActA-treated mouse 2 days post-viral-instillation. C) Morphometric analysis of TUNEL⁺ staining expressed as mean ± SEM of 3 untreated, 5 Ad-ActA and 3 Ad-GFP-treated mice per time point. Data were analysed at each time point using one-way analysis of variance (ANOVA) with Bonferonni's post-hoc test analysis. *P<0.05, **P<0.01.D) HMGB1 Immuno-staining of tissue sections from mice treated with either Ad-GFP (200X magnification) or Ad-ActA (200 and 560X magnification). HMGB1 expression is manifested as intense nuclear and diffuse cytoplasmic staining in inflammatory cells, extra-cellular staining in the parenchyma and, notably, in the lumen of vascular capillaries. Interstitial and intra-capillary staining is shown with arrows and arrowheads, respectively.

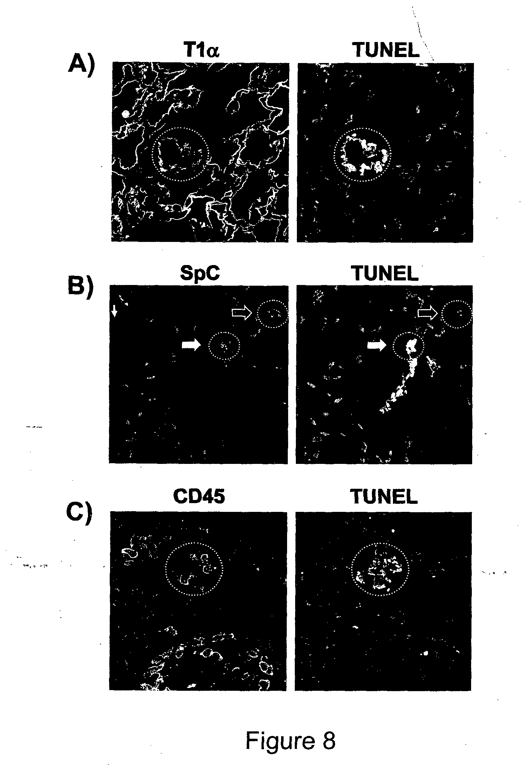

**Figure 8****. Co-staining of Ad-ActA-treated mice lungs with TUNEL, T1α, SpC and CD45**
   Immuno-staining of Ad-ActA-treated lung sections collected 48 hours post-viral-instillation with TUNEL and T1α (A), SpC (B) and CD45 (C) respectively. Figure A shows T1α positive material admixed with TUNEL⁺ debris. Figure 8 shows one TUNEL⁺ and one TUNEL⁻ Type-II-pneumonocyte (filled and open arrows, respectively). Figure C shows a cluster of CD45⁺ cells admixed with TUNEL⁺ cell debris.

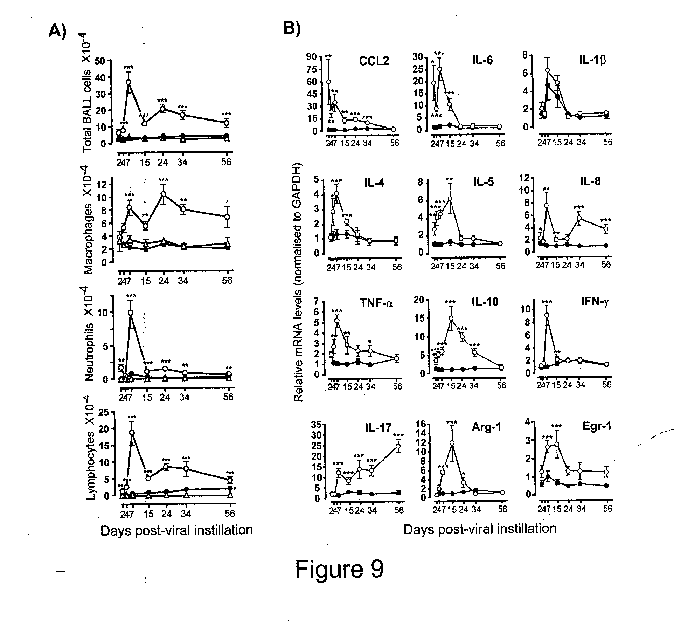

**Figure 9****. Activin-A over-expression in the lung triggers a chronic and continuously evolving inflammatory response in a murine model**
   A) Total BAL cells and differential counts of macrophages (MΦ), neutrophils (Neu) and lymphocytes (Lym) expressed as mean ± SEM. At each time point 5 Ad-ActA (open circles), 3 Ad-GFP-treated mice (dark circles) and 3 untreated controls (open triangles) were estimated. Data were compared at each time point using one-way analysis of variance (ANOVA) with Bonferonni's post-hoc test analysis. *P<0.05, **P<0.01, ***P<0.001 (Ad-ActA vs untreated and Ad-GFP) and ^{#}P<0.05 (untreated vs Ad-GFP). B) Quantitative PCR analysis of mRNA expression of various cytokines and chemokines. In all the above experiments the Ad-ActA (n = 5, open circles) and Ad-GFP (n = 3, dark circles) groups at each time point were compared with the untreated "pool" group using one-way analysis of variance (ANOVA) with Bonferonni's post-hoc test analysis. *P<0.05, **P<0.01 and ***P<0.001.

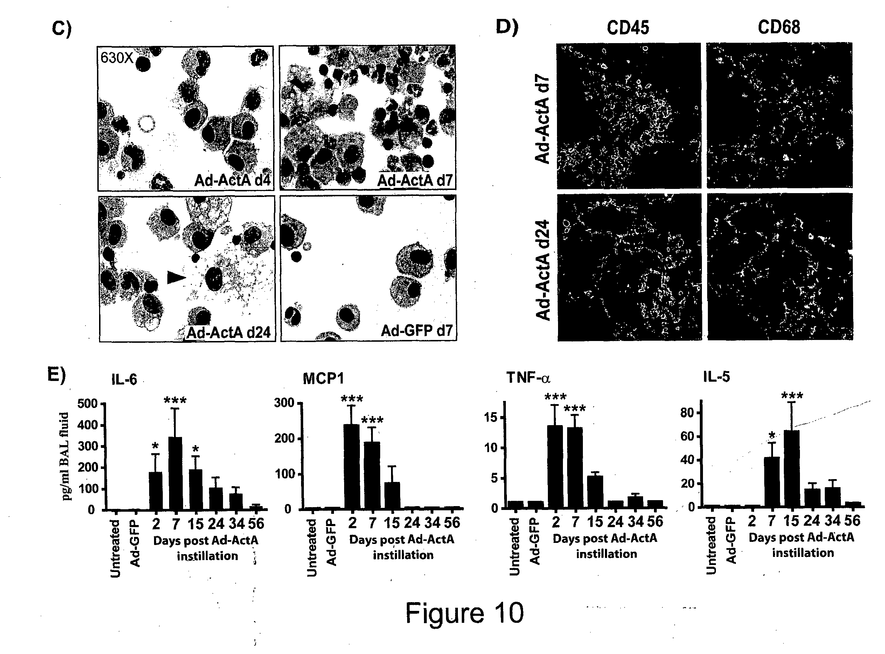

**Figure 10****. Inflammatory cells and cytokine protein levels induced by Activin-A in a murine lung**
   A) Representative Giemsa-May Grunwald-stained BAL cell cytospin preparations. B) Confocal images of sequential tissue sections stained for CD45 or CD68 on days 7 and 24 post-viral-instillation. C) Protein levels for IL-6, TNF-α, IL-5 and MCP-1 in BAL fluids from Ad-ActA (n = 5 per time point), Ad-GFP (n = 3 per time point) and untreated control mice (n= 3 per time point) were measured as described in the examples. Virus-treated groups were compared with the untreated "pool" control group using one-way analysis of variance (ANOVA) with Bonferonni's post-hoc test analysis. *P<0.05, **P<0.01 and ***P<0.001.

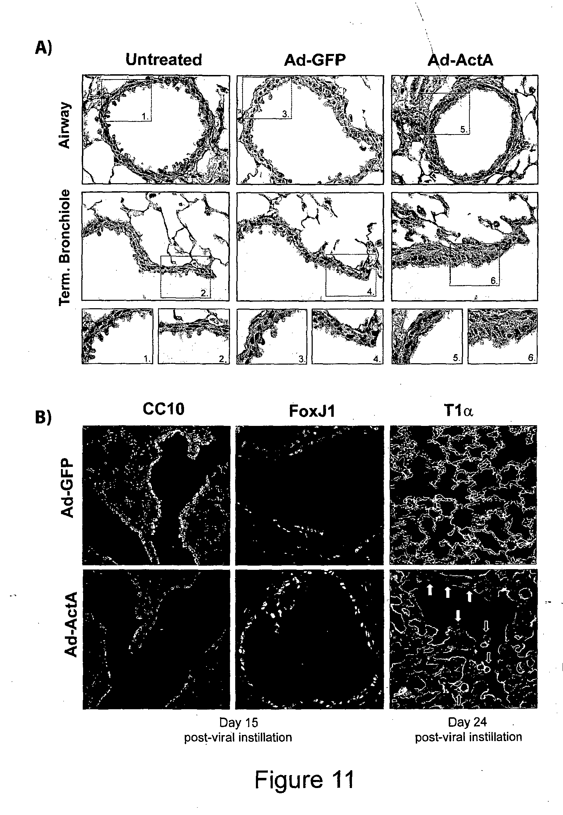

**Figure 11****. Altered epithelial cell composition in Ad-ActA-treated mice**
   A) Haematoxilin & Eosin stained lung section from untreated, Ad-GFP and Ad-ActA-treated mouse 15 days post-viral-instillation. The lower panel shows cropped regions of the images above. B) Representative confocal images of CC10, Foxj1 and T1α stained tissue sections from Ad-GFP or Ad-ActA-treated mice 15 days post-viral-instillation (for CC10 and Foxj1) and 24 days for T1α.

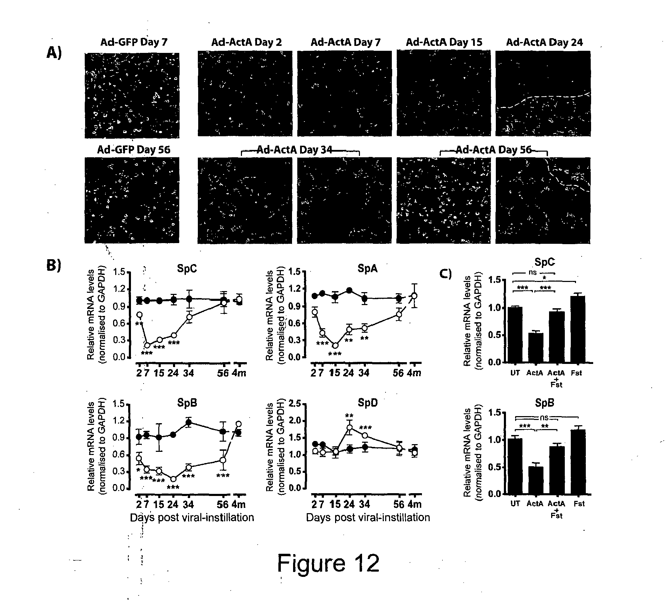

**Figure 12****. Down-regulation of the surfactant protein levels in Ad-ActA-treated mice**
   A) Confocal images from lung tissues immuno-stained for SpC at the indicated time points. The regional heterogeneity of SpC expression pattern at the later stages of the activin-A-induced response is illustrated by the inclusion of two neighbouring images (day 34 and day 56) with representative SpC-devoid and SpC-rich regions (the border between SpC-devoid and SpC-rich regions are shown with the dashed line). SpC immuno-staining was pseudo-coloured white. Cells stained green are virus infected GFP-expressing cells. B) Quantitative PCR analysis of surfactant protein mRNA expression in the Ad-ActA, Ad-GFP and untreated groups. The Ad-ActA (n = 5 per time point, open circles) and Ad-GFP (n = 3 per time point, dark circles) virus-treated groups were compared with the untreated "pool" control group using one-way analysis of variance (ANOVA) with Bonferonni's post-hoc test analysis. *P<0.05, **P<0.01 and ***P<0.001. C) Quantitative PCR analysis of the mRNA levels of SpC and SpB in the mouse lung epithelial cell line MEL12 after 24 hour culture with recombinant activin-A (10 ng/ml) and / or follistatin (30 ng/ml). Values represent mean ± SEM of four independent experiments. The groups were compared using one-way analysis of variance (ANOVA) with Bonferonni's post-hoc test analysis. *P<0.05, **P<0.01 and ***P<0.001.

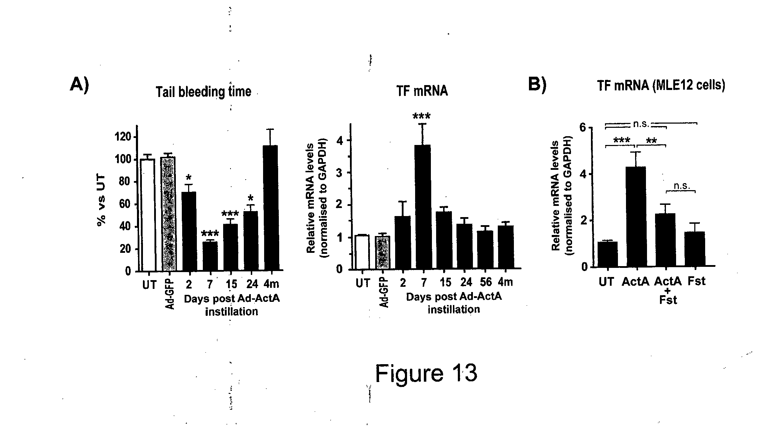

**Figure 13****. Establishment of a systemic hyper-coagulant state in the Ad-ActA-treated mice**
   A) Demonstration of a pro-coagulant state in Ad-ActA-treated mice by measuring tail bleeding time and levels of TF mRNA by quantitative PCR analysis. Values represent mean ± SEM of five to seven mice from two independent experiments compared with the untreated control group using one-way analysis of variance (ANOVA) with Bonferonni's post-hoc test analysis. *P<0.05, **P<0.01 and ***P<0.001. B) Treatment of the mouse lung epithelial cell line MEL12 with recombinant activin-A (10 ng/ml) and / or follistatin (30 ng/ml) *in vitro* for 24 hours. TF mRNA levels were analysed by quantitative PCR. Values represent mean ± SEM of four independent experiments. The groups were compared using one-way analysis of variance (ANOVA) with Bonferonni's post-hoc test analysis. **P<0.01 and ***P<0.001.

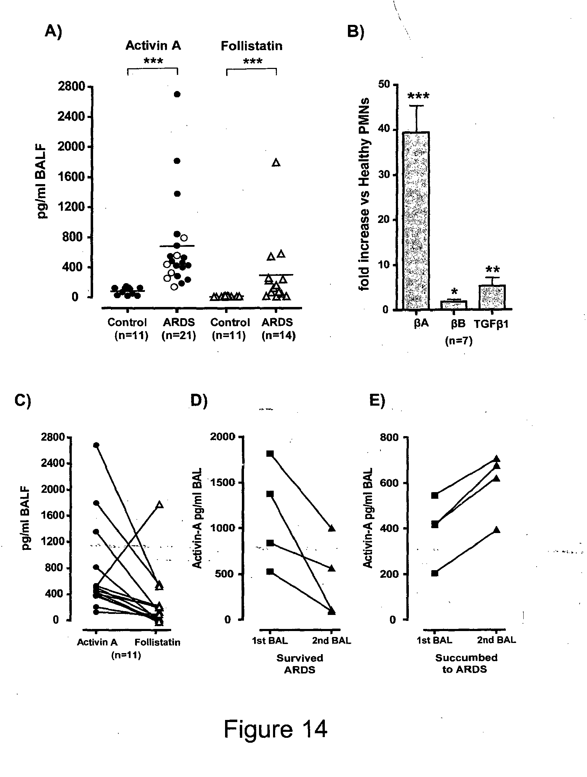

**Figure 14****. Increased levels of activin-A are found in BAL fluids of human ARDS patients and the survival of ARDS patients correlates with Activin-A kinetics.**
   A) Measurement of the activin-A and follistatin protein levels in BAL fluids of ARDS and control patients. B) Pair-wise correlation of activin-A and follistatin protein levels in the same patient. C) Quantitative PCR analysis of the activin-A, activin-B and TGFβ1 mRNA levels in seven ARDS patients where frozen BAL cells were available (indicated as open circles in A). Peripheral mononuclear cell preparations were used as controls. The groups were compared using one-way analysis of variance (ANOVA) with Bonferonni's post-hoc test analysis. *P<0.05, **P<0.01 and ***P<0.001. D&E) BAL samples were collected from ARDS patients twice, once immediately upon admission to the intensive care unit and a second time four days later. Activin-A protein levels estimated by ELISA are shown in pairs (first and second BAL). Figure 14D shows patients which had lower levels of activin-A in the second BAL sample and survived ARDS, and Figure 14F shows patients with increased levels of activin-A protein in the second BAL sample and succumbed to ARDS.

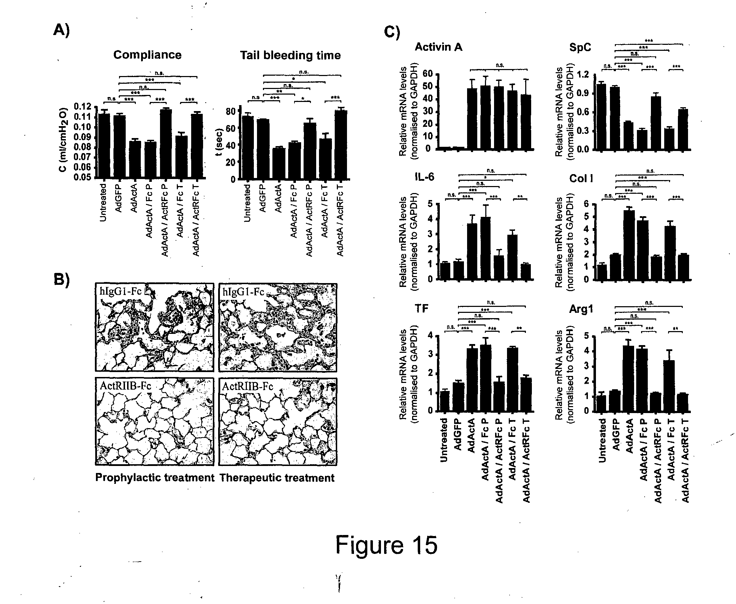

**Figure 15****. Prophylactic and therapeutic neutralisation of activin-A protects experimental mice from activin-A-induced pathology in a murine model**
   ActRIIB-Fc recombinant protein was injected i.p. (200 µg/injection) in Ad-ActA-treated mice following a prophylactic (Ad-ActA/ActRFc-P) or therapeutic (Ad-ActA/ActRFc-T) protocol as outlined in Figure 1A. As control, equal amounts of recombinant Fc fragment of human IgG1 were injected in Ad-ActA-treated mice (Ad-ActA/Fc-P and Ad-ActA/Fc-T, respectively). A) Lung compliance and tail bleeding time were analysed as described in the examples. B) Representative H&E stained sections from mice that received prophylactic or therapeutic administration of ActRIIB-Fc or control human IgG1-Fc proteins. C) mRNA levels of activin-A, SpC, IL-6, Collagen I, TF and Argl. Values are expressed as mean ± SEM of 8 mice per group compared using one-way analysis of variance (ANOVA) with Bonferonni's post-hoc test analysis. *P<0.05, **P<0.01 and ***P<0.001
**Figure 16****. Prophylactic and therapeutic neutralisation of activin-A attenuates LPS-induced Acute Lung Injury (ALI) in a murine model**
   ActRIIB-Fc recombinant protein was injected i.p. (200 µg/injection) in mice treated by intratracheal instillation of LPS (2.5 mg/kg) following a prophylactic (LPS/ActRFc-P) or therapeutic (LPS/ActRFc-T) protocol as outlined in Figure 16A. As control, equal amounts of recombinant Fc fragment of human IgG1 were injected in Ad-ActA-treated mice (LPS/Fc-P and LPS/Fc-T, respectively). Activin-A protein levels, lung compliance and tail bleeding time were analysed as described in the examples. Values are expressed as mean ± SEM of the indicated number of mice derived from two independent experiments and compared using one-way analysis of variance (ANOVA) with Bonferonni's post-hoc test analysis. *P<0.05, **P<0.01 and ***P<0.001.

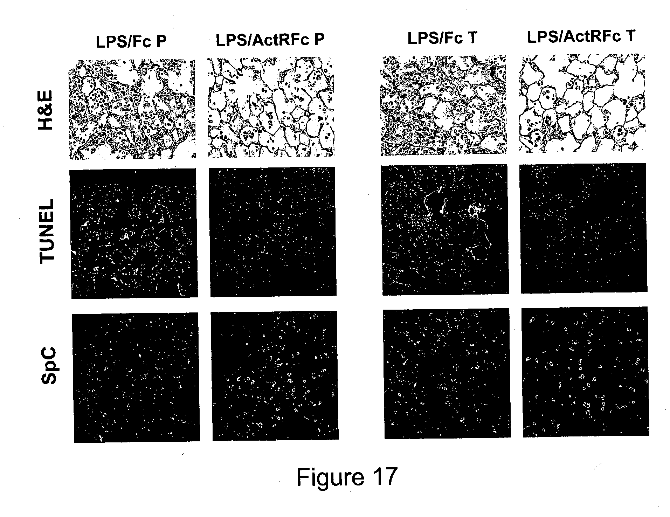

**Figure 17****. Prophylactic and therapeutic neutralisation of activin-A attenuates LPS-induced tissue injury and surfactant protein down-regulation in a murine model**
   Representative H&E, TUNEL and SpC stained tissue sections from LPS-treated mice treated with ActRIIB-Fc or control human IgG1-Fc. TUNEL and SpC immuno-staining was pseudo-coloured white.
**Figure 18****. Inflammatory response induced by intratracheal instillation of LPS in a murine model**
   ActRIIB-Fc recombinant protein was injected i.p. (200 µg/injection) in mice treated by intratracheal instillation of LPS (2.5 mg/kg) following a prophylactic protocol as outlined in Figure 8A and mice were analysed 2, 4 and 7 days post-LPS-instillation. An additional group of mice was treated following the therapeutic protocol outlined in Figure 16 and analysed on day 4 post-LPS-instillation. A) Total number of inflammatory cells in the BAL fluids and numbers of macrophages, neutrophils and lymphocytes were estimated at each time point. White bars represent untreated mice, grey and black bars represent mice which had received LPS and were treated with the control human Fcγ1 or the ActRIIB-Fc protein, respectively. On days 2 and 4, five untreated (n = 5), seven Fcγ1-treated (n = 7) and seven ActRIIB-Fc-treated mice (n = 7) were analysed. On day 7, five untreated (n = 5), three Fcγ1-treated (n = 3) and four ActRIIB-Fc-treated mice (n = 4) were analysed. Values are expressed as mean ± SEM compared at each time point with one-way analysis of variance (ANOVA) with Bonferonni's post-hoc test analysis. **P<0.01 and ***P<0.001. B) Protein levels for IL-6, TNF-α, IL-5 and MCP-1 in BAL samples from mice treated either prophylactically or therapeutically were measured in mice terminated on day 4 post-LPS-instillation, as described in the examples. Values are expressed as mean ± SEM of the indicated number of mice derived from two independent experiments and compared using one-way analysis of variance (ANOVA) with Bonferonni's post-hoc test analysis. **P<0.01 and ***P<0.001.

Other objectives and advantages of the present invention will become apparent to those skilled in the art in view of the following detailed description.

### DEFINITIONS

As used herein, the term "host defence response" ("HDR") refers to an interactive network of simultaneously activated pathways that act in synergy to increase a subject's chance of survival when said subject's homeostatic balance, in a tissue, is substantially disturbed either due to infection, tissue injury or tissue malfunction. Among the processes that comprise the HDR, particularly important to the method of the present invention, are (a) the activation of the coagulation and complement cascades, (b) the activation of the innate and adaptive immunity, and (c) the activation of the tissue repair processes.

As used herein, the term "aberrant host defence response activation" ("aberrant HDR activation") refers to the situation wherein pronounced or protracted activation of the HDR causes collateral tissue injury and contributes to development of pathology. Said activation of the HDR is evident in affected tissues by the presence of above-basal levels of endogenous activin (activin-A, activin-B or activin-AB) and development of said pathology is characterised, typically, by enhanced cell death, and / or development of a pro-coagulant state, and / or induction of an inflammatory response (in particular increased presence of neutrophils) and high levels of pro-inflammatory cytokines (including IL-6, IL-1, IL-17 and TNFα), and / or increased presence of PAMPs (including bacterial endotoxin (lipopolysacharides (LPSs)), peptidoglycan, microbial unmethylated CpG-DNA and viral double-stranded RNA) and / or DAMPs (including HMGB1, heat shock proteins (HSPs), purine metabolites, such as ATP, and uric acid). For respiratory diseases down-regulation of surfactant protein synthesis is an additional indicator of said aberrant HDR activation.

As used herein, the term "subject" refers to a mammal and includes, but is not limited to, a human or non-human mammal such as bovine, equine, canine, feline or ovine.

A used herein, the terms "treatment", "therapy" and "prophylaxis" are to be considered in their broadest context. As used herein, the term "treatment" refers to management and care of a subject or the combating of disease. The term "therapy" refers to treatment of an established disease by some remedial, rehabilitating or curative process. The term "prophylaxis" refers to treatment for preventing the development or reducing the severity of a particular disease or condition. The term "treatment" does not necessarily imply that a subject is treated until total recovery. Similarly, "therapy" and "prophylaxis" does not necessarily mean that the subject will not eventually contract a disease condition. Accordingly, "treatment" and "prophylaxis" include amelioration of the symptoms of a particular condition or preventing or otherwise reducing the risk of developing a particular condition.

As used herein, the term "disease" is to be considered in its broadest sense, including a disease condition, and refers to any deviation from or interruption of the normal structural function of any body part, organ, or system that is manifested by a characteristic set of symptoms and signs and whose etiology, pathology and prognosis may be known or unknown. Diseases included in the scope of this definition are diseases associated with aberrant HDR activation, examples of which are selected from the group consisting of: (a) acute injury of tissues inflicted by infection, toxic substances or trauma, heart infarction, wound healing pursuant to surgery, severe bums or other tissue injury, meningitis, appendicitis, renal tubular necrosis, traumatic brain injury and sepsis; (b) autoimmune diseases including, but not limited to, Rheumatoid arthritis, multiple sclerosis, inflammatory bowel disease, vasculitis, anti-phospholipid Syndrome, scleroderma, Systemic lupus erythematosous and osteoarthritis; and (c) respiratory diseases including, but not limited to, severe asthma, interstitial fibrosis, organising peumonia, non-specific interstitial pneumonia, sarcoidosis, cystic fibrosis, lung transplantation, bronchiolitis obliterans, pulmonary hypertension, pneumonia, ALI/ARDS, COPD, acute responses against infectious agents such as pathogenic viruses (including, but not limited to, influenza A viruses H5N1 and H1N1, coronaviruses SARS, and human rhinoviruses C and D).

As used herein, the term "activin" encompasses activin-A, activin-B and activin-AB, as well as variants and species homologues of these proteins. Activin-A is the homodimer of the protein chains βA and activin-AB is a heterodimer of βA and βB. As used herein, the term "activin-A" refers to the activin protein possessing accession number: NM_002192.2. Activins B, and AB are a homodimer and heterodimer, respectively, of two closely related β subunits (β_{B}β_{B} and β_{A}β_{B}). As used herein, the term "activin-B" refers to the activin protein possessing accession number: NM_002193.2.

As used herein the term "activin receptor" refers to membrane protein kinases belonging to the family of protein-serine-threonine-kinases. Activin receptors consist of two different, but related, protein kinases, type-I and type-II. Activin initiates cellular signal transduction by first binding to the type-II receptors (activin receptor type-II (ActRII)) which then recruit and phosphorylate the type-I receptors (activin receptor type-I (ActRI)). The phosphorylation of the type-I receptor leads to activation of its kinase catalytic activity. The activated type-I receptor initiates numerous intracellular signalling pathways, including the pathway mediated by the Smad proteins.

As used herein, the term "activin type-I receptor" ("ActRI") refers to one of the two types of activin receptors or activin receptor-like kinases (ALKs). There are several type-I activin receptors. The most important type-I activin receptors are ActRIA and ActRIB.

As used herein, the term "ActRIA" (or "ALK-2") refers to the human activin receptor possessing accession number: Z22534.1.

As used herein, the term "ActRIB" (or "ALK-4") refers to the human activin receptor possessing accession number: Z22536.1.

As used herein, the term "activin type-II receptor" ("ActRII") refers to one of the two types of activin receptors. Activin type-II receptors are membrane protein kinases belonging to the family of protein-serine-threonine-kinases. The most important type-II activin receptors are ActRIIA and ActRIIB.

As used herein, the term "ActRIIA" refers to the human activin receptor possessing accession number: BC069707.

As used herein, the term "ActRIIB" refers to the human activin receptor possessing accession number: BC096243.

As used herein, the term "polypeptide" refers to a polymer made up of a plurality of amino acids linked together by peptide bonds.

As used herein, the term "activin neutraliser" encompasses any molecule or chemical entity (including assemblages, complexes and the like) that is capable of inhibiting or reducing the biological activity of activin *in vivo* and / or *in vitro* and thus enables partial or complete treatment of abarrent HDR activation *in vivo.* The term includes any molecule or chemical entity that binds to activin or its receptors and prevents association between them, or inhibits the catalytic activity of the activin receptors (type-I or type-II) and blocks downstream signalling. The term also includes any molecule or chemical entity that interferes with any of the other downstream components of activin signal transduction pathway, examples of which are Smad2, Smad3 or Smad4.

As used herein, the term "soluble ActRII polypeptide" includes any naturally occurring extracellular domain of an ActRII protein as well as any variants thereof (including mutants, fragments and peptidomimetic forms) that retain a useful activity, i.e. adequate binding affinity to activin so that when produced as soluble fusion protein with, for example, the Fc-portion of a human immunoglobulin molecule, can bind in solution to activin, prevent its association with activin receptors, and hence neutralise the biological activity of activin.

As used herein, the term "fragment" of a compound, with reference to polypeptides or proteins, is a compound possessing qualitative biological activity in common with for example, a full-length polypeptide or protein from which it is derivable.

With reference to nucleic acids, nucleotides and / or polynucleotides, the term "fragment" relates to compounds including, for example: portions of a target nucleic acid sequence which encode a product having qualitative biological activity in common with, for example, a full-length polypeptide derivable from the full length nucleic acid sequence, or fragments of a target nucleic acid sequence which are suitable as specific probes or PCR primers for the detection and / or amplification of the target nucleic acid sequence, or a functional product-encoding portion thereof.

As used herein, the term "analogue", with reference to a nucleic acid sequence, means a sequence which is a derivative of a target nucleic acid sequence, the derivative comprising addition, deletion, or substitution (including conservative and non-conservative amino acid substitutions) of one or more bases and wherein the encoded polypeptide retains substantially the same function as the polypeptide encoded by the target nucleic acid molecule. Similarly, the term "analogue" as used herein refers to a derivative of a target polypeptide comprising addition, deletion, or substitution of one or more amino acids, the analogue retaining, however, substantially the same function as the target or original polypeptide. Similarly, the term "analogue" encompases variants of a polypeptide produced by alternative mRNA splicing (e.g. follistatin 315, follistatin 288, etc).

As used herein, the term "conservative amino acid substitutions" refers to the interchangeability of residues having similar side chains. For example, a group of amino acids having aliphatic side chains includes glycine, alanine, valine, leucine and isoleucine; a group of amino acids having aliphatic-hydroxyl side chains includes serine and threonine; a group of amino acids having amide-containing side chains includes asparagine and glutamine; a group of amino acids having aromatic side chains includes phenylalanine, tyrosine and tryptophan; a group of amino acids having basic side chains includes lysine, arginine and histidine; and a group of amino acids having sulfur-containing side chains includes cysteine and methionine.

As used herein, the term "non-conservative amino acid substitutions" refers to the interchangeability of residues having different side chains.

As used herein, the term "operably linked" refers to the situation wherein, for example, a nucleic acid is placed into a functional relationship with another nucleic acid sequence. For example, a promoter operably linked to a heterologous DNA, which encodes a protein, promotes the production of functional mRNA corresponding to the heterologous DNA.

As used herein, the term "follistatin" protein encompasses different variants of the follistatin protein possessing accession number: NM_006350.3, as exemplified by follistatin FS315 possessing accession number: UPI000012AC56, follistatin FS288 possessing accession number: UPI000002A9EE and follistatin FS303 possessing accession number: UPI000002A9EF.

As used herein, the term "follistatin-like 3" protein refers to a secreted glycoprotein of the follistatin-module-protein family possessing accession number: NM_005860.2.

As used herein, the term "antibody" refers to an immunoglobulin molecule able to bind to a specific epitope on an antigen (on an activin or an activin receptor), and which may be comprised of a polyclonal mixture, or be monoclonal in nature. Antibodies may be entire immunoglobulins derived from natural sources, or from recombinant sources. An antibody according to the present invention may exist in a variety of forms including, for example, whole antibody, an antibody fragment, or another immunologically active fragment thereof, such as a complementarity determining region. Similarly, the antibody may be an antibody fragment having functional antigen-binding domains, that is, heavy and light chain variable domains. The antibody fragment may also exist in a form selected from the group consisting of: Fv, F_{ab} F(ab)₂, scFv (single chain Fv), dAb (single domain antibody), bi-specific antibodies, diabodies and triabodies.

As used herein, the term "cytokine" refers to a protein released by cells that has a specific effect on the interactions between cells, on communications between cells or on the behavior of cells, thriggering among others inflammation, activation of the coagulation pathway and other tissue responses. The cytokines include the interleukins, the lymphokines, the adipokines and the chemokines.

As used herein, the term "therapeutically effective amount" includes a sufficient, but nontoxic amount of an activin neutraliser composition of the invention to provide the desired therapeutic effect. The "effective amount" will vary from subject to subject depending on one or more of a number of factors amongst, for example, the particular agent being administered, the severity of the condition being treated, the species being treated, the age and general condition of the subject and the mode, and route of administration. For any given case, an appropriate "effective amount" may be determined by one of ordinary skill in the art using only routine experimentation. Typically, "therapeutically effective amount" refers to an amount sufficient to result in one or more or the following: recession / reduction in the extent of the disease, inhibition of disease growth or progression, cessation of disease growth, relief of disease-imposed discomfort or prolongation of life of the subject having the disease or condition.

As used herein, the term "prophylactically effective amount", includes a sufficient, but nontoxic amount of an activin neutraliser composition of the invention to provide the desired therapeutic effect. The "effective amount" will vary from subject to subject depending on one or more of a number of factors amongst, for example, the particular agent being administered, the severity of the condition being treated, the species being treated, the age and general condition of the subject and the mode, and route of administration. For any given case, an appropriate "effective amount" may be determined by one of ordinary skill in the art using only routine experimentation. Typically, "prophylactically effective amount" refers to an amount sufficient to result in preventing the development of, or reducing the severity of, a particular disease or condition.

### DETAILED DESCRIPTION OF THE INVENTION

Hereinafter, the best mode for carrying out the present invention is described in detail.

### 1 Overview

The present invention was made by the discovery that an activin neutraliser, as exemplified by ActRIIB-Fc, is a useful therapeutic agent for the prophylaxis and / or therapy of diseases associated with aberrant HDR activation in a subject. Typically, these diseases involve abnormally elevated expression of activin. The results disclosed herein support the position that administration of an activin neutraliser inhibits or reduces pathology associated with aberrant HDR activation.

In a series of experiments designed to validate the consequences of a deregulated production of activin-A in the respiratory system *in vivo* applicants expressed activin-A in mouse airways using adenoviral-mediated gene transfer and monitored functional and structural alterations in the respiratory system. Applicant's experimental data demonstrate that sustained up-regulation of activin-A, at levels comparable with those measured in patients with ARDS, induces severe and continuously evolving respiratory pathology. The process begins with a wave of acute alveolar cell death and is followed by waves of inflammatory cells and soluble mediators. A high level of HMGB1, the archetypal DAMP protein, accompanies pathology through its entire duration, possibly contributing to the chronicity of the response. A systemic pro-coagulant state develops that correlates with up-regulation in TF mRNA levels. Additionally, upregulation of activin-A causes reduction of Type-1 pneumonocytes and levels of SpC, SpB and SpA, and substantial alteration in the composition of airway-lining cells with ciliated epithelium dominating in the distal airways at the expense of Clara cells. The later phase of the activin-A-induced response is characterised by a decline in production of pro-inflammatory cytokines and an increase in expression of anti-inflammatory cytokines, a shift of macrophages towards the "alternative activated" phenotype and induction of a transient fibrotic response. Clearance of collagen deposits that correlates with a shift in protease/anti-protease balance in favour of the former and, eventually, development of emphysematous lesions is the final morphological phenotype. A striking reduction of lung compliance, not fully restored at four months after viral-instillation, parallels the inflammatory and histological changes.

The severe early tissue-damage, the chronic up-regulation of HMGB1 expression, the mobilisation of neutrophils and macrophages, the profile and kinetics of the induced cytokines, the reduction of SpC, SpB and SpA expression, the alveolar edema and the deposition of hyaline membranes on alveolar walls, the transient fibrotic response and the eventual accumulation of emphysematous lesions that compose the activin-A-induced pathology are also recognised in the pathophysiology of human ALI/ARDS (Meduri 1996). This resemblance combined with detection of substantially increased levels of activin-A in BAL fluids from ARDS patients (Figure 14) demonstrates that activin-A plays a key role in the pathophysiology of this disease. Applicants discoveries complement earlier studies that have postulated a role for activin-A in the pathophysiology of sepsis (Khadaroo and Marshall 2002; Costa, Schettino et al. 2006; Jones, Mansell et al. 2007). Both represent typical examples of severe pathology caused by excessive and inappropriately controlled HDR activation. A wide variety of precipitating causes for ARDS are recognised including pneumonia and aspiration of gastric contents; however, severe sepsis is the leading cause of ARDS (Bernard, Artigas et al. 1994; Tsushima, King et al. 2009). This "cause-effect" relationship could stem from the mobilisation of similar, potentially harmful, effector mechanisms during their development, with activin-A being one of them.

Applicants experiments have unveiled novel activin-A-mediated functions that mechanistically explain its involvement in ARDS pathophysiology; namely, infliction of severe tissue injury through alveolar epithelial and endothelial cell death, reduction of surfactant-protein synthesis, alteration in the cellular composition of the airways, activation of the coagulation system and sustained induction of HMGB1 expression. The novel activities of activin-A described herein contribute to development of respiratory pathology in several ways. Thus, the transient wave of cell death and the sustained up-regulation of HMGB1 expression that follows are pathogenic. Passive release of HMGB1 by ruptured apoptotic cells and active release of additional HMGB 1 from infiltrating inflammatory cells combined with high levels of several pro-inflammatory cytokines that are produced create a highly pro-inflammatory environment during the early phases of activin-A-induced response (Bianchi 2009). High levels of HMGB1 have been detected in patients and experimental models of sepsis and, more importantly, neutralisation of HMGB1 in the animal models ameliorated pathology (Wang, Zhu et al. 2008). Induction of TF-expression and development of a systemic pro-coagulant state is another way by which activin-A contributes to pathophysiology. Up-regulation of TF in alveolar epithelial cells (Bastarache, Wang et al. 2007) and neutrophil-derived, TF-mediated, increased pro-coagulant activity has been previously described in ARDS patients (Kambas, Markiewski et al. 2008). Finally, the substantial inhibitory effect of activin-A on expression of SpC, SpB and SpA is of particular interest and provides an additional mechanism that accounts for its pathogenic action *in vivo.* SpC-deficient animals develop pneumonitis and emphysema, and families with hereditary interstitial pulmonary disease carry mutations in the SpC gene (Nogee, Dunbar et al. 2001; Glasser, Detmer et al. 2003). Remarkably, analysis of BAL samples from human ARDS patients revealed a similar pattern of surfactant protein expression, with SpC, SpB and SpA being selectively down-regulated and SpD being unaffected (Greene, Wright et al. 1999).

As a "master regulator" of the HDR, in addition to its effects on the immune system, activin-A affects other resident cells of the lung. Up-regulation of activin-A in the airways leads to substantial alterations in the composition and functionality of airway and alveolar epithelial cells (Figures 11 & 12). Interestingly, the opposite is also true since inhibition of homeostatic activin-A levels by instillation of follistatin-expressing adenoviruses in the airways of C57BL/6 mice leads to increased expression of CC10 and SpC. Thus, activin-A could play a dual function at the interphase of homeostasis and immunity, acting both as "guardian" and as "remediator" of homeostasis. Basal activin-A levels could contribute to a fine-tuning of the homeostatic balance; regulating, for example, the level of CC10 and SpC production. In contrast, high levels of activin-A, produced in response to infection, tissue injury or tissue malfunction, could function as a "red-alert"-like signal mobilising, as needed, the HDR.

A surprise discovery of the present invention is the novel demonstration of the attenuation of respiratory pathology in a mammal after systemic prophylactic and, even more so, therapeutic neutralisation of activin-A.

The present invention shows the therapeutic benefit of activin-A's neutralisation as demonstrated in the exogenous (Ad-ActA-mediated) and the endogenous (LPS-induced) activin-A-dependent models of ALI/ARDS. Even after tissue injury had been inflicted and the inflammatory response had been mobilised; neutralisation of activin-A still normalised all the pathology associated parameters. These results disclosed herein demonstrate that activin-A-induced responses do not progress autonomously but, rather, depend on the continuous presence of activin-A in the affected environment and, therefore, the contribution of activin-A to disease pathophysiology can be neutralised therapeutically. Therefore, applicants experimental data demonstrate that neutralisation of activin-A can reverse the complex and destructive cascade of aberrant HDR activation in a subject and, thus, provide a therapeutic strategy for effective prophylaxis and / or therapy of diseases associated with said aberrant HDR activation.

### 2 Activin neutralisers

Examples of activin neutralisers which are useful for the prophylaxis and / or therapy of diseases associated with aberrant HDR activation are described in detail below. These examples are not to be construed as limiting the scope of the invention in any manner. Any molecule or chemical entity (including assemblages, complexes and the like) capable of inhibiting or reducing the biological activity of activin *in vitro* and *in vivo,* as described herein, is a useful therapeutic agent for the prophylaxis and / or therapy of diseases associated with aberrant HDR activation in a subject.

### 2.1 ActRII polypeptides

In certain aspects, activin neutralisers of the present invention include soluble ActRII polypeptides, or a fragment(s) or analogue thereof. The presently disclosed soluble ActRII polypeptides, or a fragment(s) or analogue thereof, possess pharmacokinetic properties that makes them suitable as therapeutic agents for the prophylaxis and / or therapy of diseases associated with aberrant HDR activation in a subject.

A soluble ActRII polypeptide, or a fragment(s) or analogue thereof, may include one or more alterations in the amino acid sequence (e.g. in the ligand-binding domain) relative to a naturally occurring ActRII polypeptide, or a fragment(s) or analogue thereof. The alteration in the amino acid sequence may, for example, alter glycosylation of the polypeptide when produced in a mammalian, insect or other eukaryotic cell or alter proteolytic cleavage of the polypeptide relative to the naturally occurring ActRII polypeptide, or a fragment(s) or analogue thereof. Optionally, a soluble ActRII polypeptide, or a fragment(s) or analogue thereof, includes one or more modified amino acid residues selected from: a glycosylated amino acid, a PEGylated amino acid, a farnesylated amino acid, an acetylatedamino acid, a biotinylated amino acid, an amino acid conjugated to a lipid moiety, and an amino acid conjugated to an organic derivatising agent.

A soluble ActRII polypeptide may be a fusion protein having at least a portion of the ActRII polypeptides and one or more fusion domains. Well known examples of such fusion domains include, but are not limited to, an immunoglobulin heavy chain constant region (e.g. an Fc), polyhistidine, Glu-Glu, glutathione S transferase (GST), thioredoxin, maltose binding protein (MBP), or human serum albumin A fusion domain may be selected so as to confer a desired property. For example, some fusion domains are particularly useful for isolation of the fusion proteins by affinity chromatography. For the purpose of affinity purification, relevant matrices for affinity chromatography, such as glutathione-,amylase-, and nickel- or cobalt-conjugated resins are used. Many of such matrices are available in "kit" form, such as the Pharmacia GST purification system and theQIAexpres™ system (Qiagen) useful with (HIS₆) fusion partners. As another example, a fusion domain may be selected so as to facilitate detection of the ActRII polypeptides. Examples of such detection domains include the various fluorescent proteins (e.g. GFP) as well as "epitope tags", which are usually short peptide sequences for which a specific antibody is available. Well known epitope tags for which specific monoclonal antibodies are readily available include FLAG, influenza virus haemagglutinin (HA), His and c-myc tags. In some cases, the fusion domains have a protease cleavage site, such as for Factor Xa or Thrombin, which allows the relevant protease to partially digest the fusion proteins and thereby liberate the recombinant proteins there from. The liberated proteins can then be isolated from the fusion domain by subsequent chromatographic separation. In certain preferred embodiments, an ActRII polypeptide is fused with a domain that stabilises the ActRII polypeptide *in vivo* (a "stabiliser" domain). By "stabilising" is meant anything that increases serum half-life, regardless of whether this is because of decreased destruction, decreased clearance by the kidney, or other pharmacokinetic effect. Fusions with the Fc portion of an immunoglobulin are known to confer desirable pharmacokinetic properties on a wide range of proteins. Likewise, fusions to human serum albumin can confer desirable properties. Other types of fusion domains that may be selected include multimerising (e.g. dimerising and tetramerising domains and functional domains (that confer an additional biological function, such as further neutralisation of activin).

An activin neutraliser of the present invention is exemplified by a fusion protein comprising an extracellular (e.g. activin-binding) domain (ActRIIB) (SEQ ID NO: 59 and SEQ ID NO: 60) fused to a human Fcγ1 domain (SEQ ID NO: 61 and SEQ ID NO: 62).

It is understood that different elements of the fusion proteins may be arranged in any manner that is consistent with the desired functionality. For example, an ActRII polypeptide may be placed C-terminal to a heterologous domain, or, alternatively, a heterologous domain may be placed C-terminal to an ActRII polypeptide. The ActRII polypeptide domain and the heterologous domain need not be adjacent in a fusion protein, and additional domains or aminoacid sequences may be included C- or N-terminal to either domain or between the domains.

In certain embodiments, the ActRII polypeptides of the present invention contain one or more modifications that are capable of stabilising the ActRII polypeptides. For example, such modifications enhance the *in vitro* half-life of the ActRII polypeptides enhance circulatory half-life of the ActRII polypeptides or reducing proteolytic degradation of the ActRII polypeptides. Such stabilising modifications include, but are not limited to, fusion proteins (including, for example, fusion proteins comprising an ActRII polypeptide and a stabiliser domain), modifications of a glycosylation site (including, for example, addition of a glycosylation site to an ActRII polypeptide), and modifications of carbohydrate moiety (including, for example, removal of carbohydrate moieties from an ActRII polypeptide). In the case of fusion proteins, an ActRII polypeptide is fused to a stabiliser domain such as an IgG molecule (e.g. an Fc domain). As used herein, the term "stabiliser domain" not only refers to a fusion domain (e.g. Fc) as in the case of fusion proteins, but also includes non-proteinaceous modifications such as a carbohydrate moiety, or non-proteinaceous polymer, such as polyethyleneglycol.

In certain embodiments, ActRII polypeptides (unmodified or modified) of the invention can be produced by a variety of art-known techniques. For example, such ActRII polypeptides, or a fragment(s) or an analogue thereof, may be recombinantly produced using various expression systems (e.g. *E. coli,* Chinese Hamster Ovary (CHO) cells, COS cells, baculovirus) as is well known in the art. In a further embodiment, the modified or unmodified ActRII polypeptides may be produced by digestion of naturally occurring or recombinantly produced full-length ActRII polypeptides by using, for example, a protease, e.g. trypsin, thernolysin, chymotrypsin, pepsin, or paired basic amino acid converting enzyme (PACE). Computer analysis (using commercially available software, e.g. MacVector, Omega, PCGene and Molecular Simulation Inc.) can be used to identify proteolytic cleavage sites. Alternatively, such ActRII polypeptides may be produced from naturally occurring or recombinantly produced full-length ActRII polypeptides such as standard techniques known in the art, such as by chemical cleavage (e.g. cyanogen bromide, hydroxylamine and the like).

In addition, activin neutralisers such as ActRII polypeptides, or a fragment(s) or analogue thereof, can be synthesised in solution or *via* solid-phase chemistry in accordance with conventional techniques. Various automatic synthesisers are commercially available and can be used in accordance with known protocols (Stewart and Young, Solid Phase Peptide Synthesis, 2nd.Ed., Pierce Chemical Co. 1984; Tam *et al.* 1983; Merrifield 1986; Barany and Merrifield, The Peptides, Gross and Meienhofer, eds, Academic Press, New York, 1-284; Barany *et al.* 1987).

In certain embodiments, the ActRII polypeptides of the invention may further comprise post-translational modifications in addition to any that are naturally present in the ActRII polypeptides. Such modifications include, but are not limited to, acetylation, carboxylation, glycosylation, phosphorylation, lipidation, and acylation. As a result, the modified ActRII polypeptides may contain non-amino acid elements, such as polyethylene glycols, lipids, poly- or mono-saccharide, and phosphates. Effects of such non-amino acid elements on the functionality of an ActRII polypeptide maybe tested as described herein for other ActRII polypeptides, or a fragment(s) or analogue thereof. When an ActRII polypeptide is produced in cells by cleaving a nascent form of the ActRII polypeptide, post-translational processing may also be important for correct folding and/or function of the protein. Different cells (such as CHO, HeLa, MDCK293, WI38, NIH-3T3 or HEK293) have specific cellular machinery and characteristic mechanisms for such post-translational activities and may be chosen to ensure the correct modification and processing of the ActRII polypeptides.

Methods of purifying activin neutralisers, where these are ActRII polypeptides or a fragment(s) or analogues thereof, are well known to those of skill in the art. These techniques involve, at one level, the crude fractionation of the proteinaceous and non-proteinaceous fractions. Having separated the peptide polypeptides from other proteins the peptide or polypeptide of interest can be further purified using chromatographic and electrophoretic techniques to achieve partial or complete purification (or purification to homogeneity).

Various techniques suitable for use in purification will be well known to those of skill in the art. These include, for example, precipitation with ammonium sulphate, PEG, antibodies (immuno-precipitation) and the like or by heat denaturation, followed by centrifugation; chromatography such as affinity chromatography (Protein-A columns), ion-exchange, gel-filtration, reversed-phase, hydroxyl apatite, hydrophobic-interaction chromatography, isoelectric focusing, gel-electrophoresis, and combinations of these techniques. As is generally known in the art, it is believed that the order of conducting the various purification steps may be changed, or that certain steps may be omitted, and still result in a suitable method for the preparation of a substantially purified polypeptide.

### 2.2 Follistatin and follistatin-like 3 protein

In certain aspects, activin neutralisers of the present invention include follistatin and follistatin-like 3 protein, or a fragment(s) or analogue thereof.

### Follistatin

Follistatin is a monomeric glycoprotein that irreversibly binds activins and blocks their binding to the cognate activin receptors. Thus, follistatin neutralises the actions of activin via autocrine or paracrine effects (Bilezikjian, Blount et al. 2001; Welt, Sidis et al. 2002; Harrison, Gray et al. 2005). The follistatin gene consists of six exons and encodes mainly two protein forms; follistatin 315, a full-length 315 amino acid-containing polypeptide and, another polypeptide, follistatin 288, produced as a result of alternative splicing of intron 5. (Inouye, Guo et al. 1991; Krummen, Woodruff et al. 1993; Sidis, Schneyer et al. 2005). The characteristic feature of follistatin is its ability to bind activin through its three 10-cysteine "follistatin domains". The first "follistatin domain" contains also a heparin binding sequence that facilitates follistatin binding to cell surface heparin sulphated proteoglycans (Inouye, Ling et al. 1992; Sumitomo, Inouye et al. 1995; Wang, Keutmann et al. 2000). The full-length polypeptide follistatin 315 contains a C-terminal acidic tail that inhibits the, heparin binding domain-mediated, follistatin binding to cell surface proteoglycans (Keutmann, Schneyer et al. 2004). In contrast, the spliced variant, follistatin 288 does not contain this C-terminal acidic tail region and does not bind to cell surface proteoglycans (Inouye, Ling et al. 1992; Sumitomo, Inouye et al. 1995; Wang, Keutmann et al. 2000). Follistatin protein, or a fragment(s) or analogue thereof is an effective activin neutraliser and is a useful therapeutic agent for the prophylaxis and / or therapy of diseases associated with aberrant HDR activation in a subject.

### Follistatin-like 3 protein

Follistatin-like 3 protein (FSTL3) is a secreted glycoprotein that was identified due to the sequence homology it shares with follistatin. FSTL3 is encoded by a distinct gene that has one less follistatin domain, but at the protein level shares 45% identity with follistatin (Schneyer, Tortoriello et al. 2001; Tortoriello, Sidis et al. 2001; Sidis, Tortoriello et al. 2002; Schneyer, Schoen et al. 2003; Schneyer, Sidis et al. 2004).

FSTL3 binds irreversibly to activin with almost the same affinity as follistatin does. Thus, there could be some overlapping biological actions of follistatin and FSTL3. However, one important distinction between the two is the lack of the heparin binding sequence in FSTL3 that prevents its binding to cell surface proteoglycans, indicating that FSTL3 might function as follistatin 288 variant. FSTL3, or a fragment(s) or analogue thereof is an effective activin neutraliser and is a useful therapeutic agent for the prophylaxis and / or therapy of diseases associated with aberrant HDR activation in a subject.

Methods of producing activin neutralisers, where these are proteins such as follistatin or follistatin-like 3 protein, or a fragment(s) or analogue thereof, can employ conventional techniques of molecular biology, microbiology, recombinant DNA and immunology, all of which are within the skill of the art and fully explained in anyone of a number of well-known scientific publications, such as: "Molecular Cloning: A Laboratory Manual" Second Edition by Sambrook et al., Cold Spring Harbor Press (1989). For example, the gene for follistatin or follistatin-like 3 protein may be isolated from cells or tissues that express follistatin or follistatin-like 3 protein by: isolating messenger RNA from the tissue or cells; using reverse transcriptase to generate the corresponding DNA sequence; and finally using the polymerase chain reaction (PCR) with the appropriate primers to amplify the DNA sequence coding for the active follistatin or follistatin-like 3 protein amino acid sequence. Also, a polynucleotide encoding a follistatin or follistatin-like 3 protein fragment may be cloned into an appropriate expression vector, and then expressed in a suitable procaryotic, viral or eucaryotic host. Expressed follistatin or follistatin-like 3 protein can be purified according to standard procedures known in the art, including one or more of the following established procedures: protein precipitation, including ammonium sulfate, ethanol or polyethyleneglycol precipitation and immuno-precipitation; chromatographic techniques using ion-exchange, size-exclusion, reversed-phase, hydrophobic interaction, affinity, or immuno-affinity technologies and carried out by, for example, column chromatography, HPLC, or FPLC; electrophoretic techniques such as gel electrophoresis and HPEC; and the like.

For instance, for producing recombinant follistatin or follistatin-like 3 protein, or a fragment(s) or analogue thereof, for use in the present invention, the relevant DNA sequences are inserted into a suitable expression system. Preferably, a recombinant molecule or vector is constructed in which the polynucleotide sequence encoding follistatin or follistatin-like 3 protein is operably linked to a heterologous expression control sequence enabling expression of the follistatin or follistatin-like 3 protein. A number of appropriate expression vectors are known in the art for mammalian (including human) protein expression, and employed using standard molecular biology techniques. Such vectors may be selected from among conventional vector types including insects, such as baculovirus expression, or yeast, fungal, bacterial or viral expression systems.

Suitable host cells or cell lines for transfection in such a method include mammalian cells, such as Human 293 cells, Chinese hamster ovary cells (CHO), the monkey COS-1 cell line or murine 3T3 cells. Similarly bacterial cells such as the various well-known strains of E. *coli* (e.g. HB101, MC1061) and various strains *of B. subtilis, Pseudomonas,* other bacilli and the like are useful as host cells for the present invention. Many strains of yeast cells known to those skilled in the art are also available as host cells for expression of the polypeptides of the present invention. Insect cells such as *Spodoptera frugipedera* (Sf9) cells may also be used.

The vectors containing the DNA segments of interest can be transferred into the host cell by anyone of a number of well-known methods, depending on the type of cellular host. For example, calcium chloride transfection and electroporation are commonly utilised for procaryotic cells. Calcium phosphate treatment, electroporation, lipofection, biolistics or viral-based transfection may be used for other cellular hosts. Methods for transforming mammalian cells may also include the use of transfection, transformation, conjugation, polybrene, liposomes, electroporation, particle gun technology and microinjection (see, generally, Sambrook *et al., supra*).

Recombinant host cells are then advantageously grown in a selective medium, which inherently selects for the growth of those cells containing the introduced vector. The incubation conditions are ideally selected to optimise expression of the recombinant polypeptide.

Therefore, for use in the present invention, recombinant activin neutralisers may be produced by transfecting a host cell with at least one expression vector containing a recombinant polynucleotide encoding an activin neutraliser, such as follistatin or follistatin-like 3 protein, or a fragment(s) or analogue thereof, under the control of a transcriptional regulatory sequence. The transformed cell is then cultured under conditions that allow expression of the follistatin or follistatin-like 3 protein. The expressed protein may then be recovered from the cell or the culture medium, isolated, and optionally purified by appropriate means known to one of skill in the art. For example, the proteins may be isolated in soluble form following cell lysis or may be extracted using known techniques, such as in guanidine chloride.

For example, microbial cells containing the exogenous follistatin or follistatin-like 3 protein gene may be cultured in large volume reactors, collected by centrifugation and then ruptured by, for example, high pressure homogenisation. The resulting cell lysate may be resuspended in an appropriate diluent / buffer and filtered to obtain an aqueous suspension of the follistatin or follistatin-like 3 protein.

Activin neutralisers such as follistatin or follistatin-like 3 protein, or a fragment(s) or analogue thereof, may also be synthesised by methods of solid-phase chemistry well known to those of ordinary skill in the art. For example, follistatin or follistatin-like 3 protein fragments may be synthesised following the solid-phase chemistry procedures of Steward and Young [Steward, J. M. & Young, J. D., Solid Phase Peptide Synthesis. (2nd Edition) Pierce Chemical Co., Illinois, USA (1984)]. In general, such a synthesis method comprises the sequential addition of one or more amino acids or suitably protected amino acids to a growing peptide chain. Typically, functional group(s) other than one of either the amino or carboxyl group of the first amino acid is / are protected by a suitable protecting group. The protected amino acid is then either attached to an inert solid support or utilised in solution by adding the next amino acid in the sequence having the complementary (amino or carboxyl) group suitably protected and under conditions suitable for forming the amide linkage. The protecting group is then removed from this newly added amino acid residue and the next (protected) amino acid is added, and so forth. After all the desired amino acids have been linked, any remaining protecting groups, and if necessary any solid support, is removed sequentially or concurrently to produce the final polypeptide.

Amino acid changes in the activin neutraliser, such as in follistatin or follistatin-like 3 protein, or a fragment(s) or analogue thereof, may be effected by techniques well known to those persons skilled in the relevant art. For example, amino acid changes may be effected by the addition, deletion or substitution of nucleotides (conservative and / or non-conservative), whilst maintaining the proper reading frame. Such modifications in the target polynucleotide may be produced by techniques including random mutagenesis, site-directed mutagenesis, oligonucleotide-mediated or polynucleotide-mediated mutagenesis, deletion of selected region(s) through the use of existing or engineered restriction enzyme sites and the polymerase chain reaction.

The activin neutraliser of the invention may alternatively be produced as a fusion protein. For instance, it may be desirable to produce follistatin or follistatin-like 3 protein fusion proteins, to enhance expression of the protein in a selected host cell, to improve purification, or for use in monitoring the presence of follistatin or follistatin-like 3 protein in tissues, cells or cell extracts. Suitable fusion partners are well known to those of skill in the art and include: immunoglobulin Fc fragments (such as the Fc fragment of human IgG1 or IgG2, or IgG3 or IgG4 antibodies) or other polypeptides that support the necessary dimerisation of the activin neutralising polypeptide.

Methods of purifying activin neutralisers, where these are follistatin or follistatin-like 3 protein, or a fragment(s) or analogue thereof, are well known to those of skill in the art. These techniques involve, at one level, the crude fractionation of the proteinaceous and non-proteinaceous fractions. Having separated the peptide polypeptides from other proteins the peptide or polypeptide of interest can be further purified using chromatographic and electrophoretic techniques to achieve partial or complete purification (or purification to homogeneity).

Various techniques suitable for use in purification will be well known to those of skill in the art. These include, for example, precipitation with ammonium sulphate, PEG, antibodies (immuno-precipitation) and the like or by heat denaturation, followed by centrifugation; chromatography such as affinity chromatography (Protein-A columns), ion-exchange, gel-filtration, reversed-phase, hydroxyl apatite, hydrophobic-interaction chromatography, isoelectric focusing, gel-electrophoresis, and combinations of these techniques. As is generally known in the art, it is believed that the order of conducting the various purification steps may be changed, or that certain steps may be omitted, and still result in a suitable method for the preparation of a substantially purified polypeptide.

### 2.3 Low molecular weight organic compound activin neutralisers

In certain aspects, activin neutralisers of the present invention include compounds encompassing numerous chemical classes, though typically they are organic compounds, preferably low molecular weight organic compounds and their pharmaceutically acceptable salts. The presently disclosed low molecular weight organic compounds possess pharmacokinetic properties that make them suitable as therapeutic agents for the prophylaxis and / or therapy of diseases associated with aberrant HDR activation in a subject. A low molecular weight organic compound of the present invention preferably possess a molecular weight below 2000 amu, and more preferably between 200 and 1000 amu, and most preferably between 250 and 700 amu.

The low molecular weight organic compounds disclosed herein selectively inhibit CTGF-β and activin type-I or type-II receptors and are capable of inhibiting or reducing the biological activity of activin. For example they are capable of interfering with any of the downstream components of the activin signal transduction pathway, for example Smad2 and Smad3 phosphorylation. Although existing low molecular weight compounds do not sufficiently distinguish TGFβ from activin receptors, it is contemplated by applicants that low molecular weight organic compounds that are selective for activin receptors will be developed in the future and these will be even more useful therapeutic agents for the prophylaxis and / or therapy of diseases associated with aberrant HDR activation.

Non-limiting examples of currently available low molecular weight organic compound activin neutralisers comprise triarylimidazole analogues, as described by Callahan *et al.* (Callahan et al. 2002). Typically the triarylimidazole analogue is SB-431542 as described by Inman *et al.* (Inman et al. 2002).

Further non-limiting examples of currently available low molecular weight organic compound activin neutralisers include: 2-(5-benzo[1,3]dioxol-5-yl-2-*tert*-butyl-3*H-*imidazol-4-yl)-6-methylpyridine hydrochloride (371 amu), also known as SB-505124, as described by Byfield (Byfield *et al.* 2004) which is chemically related to SB-431542; 2-phenyl-4-(3-pyridin-2-yl-1H-pyrazol-4-yl) pyridine (298 amu), also known as GW6604, as described by de Gouville *et al.* (de Gouville et al. 2005) and can be prepared as described previously in WO 02/066462; and 6-[2-*tert*-butyl-5-(6-methyl-pyridin-2-yl)-1*H*-imidazol-4-yl]-quinoxaline (343 amu), also known as SB-525334, as described by Grygielko *et al.* (Grygielko et al. 2005).

Typically, the activin neutralisers contemplated herein include compounds from either natural or non-natural sources. Non-natural sources include, for example, synthetic sources. The low molecular weight activin neutralisers contemplated herein may be screened by processes such as natural product screening.

In general, compounds capable of inhibiting or reducing the biological activity of activin are identified from large libraries of both natural product or synthetic (or semi-synthetic) extracts or chemical libraries, according to methods known in the art. Those skilled in the field of drug discovery and development will understand that the precise source of test extracts or compounds is not critical to the screening procedure(s) of the invention. Compounds used in screens may include known compounds (for example, known therapeutic agents used for other diseases). Alternatively, virtually any number of unknown chemical extracts or compounds can be screened. Examples of such extracts or compounds include, but are not limited to, plant-, fungal-, prokaryotic- or animal-based extracts, fermentation broths and synthetic compounds, as well as modification of existing compounds.

Numerous methods are also available for generating random or directed synthesis (e.g. semi-synthesis or total synthesis) of any number of chemical compounds, including, but not limited to, saccharide-, lipid-, peptide- and nucleic acid-based compounds. Synthetic compound libraries are commercially available from Brandon Associates (Merrimack, N.H.) and Aldrich Chemical (Milwaukee, Wis.). Alternatively, libraries of natural compounds in the form of bacterial, fungal, plant and animal extracts are commercially available from a number of sources including Biotics (Sussex, UK), Xenova (Slough, UK), Harbor Branch Oceangraphics Institute (Ft. Pierce, Fla.), and PharmaMar, U.S.A. (Cambridge, Mass.). In addition, natural and synthetically produced libraries are produced, if desired, according to methods known in the art, e.g. by standard extraction and fractionation methods. Furthermore, if desired, any library or compound is readily modified using standard chemical, physical or biochemical methods.

When a crude extract is found to alter (e.g. inhibitor reduce) the activity of activin by at least 10%, 20%, 30%, 40%, 50%, 60%, 70%, 80% or 90%, or more, further fractionation of the positive lead extract is necessary to isolate chemical constituents responsible for the observed effect. Thus, the goal of the extraction, fractionation and purification process is the careful characterisation and identification of a chemical entity within the crude extract that alters (e.g. inhibits or reduces) the activity of activin. Methods of fractionation and purification of such heterogeneous extracts are known in the art.

If desired, compounds shown to be useful as therapeutic agents for the prophylaxis and / or therapy of diseases associated with aberrant HDR activation in a subject are chemically modified, including production of their pharmaceutically acceptable salts, hydrates, solvates and the like, according to methods known in the art.

### 2.4 Antibodies

In certain aspects, activin neutralisers of the present invention comprise antibodies raised against activin-A, activin-B or activin receptors, or immunogenic portions thereof. The presently disclosed antibodies possess pharmacokinetic properties that make them suitable as therapeutic agents for the prophylaxis and / or therapy of diseases associated with aberrant HDR activation in a subject.

An antibody (or fragment thereof) may be raised against activin-A, activin-B or activin receptors, or immunogenic portions thereof using the methods described below. For convenient production of adequate amounts of antibody(s), these may be manufactured by batch fermentation with serum free medium, and then purified *via* a multi-step procedure incorporating chromatography and viral inactivation / removal steps. For example, the antibody may be first separated by Protein A affinity chromatography and then treated with solvent/detergent to inactivate any lipid enveloped viruses. Further purification, typically by size-exclusion, reversed-phase, anion and / or cation exchange chromatographies, may be used to remove residual undesired contaminants such as proteins, solvents/detergents and nucleic acids. The antibody(s) thus obtained may be further purified and formulated into 0.9% saline using gel filtration columns. The formulated bulk preparation may then be sterilised and viral filtered and dispensed.

These antibodies can include but are not limited to; polyclonal, monoclonal, chimeric, single chain, Fab fragments, and a Fab expression library.

A monoclonal antibody refers to an antibody secreted by a single clone of antibody-producing cells and which is mono specific for a particular antigen or epitope. Therefore, a monoclonal antibody displays a single binding affinity for any antigen with which it immuno-reacts.

Activin-A, activin-B or activin receptor antibodies may be raised using methods well known to those skilled in the art. For instance, a monoclonal antibody, typically containing Fab portions, may be prepared using the hybridoma technology described in Antibodies-A Laboratory Manual, Harlow and Lane, eds., Cold Spring Harbor Laboratory, N.Y. (1988). Any technique that provides for the production of antibody molecules by continuous cell lines in culture may be used. Suitable techniques include the hybridoma technique originally developed by Kohler *et al.* (Kohler et al. 1975), the trioma technique, the human B-cell hybridoma technique, and the EBV-hybridoma technique to produce human monoclonal antibodies (Cole et al., in Monoclonal Antibodies and Cancer Therapy, pp. 77-96, Alan R. Liss, Inc., (1985)). Immortal, antibody-producing cell lines can be created by techniques other than fusion, such as direct transformation of B lymphocytes with oncogenic DNA, or transfection with Epstein-Barr virus. See for example, M. Schreier et al. "Hybridoma Techniques" (1980); Hammerling et al. "Monoclonal Antibodies and T-cell Hybridomas" (1981); and Kennett et al. "Monoclonal Antibodies" (1980).

There are also various procedures known in the art which may be used for the production of polyclonal antibodies to activin-A, activin-B or activin receptors, or fragments thereof. For example, one or more host animals can be immunised by injection with the relevant polypeptide, or a derivative (e.g. fragment or fusion protein) thereof. Suitable hosts include, for example, rabbits, mice, rats, sheep, goats, etc.

### 2.5 Antisense nucleic acids, ribozymes, triplex agents, siRNAs and miRNAs

In certain aspects, activin neutralisers of the present invention include antisense nucleic acids, ribozymes, triplex agents, siRNAs and miRNAs. The presently disclosed antisense nucleic acids, ribozymes, triplex agents, siRNAs and miRNAs possess pharmacokinetic properties that make them useful therapeutic agents for the prophylaxis and / or therapy of diseases associated with aberrant HDR activation in a subject.

The presently disclosed activin neutralisers where these are antisense nucleic acids, ribozymes, triplex agents and siRNAs inhibit or reduce the biological activity of activin *in vivo* by targeting activin, activin receptor (s) or activin-signalling-pathway key regulators at the mRNA level.

An activin neutraliser that reduces mRNA expression levels of, for example, activin-A, activin-B, activin type-I receptors (ALK-4 or ALK-2) or activin type-II receptors (ActRIIA or ActRIIB) and consequently reduces expression of activin, such as activin-A, activin-B or activin-AB, or an activin type-I receptor, such as ALK-2 and ALK-4 or a type-II receptor, such as ActRIIA or ActRIIB, will be a useful therapeutic agent for the prophylaxis and / or therapy of diseases associated with aberrant HDR activation.

Where inhibition of expression of activin, activin receptor or activin-signalling-pathway key regulator is desirable for prophylaxis and / or therapy of diseases associated with aberrant HDR activation, inhibitory nucleic acid sequences that interfere with expression of activin, activin receptors or activin-signalling-pathway key regulators at the transcriptional or translational level can be used. The strategy called antisense, antigene or RNA-interference can be applied.

Said strategies utilise, for example, antisense nucleic acids, ribozymes, triplex agents, siRNAs or micro-RNAs (miRNAs) to block transcription or translation of activin, for example activin-A, activin-B or activin-AB, or an activin type-I receptor, including ALK-2 and ALK-4, or a type-II receptor, including ActRIIA or ActRIIB, either by masking the respective mRNA with an antisense nucleic acid or DNA with a triplex agent, by cleaving the nucleotide sequence with a ribozyme or by destruction of the mRNA through a complex mechanism involved in RNA-interference.

Antisense nucleic acids are DNA or RNA molecules or nucleic acid analogues (e.g. hexitol nucleic acids or peptide nucleic acids) that are complementary to at least a portion of a specific mRNA molecule (Weintraub 1990). In the cell, the antisense nucleic acids hybridise to the corresponding mRNA, forming a double-stranded molecule. The antisense nucleic acids interfere with the translation of the mRNA, since the cell will not translate an mRNA that is double-stranded. Antisense oligomers of about 15 nucleotides are preferred, since they are easily synthesised and are less likely to cause problems than larger molecules when introduced into the target cell which produces activin such as activin-A, activin-B or activin-AB, or expresses an activin receptor. Also nucleic acids or analogues, complementary to the translation initiation site, e.g. between -10 or +10 regions of an activin nucleotide sequence, are preferred.

The potency of antisense oligonucleotides for inhibiting activin or an activin receptor may be enhanced using various methods including addition of polylysine, encapsulation into liposomes (antibody targeted, cationic acid, Sendai virus-derived, etc.) or into nano-particles in order to deliver the oligonucleotides into cells. Other techniques for enhancing the antisense capacity of oligonucleotides exist, such as the conjugation of the antisense oligonucleotides for example to "cell penetrating peptides" (Manoharan, Inamati et al. 2002).

Use of, for example, an oligonucleotide or a PNA (peptide nucleic acid) to stall transcription is known as the antigene strategy (e.g. triplex formation). In the case of oligonucleotides, the oligomer winds around double-helical DNA (major groove) forming a three-stranded helix. Therefore, these antigene compounds can be designed to recognise a unique site on a chosen gene and block transcription of that gene *in vivo* (Maher et al. 1991; Helene 1991; Casey and Glazer 2001; Pooga, Land et al. 2001; Nielsen 2000). Antigene oligonucleotides as well as PNAs are easily synthesised by the skilled artisan and are even commercially available.

Ribozymes are molecules possessing the ability to specifically cleave target single stranded RNA in a manner analogous to DNA restriction endonucleases. Through the modification of nucleotide sequences which encode these RNAs, it is possible to engineer molecules that recognise specific nucleotide sequences in an RNA molecule and cleave it (Cech J. Amer. Med. Assn. 1988; 260:3030). A major advantage of this approach is that, because they are sequence-specific, only mRNAs with particular sequences are inactivated.

There are two basic types of ribozymes namely, tetrahymena-type (Hasselhoff 1988) and "hammerhead"-type. Tetrahymena-type ribozymes recognise sequences which are four bases in length, while "hammerhead"-type ribozymes recognise base sequences 11-18 bases in length. The longer the recognition sequence of the ribozyme the greater the likelihood that the sequence will occur exclusively in the target mRNA species. Consequently, hammerhead-type ribozymes are preferable to tetrahymena-type ribozymes for inactivating a specific mRNA species and 18-based recognition sequences are preferable to shorter recognition sequences.

Specific ribozyme cleavage sites within any potential RNA target are initially identified by scanning the target molecule for ribozyme cleavage sites which include the following sequences, GUA, GUU and GUC. Once identified, short RNA sequences of between 15 and 20 ribonucleotides corresponding to the region of the target gene containing the cleavage site may be evaluated for predicted structural features such as secondary structure that may render the oligonucleotide sequence unsuitable. The suitability of candidate targets may also be evaluated by testing their accessibility to hybridization with complementary oligonucleotides, using ribonuclease protection assays.

RNA-interference involves the insertion of small pieces of double stranded (ds) and even single stranded RNA into a cell. If the dsRNA corresponds with a gene in the cell, it will promote the destruction of mRNA produced by that gene, thereby preventing its expression. The technique has been shown to work on a variety of genes, even in human cells and *in vivo.* For example, small interfering RNAs (siRNA), short-hairpin RNAs (shRNA) or vectors expressing such nucleic acids can be applied in the RNA interference strategy in order to inhibit the translation of activin-, activin receptor- or activin-signalling-pathway key regulator-mRNA.

Anti-sense RNA, DNA molecules and analogues, ribozymes, antigene compounds or nucleic acids for RNA interference of the invention may be prepared by any method known in the art for the synthesis of nucleic acid molecules. These include techniques for chemically synthesising oligonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesise antisense RNA constitutively or inducibly, depending on the promoter used, can be introduced stably into cell lines.

MicroRNAs (miRNAs) are small, non-coding, single-stranded RNAs that comprise a new class of gene regulators (Bartel 2004). miRNAs are transcribed from the genome as longer precursor molecules which are cleaved by the ribonuclease Drosha into approximately 70-100 nt long oligonucleotides charactersised by a distinct hair-pin structure. Following nuclear export, this precursor is further cleaved by the RNAse Dicer to yield a 17-25 nt double-stranded oligonucleotide that enters the RNA-induced silencing complex (RISC). RISC is a multi-protein complex that separates the mature strand from the passenger strand and facilitates the interaction of mRNAs with sequences that are complementary to the mature miRNA. RISC loaded with miRNA and the target mRNA inhibits the translation of the mRNA by either a silencing mechanism or by degradation of the mRNA. In most cases, the miRNA and mRNA sequences are partially complementary which enables miRNAs to target a broad but nevertheless a selective set of mRNAs.

The therapeutic application of miRNAs involves two strategies. One strategy is directed toward a gain-of-function and aims to inhibit pathogenic miRNAs by using miRNA antagonists, such as anti-miRs, locked-nucleic acids (LNA) or antagomiRs. The second strategy, miRNA replacement, involves the re-introduction of a pathology suppressing miRNA mimic to restore a loss-of-function (Bader, Brown et al. 2010).

Antisense nucleic acids, ribozymes, triplex agents, siRNAs and miRNAs are easily synthesised by the skilled artisan and are also commercially available.

Also included within the scope of the present invention are prodrugs of activin neutralisers described herein above. Typically, these prodrugs are functional derivatives of activin neutralisers which are readily converted *in vivo* to the required compound for use in the present invention. Typical procedures for the selection and preparation of prodrugs are established and described in available texts such as, for instance, H. Bundgaard (Ed), Design of Prodrugs, Elsevier, 1985.

### 3 Pharmaceutical compositions and dosages

In certain aspects, the activin neutralisers described herein can be used to manufacture a medicament for the prophylaxis and / or therapy of diseases associated with aberrant HDR activation in a subject and, accordingly, the present invention provides pharmaceutical compositions comprising at least one activin neutraliser for the prophylaxis and / or therapy of diseases associated with aberrant HDR activation in a subject. The activin neutraliser compositions of the present invention are administered locally or *via* systemic circulation. Said compositions comprise a therapeutically effective or prophylactically effective amount of at least one activin neutraliser in admixture with pharmaceutically acceptable carriers, adjuvants and / or diluents. A mixure of different activin neutralisers can also be used to manufacture a medicament for the prophylaxis and / or therapy of diseases associated with aberrant HDR activation in a subject.

In certain embodiments, activin neutraliser compositions of the present invention, where these comprise soluble ActRII polypeptides, or a fragment(s) or analogue thereof, follistatin or follistatin-like 3 protein, or a fragment(s) or analogue thereof, or antibodies raised against activin-A, activin-B or activin receptors or immunogenic portions thereof (or any other proteinaceous activin neutraliser), as described above, due to their proteinaceous nature and possible difficulties of being absorbed in the alimentary tract, are to be administered via inhalation, or injection routes of administration.

In certain embodiments, activin neutraliser compositions of the present invention, where these comprise low molecular weight organic compounds, or their pharmaceutically acceptable salts, solvates, hydrates and the like, as described above, are to be administered via oral, inhalation or injection routes of administration.

In certain embodiments, activin neutraliser compositions of the present invention, where these comprise antisense nucleic acids, ribozymes, triplex agents, siRNAs or miRNAs as described above, can be administered by delivery techniques that comprise, amongst others, biochemical microinjection, liposome-mediated delivery, oligonucleotide-conjugates (with groups like cholesterol, cell-penetrating peptides, or ligands for cellular receptors), polyethylenimine-mediated delivery, polymer delivery, the use of biodegradable microparticles and electroporation. The antisense oligonucleotide can be dissolved in the hydrophilic inside of the vesicles and delivered to the cell by fusion of the vesicle with membranes. The delivery process can be improved by conjugation of the antisense oligonucleotide with cholesterol (Bijsterbosch, Manoharan et al. 2001) or by the use of cationic lipids for liposome formulation (Weisman, Hirsch-Lerner et al. 2004), or by the use of ampoteric liposome formulations (Andreakos, Rauchhaus et al. 2009). Dendrimers have also been applied for increasing the uptake of oligonucleotides into cells. PAMAM dendrimers are cationic polymers that have considerable effectiveness in gene transfer or oligonucleotide delivery at the cell (Yoo and Juliano 2000).

The pharmaceutical composition of the present invention may contain materials for modifying, maintaining or preserving, for example, the pH, osmolarity, viscosity, clarity, colour, isotonicity, odour, sterility, stability, rate of dissolution or release, adsorption or penetration of the composition. Suitable materials include, but are not limited to: amino acids (such as glycine, glutamine, asparagine, arginine or lysine); antimicrobials; antioxidants (such as ascorbic acid, sodium sulphite or sodium hydrogen-sulfite); buffers (such as borate, bicarbonate, Tris-HCl, citrates, phosphates, other organic acids); bulking agents (such as mannitol or glycine),chelating agents (such as ethylenediamine tetraacetic acid (EDTA)); complexing agents (such as caffeine, polyvinylpyrrolidone, betacyclodextrin or hydroxypropylbeta-cyclodextrin); fillers; monosaccharides; disaccharides and other carbohydrates (such as glucose, mannose, or dextrins); proteins (such as serum albumin, gelatin or immunoglobulins); colouring; flavouring and diluting agents; emulsifying agents; hydrophilic polymers (such as polyvinylpyrrolidone); low molecular weight polypeptides; salt-forming counter ions (such as sodium); preservatives (such asbenzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (suchas pluronics, PEG, sorbitan esters, polysorbates such as polysorbate 20, polysorbate 80, triton, tromethamine, lecithin, cholesterol, tyloxapal); stability enhancing agents (sucrose or sorbitol); tonicity enhancing agents (such as alkali metal halides (preferably sodium or potassium chloride), mannitol and sorbitol); delivery vehicles; diluents; excipients and / or pharmaceutical adjuvants [Remington's Pharmaceutical Sciences, 18th Edition, A.R. Gennaro, ed., Mack Publishing Company, (1990)].

The optimal pharmaceutical composition of the present invention will be determined by one skilled in the art depending upon, for example, the intended route of administration, delivery format and desired dosage. See for example, Remington's Pharmaceutical Sciences, *supra.* Such compositions may influence the physical state, stability, rate of *in vivo* release and rate of *in* vivo clearance of an activin neutraliser. For example, suitable compositions may be water for injection and physiological saline solution for parenteral administration.

The primary vehicle or carrier in a pharmaceutical composition of the present invention may be either aqueous or non-aqueous in nature. For example, a suitable vehicle or carrier may be water for injection, physiological saline solution or artificial cerebrospinal fluid, possibly supplemented with other materials common in compositions for parenteral administration. Neutral buffered saline or saline mixed with serum albumin are further exemplary vehicles. Other exemplary pharmaceutical compositions comprise Tris buffers, or acetate buffers, which may further include sorbitol or a suitable substitute thereof. In one embodiment of the present invention, compositions may be prepared for storage by mixing the selected composition having the desired degree of purity with optional formulation agents [Remington's Pharmaceutical Sciences, *supra*] in the form of a lyophilised cake or an aqueous solution. Further, the therapeutic composition may be formulated as a lyophilisate using appropriate excipients such as sucrose.

The formulations can be delivered in a variety of methods, for example, by inhalation therapy, orally or by injection. When parenteral administration is contemplated the therapeutic compositions for use in the present invention may be in the form of a pyrogen-free parenterally acceptable aqueous solution comprising the desired activin neutraliser in a pharmaceutically acceptable vehicle. A particularly suitable vehicle for parenteral injection is sterile distilled water in which an activin neutraliser is formulated as a sterile, isotonic, solution properly preserved. Yet another preparation can involve the formulation of the desired activin neutraliser with an agent, such as injectable microspheres, bio-erodible particles, polymeric compounds (polylactic acid and polyglycolic acid), beads or liposomes that provides for the controlled or sustained release of the product which may then be delivered *via* a depot injection. Hyaluronic acid may also be used, and this may have the effect of promoting sustained duration in the circulation. Other suitable means for the introduction of the desired activin neutraliser include implantable drug delivery devices.

In another embodiment, pharmaceutical formulations of the present invention suitable for injectable administration may be formulated in aqueous solutions, preferably in physiologically compatible buffers such as Hanks' solution, Ringer's solution or physiologically buffered saline. Aqueous injection suspensions may contain substances that increase the viscosity of the suspension such as sodium carboxymethyl cellulose, sorbitol ordextran. Additionally, suspensions of the activin neutralisers may be prepared as appropriate oily injection suspensions. Suitable lipophilic solvents or vehicles include fatty oils, such as sesame oil, or synthetic fatty acid esters, such as ethyl oleate, triglycerides or liposomes. Non-lipid polycationic amino polymers may also be used for delivery. Optionally, the suspension may also contain suitable stabilisers or agents to increase the solubility of the activin neutralisers and allow for the preparation of highly concentrated solutions. In another embodiment, a pharmaceutical composition may be formulated for inhalation. Inhalation solutions may also be formulated with a propellant for aerosol delivery. In yet another embodiment, solutions may be nebulised. Pulmonary administration is further described by Labiris *et al.* (Labiris and Dolovich 2003) which describes inhalant delivery devices and drug formulations and WO 94/20069 which describes pulmonary delivery of chemically modified proteins.

It is also contemplated that certain formulations of the present invention may be administered orally. In one embodiment of the present invention, activin neutralisers that are administered in this manner can be formulated with or without those carriers customarily used in the compounding of solid dosage forms such as tablets and capsules. For example, a capsule may be designed to release the active portion of the formulation at the point in the gastrointestinal tract when bioavailability is maximised and pre-systemic degradation is minimised. Additional agents can be included to facilitate absorption of the therapeutic activin neutraliser. Diluents, flavourings, low melting point waxes, vegetable oils, lubricants, suspending agents, tablet disintegrating agents and binders may also be employed. Pharmaceutical compositions for oral administration can also be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the subject.

Pharmaceutical preparations of the present invention for oral use can be obtained through combining activin neutralisers with solid excipients and processing the resultant mixture of granules (optionally, after grinding) to obtain tablets or dragee cores. Suitable auxiliaries can be added, if desired. Suitable excipients include carbohydrate or protein fillers, such as sugars, including lactose, sucrose, mannitol and sorbitol; starch from corn, wheat, rice, potato or other plants; cellulose, such as methyl cellulose, hydroxypropylmethyl-cellulose or sodium carboxymethylcellulose; gums, including arabic and tragacanth; and proteins, such as gelatin and collagen. If desired, disintegrating or solubilising agents may be added such as the cross-linked polyvinylpyrrolidone, agar and alginic acid, or a salt thereof such as sodium alginate.

Dragee cores may be used in conjunction with suitable coatings, such as concentrated sugar solutions, which may also contain gum arabic, talc, polyvinylpyrrolidone, carbopol gel, polyethylene glycol, and / or titanium dioxide, lacquer solutions, and suitable organic solvents or solvent mixtures. Dyestuffs or pigments may be added to the tablets or dragee coatings for product identification or to characterise the quantity of activin neutraliser (i. e. dosage).

Pharmaceutical preparations of the present invention that can be used orally also include push-fit capsules made of gelatin, as well as soft, sealed capsules made of gelatin and a coating, such as glycerol or sorbitol. Push-fit capsules can contain active ingredients mixed with fillers or binders, such as lactose or starches, lubricants, such as talc or magnesium stearate, and, optionally, stabilisers. In soft capsules, the activin neutralisers may be dissolved or suspended in suitable liquids, such as fatty oils, liquid, or liquid polyethylene glycol with or without stabilisers.

Additional pharmaceutical compositions of the present invention will be evident to those skilled in the art, including formulations involving activin neutralisers in sustained- or controlled-delivery formulations. Techniques for formulating a variety of other sustained- or controlled-delivery means, such as liposome carriers, bio-erodible microparticles or porous beads and depot injections, are also known to those skilled in the art. See for example, WO 93/015722 that describes controlled release of porous polymeric microparticles for the delivery of pharmaceutical compositions. Additional examples of sustained-release preparations include semi-permeable polymer matrices in the form of shaped articles (e.g. films or micro capsules). Sustained-release matrices may include polyesters, hydrogels, polylactides (US 3773919, EP-B-0058481), copolymers of L-glutamicacid and gamma ethyl-L-glutamate (Sidman, Steber et al. 1983), poly (2-hydroxyethyl-methacrylate) (Langer *et al.,* 1981; Langer *et al.* 1982), ethylene vinyl acetate (Langer *et al.,* 1981; Langer *et al.* 1982) or poly-D-(-)-3-hydroxybutyric acid (EP-A-0133988). Sustained-release compositions also include liposomes, which can be prepared by any of several methods known in the art. See for example, Eppstein *et al.,* (Eppstein, Marsh et al. 1985), EP-B-0036676, EP-B-0088046 and EP-B-0143949.

The pharmaceutical composition of the present invention to be used for *in* vivo administration typically must be sterile. This may be accomplished by filtration through sterile filtration membranes. Where the composition is lyophilised, sterilisation using this method may be conducted either prior to or following lyophilisation and reconstitution. The composition for parenteral administration may be stored in lyophilised form or in solution. In addition, parenteral compositions generally are placed into a container having a sterile access port, for example, an intravenous solution bag or vial having a stopper pierceable by a hypodermic injection needle.

Once the pharmaceutical composition has been formulated, it may be stored in sterile vials as a solution, suspension, gel, emulsion, solid, or a dehydrated or lyophilised powder. Such formulations may be stored either in a ready-to-use form or in a form (e.g. lyophilised) requiring reconstitution prior to administration.

In a specific embodiment, the present invention is directed to kits for producing a single-dose administration unit. The kits may each contain both a first container having a dried activin neutraliser and a second container having an aqueous formulation. Also included within the scope of this invention are kits containing single and multi-chambered pre-filled syringes (e.g. liquid syringes and lyosyringes).

An effective amount of a pharmaceutical composition of the present invention to be employed therapeutically will depend, for example, upon the therapeutic context and objectives. One skilled in the art will appreciate that the appropriate dosage levels for treatment will thus vary depending, in part, upon the activin neutraliser delivered, the indication for which the activin neutraliser is being used, the route of administration, and the size (bodyweight, body surface or organ size) and condition (the age and general health) of the patient. Accordingly, the clinician may titre the dosage and modify the route of administration to obtain the optimal therapeutic effect. A typical dosage may range from about 0.1 mg/kg to up to about 500 mg/kg or more, depending on the factors mentioned above. Activin neutraliser compositions may be preferably injected or administered intravenously. Long-acting pharmaceutical compositions may be administered every three to four days, every week, or biweekly depending on the half-life and clearance rate of the particular formulation. The frequency of dosing will depend upon the pharmacokinetic parameters of the activin neutraliser in the formulation used. Typically, a composition is administered until a dosage is reached that achieves the desired effect. The composition may therefore be administered as a single dose or as multiple doses (at the same or different concentrations / dosages) over time, or as a continuous infusion. Further refinement of the appropriate dosage is routinely made. Appropriate dosages may be ascertained through use of appropriate dose-response data.

The route of administration of the pharmaceutical composition of the present invention is in accord with known methods, e.g. orally, through injection by intravenous, intraperitoneal, intracerebral (intraparenchymal), intracerebroventricular, intramuscular, intra-ocular,intraarterial, intraportal, intralesional routes, intramedullary, intrathecal, intraventricular, transdermal, or subcutaneous; as well as intranasal, pulmonary, or sublingual means, by sustained release systems or by implantation devices. Where desired, the compositions may be administered by bolus injection or continuously by infusion, or by implantation device.

Alternatively or additionally, the composition may be administered locally via implantation of a membrane, sponge, or another appropriate material on to which the desired activin neutraliser has been absorbed or encapsulated. Where an implantation device is used, the device may be implanted into any suitable tissue or organ, and delivery of the desired molecule may be *via* diffusion, timed-release bolus or continuous administration.

The compositions of the present invention may be used alone or in combination with other therapeutic agents to enhance their therapeutic effects or decrease potential side effects (see combination of therapies below).

### 4 Prophylaxis and / or therapy of disease using activin neutraliser compositions

In certain aspects, the present invention provides methods and pharmaceutical compositions for reducing the amount of, or inhibiting, the biological activity of activin *in vivo* by contacting said activin or activin receptor with an activin neutraliser or neutralising activin, activin receptor(s) or an activin-signaling-pathway key regulator with a low molecular weight organic compound or targeting activin or activin receptor, or activin signalling pathway key regulators at the mRNA level with antisense nucleic acids, ribozymes, triplex agents, siRNAs or miRNAs. Activin neutralisers have a high affinity for activin or activin receptor(s) protein or mRNA and are capable of neutralising the biological activities of activin.

In one aspect, the present invention provides methods for the prophylaxis and / or therapy of diseases associated with aberrant HDR activation in a subject by administering a prophylactically effective or therapeutically effective amount of an activin neutraliser composition to said subject.

Accordingly, the pharmaceutical compositions of the present invention are useful for treating diseases associated with aberrant HDR activation in a subject. Diseases that may be treated by activin neutraliser compositions according to the method of the present invention include, but are not limited to: (a) acute injury of tissues inflicted by infection, toxic substances or trauma, heart infarction, wound healing pursuant to surgery, severe bums or other tissue injury, meningitis, appendicitis, renal tubular necrosis, neurodegenerative diseases, traumatic brain injury and sepsis; (b) autoimmune diseases including, but not limited to, Rheumatoid arthritis, multiple sclerosis, inflammatory bowel disease, vasculitis, anti-phospholipid Syndrome, sclerodemia, Systemic lupus erythematosous and osteoarthritis; and (c) respiratory diseases including, but not limited to, severe asthma, interstitial fibrosis, organising peumonia, non-specific interstitial pneumonia, sarcoidosis, cystic fibrosis, lung transplantation, bronchiolitis obliterans, pulmonary hypertension, pneumonia, ALI/ARDS, COPD, acute responses against infectious agents such as pathogenic viruses (including, but not limited to, influenza A viruses H5N1 and H1N1, coronaviruses SARS, and human rhinoviruses C and D).

In a preferred embodiment, diseases that may be treated by administering a prophylactically effective amount and / or therapeutically effective amount of an activin neutraliser composition to a subject according to the method of the present invention are selected from the group consisting of ALI/ARDS, sepsis and COPD.

The activin neutraliser compositions of the present invention have been demonstrated to be effective in the prophylaxis and / or therapy of diseases associated with aberrant HDR activation in two murine disease models, namely, an adenovirus-instillation (set forth in Example 9) and an LPS-installation-based model (set forth in Examples 10 and 11). Furthermore, the relevance of the present invention to human disease has been substantiated by measuring dys-regulated expression of activin-A in human ARDS subjects and, more importantly, demonstrating a correlation between sustained activin-A expression and survival of ARDS subjects (set forth in Example 8).

According to the method of the present invention, administering a prophylactically effective or therapeutically effective amount of an activin neutraliser composition to a subject inhibits the capacity of activin to induce a hyper-coagulant state in said subject (as demonstrated herein in Examples 7, 9 and 10). Extensive cell death constitutes a key component of the pathophysiology of, and is a desirable therapeutic target for pharmacological intervention of, for example, ARDS, sepsis, COPD, respiratory and autoimmune diseases, acute responses to infectious agents (e.g. viruses, bacteria and fungi), acute responses to toxic substances and wound healing. Normalisation of the coagulation pathway by reducing or inhibiting the biological activity of activin will be beneficial to subjects diagnosed with the aforementioned diseases.

According to the method of the present invention, administering a prophylactically effective or therapeutically effective amount of an activin neutraliser composition to a subject inhibits the capacity of activin to suppress expression of surfactant proteins in said subject (as demonstrated herein in Examples 6, 9 and 10). Reduced expression or function of surfactant proteins constitute a key component of the pathophysiology of, and is a desirable therapeutic target for pharmacological treatment of, for example, ARDS, sepsis, COPD and other respiratory diseases. Normalisation of surfactant protein expression by reducing or inhibiting the biological activity of activin will be beneficial to subjects diagnosed with the aforementioned diseases.

According to the method of the present invention, administering a prophylactically effective or therapeutically effective amount of an activin neutraliser composition to a subject inhibits the capacity of activin to cause acute cell death in tissues in said subject, wherein said activin is aberrantly up-regulated (as demonstrated herein in Examples 4 and 10). Extensive cell death constitutes a key component of the pathophysiology of, and is a desirable therapeutic target for pharmacological treatment of, for example, ARDS, sepsis, COPD, respiratory and autoimmune diseases, acute responses to infectious agents (e.g. viruses, bacteria and fungi), acute responses to toxic substances, wound healing, and protection from the consequences of severe tissue stress. Reducing or inhibiting the biological activity of activin will be beneficial to subjects diagnosed with the aforementioned diseases.

According to the method of the present invention, administering a prophylactically effective or therapeutically effective amount of an activin neutraliser composition to a subject inhibits the capacity of activin to induce pathogenic inflammatory responses in said subject, e.g. reduction of levels of neutrophils, IL-6, TNFα, IL-5 and MCP-1 (as demonstrated herein in Examples 4, 5, 9 and 11). Dys-regulated expression of cytokines, including the aforementioned, constitutes a key component of the pathophysiology of, and is a desirable target for pharmacological intervention of, for example, ARDS, sepsis, COPD, respiratory and autoimmune diseases, acute responses to infectious agents (e.g. viruses, bacteria and fungi), acute responses to toxic substances and wound healing. Normalisation of said cytokine expression by reducing or inhibiting the biological activity of activin will be beneficial to subjects diagnosed with the aforementioned diseases.

### 5 Treatment of disease using activin neutraliser combination therapies

In certain aspects, the present invention contemplates methods for a combination of therapies for the prophylaxis and / or therapy of diseases associated with aberrant HDR activation in a subject. The activin neutraliser composition may be co-administered with at least one additional therapeutic agent which may facilitate the desired prophylactic or therapeutic outcome.

Typically, when ALI/ARDS, sepsis and, even more so, COPD are diagnosed the pathogenic processes associated with aberrant HDR activation have been active for a significant amount of time. Therefore, the present invention contemplates methods for an effective therapy which, in addition to neutralising activin present in the subject at the onset of treatment (reducing further activin-stimulated production of pathogenic mediators such as pro-coagulant, pro-inflammatory and pro-fibrotic mediators and cells), also neutralises pathogenic mediators that have accumulated in the affected tissues induced by activin before the initiation of treatment. Examples of said accumulated pathogenic mediators are pro-coagulant, pro-inflammatory or pro-fibrotic mediators and their respective cells.

Accordingly, the present invention contemplates methods for various combinations of therapies that comprise: (a) neutralisation of activin to attenuate further activin-mediated stimulation of pathogenic mediator synthesis; and (b) neutralisation of already produced harmful mediators by specifically targeting said mediators pharmacologically with a conventional therapeutic approach.

According to the method of the present invention, a combination of therapies comprises administering a prophylactically effective or therapeutically effective amount of an activin neutraliser composition co-administered to a subject with at least one additional therapeutic agent that targets downstream, activin-induced harmful processes. It is contemplated that therapeutic benefits will be achieved by administering lower doses of medicaments which previously targeted individual HDR processes thus, for example, minimising the side effects of, for example, anti-coagulant and anti-inflammatory medicaments.

Examples of a combination of treatments contemplated herein include, but are not limited to: (a) a combined therapy with activin neutralisers and anti-inflammatory therapeutic agents such as corticosteroids. It is contemplated that such treatment will lead to better action of corticosteroids by preventing the development of corticosteroid resistance or increasing their efficacy. This combined therapy will be useful for the prophylaxis and / or therapy of the aforementioned diseases; (b) a combined therapy with activin neutralisers and neutralisers of pro-inflammatory and pro-fibrotic cytokines including, but not limited to, IL-4, IL-5, IL-6, TNF-α, IL-17 and MCP-1. This combined therapy will be useful for the prophylaxis and / or therapy of diseases where aberrant HDR activation is associated with dys-regulated expression of the aforementioned cytokines; (c) a combined therapy with activin neutralisers and agents able to block leukocyte extravasation into inflamed tissues. The movement of leukocytes from the peripheral blood to inflammatory sites is a fundamental component of the HDR activation. Blocking of leukocyte extravasation into affected tissues will help to prevent pathology associated with harmful inflammation. Agents suitable for such combined therapy may include, without being limited to, antibodies or other agents capable of neutralising the interactions of leukocytes with vascular adhesion protein-1 (VAP-1), P-selectin glycoprotein ligand-1 (PSGL-1), CD44 and others. This combined therapy will be useful for the prophylaxis and / or therapy of diseases including but not limited to ALI, ARDS, sepsis and COPD where aberrant HDR activation leads to exaggerated accumulation of harmful inflamatory cells, such as neutrophils, in the affected tissues; (d) a combined therapy with activin neutralisers and neutralisers of DAMPs including, but not limited to, HMGB-1 and TREM-1. This combined therapy will be useful for the prophylaxis and / or therapy of aberrant HDR activation is associated with dys-regulated expression of the aforementioned DAMPs; (e) a combined therapy with activin neutralisers and neutralisers of the coagulation system including, but not limited to, activated protein C (APC), active site inhibited factor VIIa (ASIS), tissue factor pathway inhibitor (TFPI), anti-thrombin (AT), low-molecular weight heparin, vitamin K antagonists and acetylsalicylic acid. This combined therapy will be useful for the prophylaxis and / or therapy of the aforementioned diseases; (f) a combined therapy with activin neutralisers and neutralisers of the complement system including, but not limited to, endogenous soluble complement inhibitors (C1 inhibitor, recombinant soluble complement receptor 1, antibodies) and agents neutralising key proteins of the complement cascade reaction such as, but not limited to, agents neutralising the action of the complement-derived anaphylatoxin C5a, agents interfering with complement receptor 3 (CR3, CD18/11b)-mediated adhesion of inflammatory cells to the vascular endothelium, anti-C5 antibody therapy (eculizumab), anti-C5 receptor antibodies or neutralising polypeptides or low molecular weight inhibitors. This combined therapy will be useful for the prophylaxis and /or therapy of the aforementioned diseases; (g) a combined therapy with activin neutralisers and neutralisers of apoptotic and / or necrotic cell death including, but not limited to, caspase inhibitors. This combined therapy will be useful for the prophylaxis and / or therapy of the aforementioned diseases; (h) a combined therapy with activin neutralisers and surfactant replacement agent for the treatment of respiratory diseases. This combined therapy will be useful for the prophylaxis and / or therapy of respiratory diseases; and (i) a combined therapy with activin neutralisers and therapeutic agents targeting infectious agents including, but not limited to, antibiotics, anti-fungal and anti-viral medicaments. This combined therapy will be useful for the prophylaxis and / or therapy of infectious diseases associated with aberrant HDR activation in a subject.

### EXAMPLES

Hereinafter, the present invention is described in more detail and specifically with reference to the Examples which should not be construed as limiting the scope of the invention in any manner.

### Materials and Methods

### Animals

C57BL/6 female mice were obtained from Charles River Laboratories, Inc., MA, USA and were maintained in individually ventilated cages (IVC) under specific pathogen-free conditions in a temperature-controlled room (23° C ± 1° C) under a 12-hour light / dark cycle and *ad libitum* access to food and water. All procedures had received prior approval from the Institutional and Regional Ethical Review Boards and were in accordance with the US National Institutes of Health Statement of Compliance (Assurance) with Standards for Humane Care and Use of Laboratory Animals (#A5736-01).

### Production of recombinant adenovirus vectors

Adenovectors expressing human activin-A (the mature mouse and human activin-A polypeptides are identical at the amino-acid level) and the green fluorescent protein (pAdTrack-CMV-activin-A/GFP) or the green fluorescent protein only, (pAdTrack-CMV-GFP), referred to hereafter as Ad-ActA and Ad-GFP, respectively, were a kind gift of T. Fotsis (Medical School, University of Ioannina) (Panopoulou, Murphy et al. 2005). Adenoviruses were produced by transfecting HEK293A cells, purified by two rounds of caesium chloride density gradient ultracentrifugation and chromatography using PD-10 Sephadex columns (GE HealthCare Bio-Sciences, Uppsala, SE). Viral aliquots were titred on HEK293A cells using the Adeno-X™ Rapid Titer Kit (Clontech Laboratories). Endotoxin levels in all preparations were lower than 0.1 EU/ml, as demonstrated with the LAL Endotoxin Detection Assay (Cambrex Bio Science).

Applicants used adenoviruses rather than an inducible transgenic expression-system for a number of reasons. Firstly, this approach has been successfully utilised to validate the pathogenic potential of several cytokines and growth factors and has provided valuable information regarding the putative role of these molecules in human respiratory diseases (Sime, Xing et al. 1997) (Sime, Xing et al. 1997) (Bonniaud, Martin et al. 2004) (Fujita, Shannon et al. 2003) (Kolb, Margetts et al. 2001) (Xing, Ohkawara et al. 1997). Secondly, the available systems for lung-specific gene expression are based on the SpC and CC10 promoters, both of which are dramatically down-regulated by activin-A (Figures 11 & 12), and are thus unlikely to support sustained expression of activin-A *in vivo.* Finally, applicants reasoned that the provision of a non-pathogenic pro-inflammatory trigger by the adenovirus particles would simulate, more closely, the conditions under which activin-A is up-regulated *in vivo.*

### Itra-tracheal instillation of recombinant adenovirus

8 × 10⁸ infectious particles of Ad-ActA or control Ad-GFP virus (corresponding to approximately 1.5 × 10⁸ pfus) diluted in 40 µl PBS were instilled intra-trachealy (IT) into anesthetised C57BL/6 female mice, 12 to 16 weeks of age, weighing 20 to 22 g. The dose of 8 × 10⁸ infectious particles was selected for applicants experiments since it allowed the development of a non-lethal and eventually remitting phenotype providing applicants the opportunity to describe the full spectrum of phenotypic changes induced by activin-A.

### Intra-tracheal LPS instillation

C57BL/6 female mice (weight 20 to 25 g) were treated intra-tracheally with LPS (2.5 mg/kg) from *E. coli* 0111:B4 in endotoxin-free PBS, or PBS alone. Mice were analysed 2, 4 and 7 days later.

### In vivo neutralisation of activin-A

For prophylactic or therapeutic neutralisation of the ectopically expressed activin-A, 200 µg/injection/mouse of either a soluble AcRIIB fusion protein or a control human IgG1 Fc fragment (BioXcell) were injected i.p. according to the schemes outlined in Figures 1 & 16.

### Cloning of ActRIIBecd-FcHis6 fusion protein, expression in CHO cells and purification

The human ActRIIB ectodomain was amplified *via* PCR with the following primers (5' to 3') ggactagtaa catgacggcg ccctgg (SEQ ID NO: 59) and ccagatcgcg gtgggggctg tcgg (SEQ ID NO: 60) from a plasmid containing the human ActRIIB sequence (in pCR-Blunt II-TOPO AM2-G17 ActRIIB, I.M.A.G.E. clone #40005760, The IMAGE Consortium). A version of the human IgG1 Fc domain incorporating a C-terminal His6 tag was produced by PCR amplifying the Fc domain (5' to 3') gcagatctaa tcgaaggtcg tggtgatccc aaatcttgtg ac (SEQ ID NO: 61) and tccctgtctc cgggtaaaca ccatcaccat caccattgag cgccgctt (SEQ ID NO: 62) from the pIgPlus expression plasmid. The PCR products were sub-cloned into the pGEM-T easy (Promega) vectors, sequenced and fused before cloning into the expression vector pEFIRES-p (Hobbs, Jitrapakdee et al. 1998). Chinese hamster ovary (CHO) cells were transfected with the ActRIIBecd-FcHis6 expression vector via lipofection (Fugene 6, Roche) and selected with puromycin (Sigma-Aldrich) as previously described (Labiris and Dolovich 2003). During expansion, cells were grown in DMEM supplemented with 2 mmol/l L-glutamine, 100 µl/ml streptomycin, 100 IU/ml penicillin and 10% FCS. DMEM-Hams F-12 (1:1) supplemented with 2 mmol/l L-glutamine, 100 µl/ml streptomycin, 100 IU/ml penicillin, 2.5% BSA and 6.6 ml/l heparin was used as production medium. Cell culture supernatants were clarified by filtration through a 0.22 µm membrane (Steritop, Millipore). NaCl, 1 M, and imidazole, 5 mM, were added into the clarified supernatants and the solution was pumped through a Ni²⁺-loaded HiTrap column (GE Healthcare Life Sciences, Uppsala, Sweden) at 4° C. Protein was eluted with raising imidazole concentrations, dialysed against PBS and finally concentrated with Amicon Ultra concentrator (30000 MWCO, Millipore).

### Measurements of lung mechanics

Mice were anesthetised with intra-peritoneal injection of 0.051 ml/10 g bodyweight of a mixture of ketamine (20 mg/ml, Merial SAS) and xylazine (1.3 mg/ml, Bayer AG). Once surgical anaesthesia had been established the trachea was exposed, cannulated with a blunted metal 18-gauge needle and connected to a computer-controlled small-animal ventilator (FlexiVent, SCIREQ) (Zosky, von Garnier et al. 2004; Collins, Gualano et al. 2005) (Pillow, Korfhagen et al. 2001). The mice were placed on a 37° C heating pad to maintain body temperature during the experiment and ventilated with a tidal volume of 8 ml/kg, at a normal respiratory rate (150 breaths/minute). A positive end-expiratory pressure (PEEP) of 0.2 kPa was established by placing the expiratory line in a water trap (Bates, Schuessler et al. 1997). Each mouse was paralysed with intra-peritoneal injection of 0.05 ml/10 g bodyweight of rocuronium bromide (0.01 mg/ml, N.V. Organon) to suppress spontaneous breathing and allowed to equilibrate on the ventilator. The lung volume history of the mice was standardised prior to measurement of lung mechanics using three deep inflations at total lung capacity (TLC). Interference of the ventilator's mechanical parts or the tracheal cannula to the measurements was eliminated using dynamic calibration as previously described (Tomioka, Bates et al. 2002). The static compliance (Cst), which reflects the stiffness of the lungs, total lung (R_{L}) and Central airway (Rn) resistance which assesses the level of constriction in the lungs were analysed and each individual value represents the average of three replicate measurements.

### Tail bleeding time analysis

Tail vein bleeding time was determined in a blind manner as previously described (Broze, Yin et al. 2001) with some modifications. Briefly, mice were anesthetised and placed prone on the site of a 37° C water bath. A transverse incision was made with a scalpel over a lateral vein, removing 1 cm of the tip of the tail. The tail was immersed in a tube containing normal saline (37° C). The time from the incision to the cessation of bleeding was recorded as the bleeding time.

### Evans Blue leakage method

Microvascular permeability was determined using the Evans Blue dye leakage assay. Briefly, Evans Blue dye (EBD 20 mg/kg, Sigma-Aldrich) was injected into the tail vein 30 minutes before termination of the experiment. Thereafter, thoracotomy was performed and lungs were perfused free of blood with PBS under 60 cm hydrostatic pressure for 10 minutes *via* the right ventricle. Lungs were removed from the thoracic cavity and the trachea and the main stem bronchi and surrounding mediastinal structures were removed. The left lung was weighed and then incubated with PBS (1 volume) and of formamide (2 volumes) for 48 hours at room temperature. The extracted dye was quantitated in a spectrophotometer by measuring absorbance at 620 nm and Evans Blue dye concentration (microgram EBD per lobe) was calculated against a standard curve.

### Mouse BAL samples

Bronchoalveolar lavage (BAL) was obtained by lavaging the airways of mice two times with 0.5 ml PBS via a tracheal cannula. Cells were counted, pelleted onto glass slides and differential cell counts for eosinophils, lymphocyte/mononuclear cells, neutrophils and macrophages was performed on Giemsa-May Grunwald-stained cytospins.

### Histology

Lungs were inflated through tracheotomy to 17 cm hydrostatic pressure with 10% buffered formalin. Lungs were excised en bloc, submersed in 10% buffered formalin overnight and processed for paraffin embedding and sectioning. In some experiments, the right lung lobe was ligated and inflated with formalin and the left lobes were recovered for RNA and protein preparations. 3 to 5 µm histological sections, cut in the sagital plane at the level of the respiratory tree, were loaded onto a poly-Lysine slide, de-paraffinised in xylene and rehydrated in graded ethanol dilutions and distilled H₂O and, thereafter, stained with Haematoxilin & Eosin, and PicroSirius Red for collagen or were used for immuno-staining.

### Immuno-staining

After blocking with normal donkey or goat serum depending on the species of the secondary antibody, tissues were incubated overnight at 4° C with the following primary antibodies: goat anti-CC10 (1 µg/ml; Santa Cruz Biotechnologies), rat anti-CD45 (0,62 µg/ml; Beckton-Dickinson), rabbit anti-SpC (1:1000; Millipore), mouse anti-α smooth muscle actin-Cy3 (1:500; Sigma-Aldrich), Syrian hamster anti-T1α (1:1000; DSHB, University of Iowa, Department of Biology). Chicken-anti GFP (1:500) and rabbit anti-HMGB1 (1:100) were obtained from Abcam.Rat anti-CD68 (2 µg/ml; Serotec), and Mouse anti-FoxJ1 (1:1500; generous gift of Dr. S. Brody, Washington University). Antigen retrieval with 10 mM Sodium Citrate buffer, pH: 6.0 for 30 minutes in a 80° C water-bath followed by 30 minutes incubation on ice was performed before immuno-staining for CC10, CD45, SpC and HMGB1. Antigen retrieval for FoxJ1 immuno-staining was performed in 50 mM Tris pH: 9.5 solution. Chicken Y globulin, rat gamma globulin (Jackson ImmunoResearch Europe Ltd), rabbit and goat gamma globulin (Dako Cytomation) were used as control primary antibodies.

The following secondary antibodies were used: Goat anti-rabbit HRP (Santa Cruz), goat anti-rabbit biotinylated (Vector Labs), donkey anti-goat Texas Red, donkey anti-chicken FITC, donkey anti-rabbit Texas Red (Jackson ImmunoResearch Europe Ltd), goat anti-rat Alexa 488, goat anti-rat Alexa 594, goat anti-mouse Alexa 568, goat anti-Syrian hamster Alexa 568, goat-anti hamster Alexa 488, streptavidin Alexa 569 and goat anti-rabbit 488 (Invitrogen Corporation). After staining, sections were covered with DAPI containing fluorescent mounting medium (Molecular Probes). For immunohistochemistry, development was performed with DAB (DAKO K3468) and tissues were counterstained with Mayer's heamatoxylene dehydrated in series ethanol and mounted with DPX medium (VWR).

Haematoxilin & Eosisn, immunohistochemistry or immunofluorescence images were recorded using a Leica DMR2 microscope equipped with a DFC320 digital camera or a LEICA TCS-SP5 confocal microscope (Leica Microsystems, Wetzlar, Germany). For morphometric analysis 20 to 30 optical fields at 200X final magnification were recorded and used to compute mean group values for each of the stated measurements using the ImageJ 1.41 program. Airways and blood vessels were excluded during the analysis.

### TUNEL staining

*In situ* labelling of fragmented DNA was performed by the TUNEL method using the terminal transferase recombinant kit (Roche Diagnostics GmbH). Briefly, de-paraffinised and rehydrated sections were incubated at room temperature for 10 minutes with the (equilibrium) mixture (devoid of TdT and nucleotides) and then with the TUNEL reaction mixture for 1 hour at 37° C in a humidified chamber. DNA fragments were labelled with fluorescein-conjugated dUTPs (Molecular Probes) using TdT (Roche Diagnostics GmbH). Labelling solution without addition of TdT enzyme was used as a negative control whereas sections pre-treated with DNase (Promega Corporation) were used as positive controls. Labelling reaction was terminate by immersing sections in 1 × SCC solution for 15 minutes at room temperature. Sections were washed (5 × 10 minutes in 1 × PBS) and then counterstained with DAPI for localisation of nuclei. For double staining with TUNEL and antibodies, sections were treated for antigen retrieval then stained for TUNEL and finally immuno-stained with the appropriate antibodies. Incubation time with the primary antibodies in this case was 1 hour at 37° C.

### Activin-A, follistatin, TGFβ and cytokine protein quantification

The levels of activin-A, follistatin and TGFβ were determined using appropriate ELISA kits (R&D Systems) according to the manufacturer's instructions. TGF-β1 evaluation was done before and after acid activation to assess the levels of activated and latent TGF-β1, respectively. Levels of IL-5, IL-6, MCP-1 and TNF-α were determined in the BAL fluids using a MILLIPLEX™ MAP kit (Millipore, Billerica, MA) and a Luminex200 instrument. The assays were performed according to the manufacturer's specifications. Cytokines were quantified using (five-point), five-fold serial diluted, calibration curves constructed from the provided standard. Data analysis was performed using the Luminex 100 IS software version 2.3.182.

### mRNA expression analysis

Total mouse lung RNA was isolated using the TriReagent protocol (Sigma-Aldrich). Quantitative PCR analysis was performed using primer pairs selected using the Beacon Designer v7.01 software (Premier Biosoft International), details of which are set forth below. Data were collected using a Chromo4 RT-PCR detector and analysed with the Opticon Monitor 3 software (Bio-Rad Laboratories). Relative levels of mRNA expression were normalised to GAPDH and calculated according to the ΔΔC_{T} method (Livak and Schmittgen 2001; Schmittgen and Livak 2008). The specificity of the PCR primers was verified by melting curve analysis and direct sequencing of the amplification products.

Primer sequences (5' to 3') and amplicon lengths (quantitative PCR analysis):

| | | | | |
|---|---|---|---|---|
| InhβA | F | agcagacctc ggagatcatc | 136 bp | SEQ ID NO: 1 |
| InhβA | R | ttggggactt ttaggaagag c | 136 bp | SEQ ID NO: 2 |
| eGFP | F | accactacct gagcacccag tc | 110 bp | SEQ ID NO: 3 |
| eGFP | R | gtccatgccg agagtgatcc | 110 bp | SEQ ID NO: 4 |
| TGF-β1 | F | tgacgtcact ggagttgtac gg | 170 bp | SEQ ID NO: 5 |
| TGF-β1 | R | ggttcatgtc atggatggtg c | 170 bp | SEQ ID NO: 6 |
| IL-1β | F | caaccaacaa gtgatattct ccatg | 152 bp | SEQ ID NO: 7 |
| IL-1β | R | gatccacact ctccagctgc | 152 bp | SEQ ID NO: 8 |
| IL-4 | F | acaggagaag ggacgccat | 95 bp | SEQ ID NO: 9 |
| IL-4 | R | gaagccctac agacgagctc a | 95 bp | SEQ ID NO: 10 |
| IL-5 | F | agcacagtgg tgaaagagac ctt | 117 bp | SEQ ID NO: 11 |
| IL-5 | R | tccaatgcat agctggtgat tt | 117 bp | SEQ ID NO: 12 |
| IL-6 | F | gaggatacca ctcccaacag acc | 141 bp | SEQ ID NO: 13 |
| IL-6 | R | aagtgcatca tcgttgttca taca | 141 bp | SEQ ID NO: 14 |
| IL-8 (KC) | F | attgtccaaa agatgctaaa agg | 104 bp | SEQ ID NO: 15 |
| IL-8 (KC) | R | tgtatagtgt tgtcagaagc c | 104 bp | SEQ ID NO: 16 |
| IL-10 | F | ggttgccaag ccttatcgga | 191 bp | SEQ ID NO: 17 |
| IL-10 | R | acctgctcca ctgccttgct | 191 bp | SEQ ID NO: 18 |
| IL-13 | F | agaccagact cccctgtgca | 123 bp | SEQ ID NO: 19 |
| IL-13 | R | tgggtcctgt agatggcatt g | 123 bp | SEQ ID NO: 20 |
| IL-17a | F | gctccagaag gccctcaga | 142 bp | SEQ ID NO: 21 |
| IL-17a | R | agctttccct ccgcattga | 142 bp | SEQ ID NO: 22 |
| TNFα | F | catcttctca aaattcgagt gacaa | 175 bp | SEQ ID NO: 23 |
| TNFα | R | tgggagtaga caaggtacaa ccc | 175 bp | SEQ ID NO: 24 |
| IFNg | F | tcaagtggca tagatgtgga agaa | 92 bp | SEQ ID NO: 25 |
| IFNg | R | tggctctgca ggattttcat g | 92 bp | SEQ ID NO: 26 |
| Fst | F | cgacaatact ctcttcaagt gg | 130 bp | SEQ ID NO: 27 |
| Fst | R | gggtttattc ttcttgttca ttcg | 130 bp | SEQ ID NO: 28 |
| ActRIB | F | cactgacacc atagacattg c | 82 bp | SEQ ID NO: 29 |
| ActRIB | R | gattgtctcg tcaaggactt c | 82 bp | SEQ ID NO: 30 |
| SpC | F | gctccactgg catcgttgtg | 124 bp | SEQ ID NO: 31 |
| SpC | R | gcgaaagcct caagactagg g | 124 bp | SEQ ID NO: 32 |
| SpB | F | ccttgtcctc cgatgttc | 146 bp | SEQ ID NO: 33 |
| SpB | R | cctgttcact ggtgttcc | 146 bp | SEQ ID NO: 34 |
| SpA | F | caaacaatgg gagtcctcag | 107 bp | SEQ ID NO: 35 |
| SpA | R | cacatctctc taatggtatc aaag | 107 bp | SEQ ID NO: 36 |
| SpD | F | aggaatcaaa ggtgaaagc | 109 bp | SEQ ID NO: 37 |
| SpD | R | ctgatagtgg gagaaggc | 109 bp | SEQ ID NO: 38 |
| Arg1 | F | cagaagaatg gaagagtcag | 250 bp | SEQ ID NO: 39 |
| Arg1 | R | cagatatgca gggagtcacc | 250 bp | SEQ ID NO: 40 |
| ColIa1 | F | gagcggagag tactggatcg | 142 bp | SEQ ID NO: 41 |
| ColIa1 | R | gttcgggctg atgtaccagt | 142 bp | SEQ ID NO: 42 |
| ColIIIa1 | F | aggctgaagg aaacagcaaa | 116 bp | SEQ ID NO: 43 |
| ColIIIa1 | R | tagtctcatt gccttgcgtg | 116 bp | SEQ ID NO: 44 |
| TIMP1 | F | ctctggcatc tggcatcc | 162 bp | SEQ ID NO: 45 |
| TIMP1 | R | gctggtataa ggtggtctcg | 162 bp | SEQ ID NO: 46 |
| TIMP2 | F | agaaggagat ggcaagatg | 157 bp | SEQ ID NO: 47 |
| TIMP2 | R | ggaggagatg tagcaagg | 157 bp | SEQ ID NO: 48 |
| MMP2 | F | ttgagaagga tggcaagtat gg | 100 bp | SEQ ID NO: 49 |
| MMP2 | R | tggaagcgga acgggaac | 100 bp | SEQ ID NO: 50 |
| MMP9 | F | tatctgtatg gtcgtggctc taag | 75 bp | SEQ ID NO: 51 |
| MMP9 | R | tgctgtcggc tgtggttc | 75 bp | SEQ ID NO: 52 |
| TF | F | accatttaca aacgccccaa ag | 162 bp | SEQ ID NO: 53 |
| TF | R | acttgccgca gggtgagg | 162 bp | SEQ ID NO: 54 |
| CTGF | F | agcctcaaac tccaaacacc | 185 bp | SEQ ID NO: 55 |
| CTGF | R | caacagggat ttgaccac | 185 bp | SEQ ID NO: 56 |
| GAPDH | F | ccagtatgac tccactcacg | 97 bp | SEQ ID NO: 57 |
| GAPDH | R | ctcctggaag atggtgatgg | 97 bp | SEQ ID NO: 58 |

### In vitro stimulation of MLE12 and MLE15 cells

MLE12 and MLE15 mouse lung epithelial cell lines were obtained from Dr. J.A. Whitsett (Cincinnati Children's Hospital Medical Centre). Cells cultured in DMEM supplemented with 10% FCS, 1% Pen/Strep (Invitrogen Corporation), 0.5% Garamamycin (Schering Plough). Cultures, 70% confluent, were treated for 24 hours with the indicated doses of recombinant human activin-A, follistatin and TGF-β1 (Peprotech). Cells were harvested for RNA extraction and RT-PCR analysis as described herein below.

### Human BAL samples

BAL fluid was collected from twenty-one mechanically ventilated patients diagnosed with ARDS (Bernard, Artigas et al. 1994) in the intensive care units of the Evangelismos Hospital, Athens (n = 14) and the University Hospital of Alexandroupolis (n = 7). ARDS patients were deeply sedated (Ramsay sedation score = 6) with midazolam and fentanyl; neuromuscular blockade with cisatracurium was used in concordance with recent guidelines. Volume assist-control mechanical ventilation was administered according to the ARDSnet protocol [The Acute Respiratory Distress Syndrome Network. N Engl J Med 342:1301-1308]. BAL fluid was also collected from eleven control patients who underwent diagnostic bronchoscopy for cluonic cough and / or blood-stained sputum; the diagnostic workup of these patients did not ultimately reveal any remarkable respiratory pathology. Approval for the BAL protocol was obtained by the Institutional Review Boards of the aforementioned hospitals. Also, informed, written next-of-kin consent (for ARDS patients) or, informed, written patient consent (from controls) were obtained. BAL was performed as described previously (Weisman, Hirsch-Lerner et al. 2004). Briefly, six aliquots of 20 ml of sterile normal saline at 37° C were infused through the working channel of the bronchoscope. The initial 20 ml portion of the BAL fluid aspirate was used for microbiological cultures and the rest was stored in ice-cold tubes. Subsequently, the BAL was filtered through sterile gauze and centrifuged at 500 ×g for 15 minutes at 4° C. The supernatant was stored at -70° C for later analysis.

Patient characteristics and ventilator settings (volume assist-control mode with square-wave inspiratory flow) are set forth in the table below.

| | ARDS Patients (n = 21) | Controls (n = 11) ^{d} |
|---|---|---|
| Age (years) | 53.2 ± 19.6 | 53.3 ± 11.0 |
| Sex (male / female) | 15/6 | 5 / 6 |
| Body mass index (kg/m²) | 25.8 ± 5.0 | 24.0 ± 2.5 |
| Simplified acute physiology II score a | 36.2 ± 14.2 | ND |
| PaO₂/inspired O₂ fraction (mmHg) ^{b} | 108.2 ± 39.2 | NR |
| Inspired O₂ fraction ^{b, c} | 0.79 ± 0.19 | NR |
| PaCO₂ (mmHg) ^{b} | 47.0 ± 8.5 | NR |
| Positive end-expiratory pressure (cmH₂O) ^{b} | 12.3 ± 3.1 | NA |
| Tidal volume (L) / (mL/kg PBW) ^{b} | 0.46 ± 0.06 / 6.5 ± 0.6 | NA |
| Plateau airway pressure (cmH₂O) ^{b} | 29.8 ± 3.2 | NA |
| Mean airway pressure (cmH₂O) ^{b} | 18.7 ± 3.6 | NA |
| Time from ARDS diagnosis (hours) ^{c} | 16.5 ± 18.9 | NA |
| Pulmonary ARDS - n° / total n°(%) | 12/21 (57.1) | NA |

Values are expressed as mean ± SEM, unless otherwise specified. PBW, predicted body weight; ALI, acute lung injury; ARDS, acute respiratory distress syndrome; ND, not determined; NR, not recorded; NA, not applicable. For males, PBW was calculated as "50 + [Height (cm) - 152.4] • 0.91"; for females, "50" was replaced by "45.5". ^{a}, Determined within 12 hours before the bronchoalvelar lavage (BAL). ^{b}, Recorded / determined within 60 minutes before BAL. ^{c}, Refers to the time interval between the establishment of ARDS diagnosis and BAL. ^{d}, Bronchoscopy and BAL was performed as part of the diagnostic workup for chronic cough (5 patients), hemoptysis (3 patients), suspected pneumonia (2 patients) and suspected tuberculosis (1 patient).

### Statistics

Results are expressed as mean ± SEM. Data were analysed using one-way analysis of variance (ANOVA) with Bonferonni's post-hoc test analysis. Due to minimal variations in the values of the untreated mice in Figures 1B, 4B, 4C, 9B, 10C, 12B & 13A, for analysis, the mean values of each time point were used to create an untreated control "pool" group (n = 7). The Ad-ActA (n = 5 per time point) and Ad-GFP (n = 3 per time point) groups at each time point were compared with the untreated "pool" group using one-way analysis of variance (ANOVA) with Bonferonni's post-hoc test analysis. Statistical significance of one asterisk (*) corresponds to p<0.05, two asterisks (**) to p<0.01 and three asterisks (***) to p<0.001.

### Results

### Example 1: Activin-A over-expression in the airways causes severe and lasting respiratory pathology in a murine model

Applicants expressed activin-A in the airways of C57BL/6 mice using adenovirus-mediated gene transfer and studied the mice for up to four months according to the scheme outlined in Figure 1A. Analysis of BAL fluids demonstrated homeostatic levels of activin-A throughout the experiment in the untreated control and Ad-GFP-treated mice (approximately 150 pg/ml and approximately 300 pg/ml, respectively). In the Ad-ActA group increased expression of activin-A was evident from day 2 (approximately 5 ng/ml), peaked on day 7 (approximately 28 ng/ml) and, despite a gradual decline, it remained high for up to 56 days (Figure 1B). A substantial increase in the mRNA levels of follistatin, which was evident on day 7, reached maximum levels around day 24 and persisted until day 34 (Figure 1B).

The physiological *sequelae* of activin-A over-expression was evaluated by measuring static (Cst) and dynamic (Cdyn) compliance, airway (Rn) and total lung resistance (R). In the Ad-ActA-treated mice, Cst decreased gradually to a value approximately about 40% below the baseline by day 24, remained low until day56 and was partially normalised at four months. Airway resistance increased in the Ad-ActA-treated mice; however, the change was less dramatic than in Cst (Figure 2A). Changes in Cdyn and R followed those of Cst (data not shown).

Histological evaluation of H&E stained lung sections demonstrated sustained alveolar remodelling in the Ad-ActA-treated mice (Figure 2B). From day 2, and reaching maximum levels around day 24, the alveolar septa were gradually thickened by infiltrating inflammatory cells without, however, distorting grossly lung architecture. Between days 24 to 35, however, a heterogeneous histopathology emerged with lung fields exhibiting thickened alveolar walls with honeycombing-type morphology often adjacent to regions of thinning and fibrillation of the alveolar walls with a typical hyper-inflation and emphysematous-type morphology. Disorganisation of elastic fibres and higher incidence of hyaline membrane formation was associated with areas of alveolar edema (Figure 3). From day 35, and onwards, the number of cells infiltrating the alveolar septa declined whereas regions with alveolar wall fibrillation and hyperinflation became the dominant histological feature that persisted up to four months.

### Example 2: Development of hyaline membranes in Ad-ActA treated mice

Hyaline membranes are a key diagnostic correlate of a range of lung pathologies involving microvascular injury, such as neonatal respiratory distress syndrome (RDS) and adult respiratory distress syndrome (ARDS). In these aggressive conditions the hyaline membranes are distributed in a diffuse pattern within the lung parenchyma and are often present as complex structures; involving cytoplasm and nucleoplasm of dead cells, plasma exudates and other debris. In their early form, they often have a uniform appearance with a largely eosinophilic colouration which may vary according to varying predisposing factors. They are localised on the borders of the alveolar arch and can achieve significant thickness, with their overall structure being modified by the biomechanics of the alveolar sac.

By contrast, hyaline membranes in rodent models of lung pathology are much more basic structures often seen as disconnected crescent shapes laying upon the alveolar arc with a range of tinctorial stain reactions that reflects their composition. They are often associated with areas of microvascular injury, loss or microvascular sclerosis and may therefore be considered as primarily a structure correlate of plasma exudation. Although there are clear structural differences between human disease presentation and rodent models, it is probable that both reflect microvascular injury and so, chronologically, the rodent lesion may be correlative with the early stage of the human pathology. In this study applicants describe the presence of these disconnected hyaline membranes in this murine model and the associated presence of pathology to the alveolar capillary bed (Figure 3). Given that the murine model would not show the range of predisposing factors and co-morbidities seen in human hyaline-associated lung disease it is likely that the maturation of an edematous phase to lung injury into a hyaline phase, and thence to a fibroplastic phase, reflect a more direct relationship between injury and outcome in the murine system than man. Furthermore, using the Evan's Blue extravasation assay, applicants demonstrate that activin-A over-expression in the lung leads to substantial micro-vascular injury as assessed by the retention of Evan's Blue dye in the lung parenchyma (Figure 3B & C).

In applicants view, the presence of hyaline membranes and the substantial extravasation of Evan's Blue dye substantiate beyond any doubt that this model system is reflective of key components of aberrant HDR activation, and ARDS lung pathology in particular.

### Example 3: Activin-A induces a transient fibrotic response that precedes the development of emphysematous lesions in a murine model

The dramatic remodelling of the lung parenchyma, the development of "honey-combing" like lesions detected 24 to 34 days post-viral-instillation in the Ad-ActA treated group and primarily the development of persistent reduction of lung compliance prompted applicants to further investigate the contribution of fibrosis in the activin-A-induced process.

Staining of tissue sections for collagen demonstrated development of a transient fibrotic response in the parenchyma of the Ad-ActA treated mice that preceded the development of emphysematous lesions (Figure 4A). Morphometric analysis of aSMA immuno-staining, collagen deposition and airspace enlargement in the alveolar compartment illustrated the sequential development of these parameters with aSMA expression reaching maximum around day 24, collagen deposition around day 34 and emphysema gradually reaching maximal levels at 4 months (Figure 4B).

Accumulation of collagen was associated with an increase in the levels of collagens I and III, TIMP1 and CTGF (Figure 4C). Interestingly, the transient fibrotic response induced by activin-A over-expression was not associated with any increase in the TGFβ mRNA level (Figure 4C) or with the total or active TGFβ protein level in total lung protein extracts (Figure 5). Contrary, significantly reduced level of active TGFβ was detected in the Ad-ActA-treated groups. Very low and similar levels of total TGFβ were also detected in BAL fluids from untreated (31.8 ± 12.25 pg/ml BAL), Ad-GFP-treated (31.38 ± 11.79 pg/ml BAL) or Ad-ActA-treated mice (24.45 ± 13.45 pg/ml BAL). Thus, it is unlikely that TGFβ is involved in the Ad-ActA-induced phenotype, although a minor contribution cannot be definitively excluded at the moment.

The resolution of collagen deposition and the development of emphysematous lesions correlated with a decline of TIMP1 back to the control levels and a concomitant increase in the mRNA levels for MMP2 and MMP9. No statistically significant alteration in any of the analysed parameters was observed in the Ad-GFP-treated and untreated controls (Figure 4C).

Fibrosis and emphysema are clinically distinct pathologies; however, several studies have found that emphysema can be accompanied by fibrotic changes in the lungs of both humans and animal models (Hale, Ewing et al. 1984; Lundblad, Thompson-Figueroa et al. 2005) and, indeed, emphysema and fibrotic lesions commonly co-exist in the lungs of COPD patients. The consecutive appearance of fibrotic and emphysematous lesions in the lungs of the Ad-ActA-treated mice shows that emphysema can develop as a result of collateral damage inflicted during an attempt to clear collagen deposits from the alveolar area. These findings link dys-regulated activin-A expression to the development of emphysema and, consequently, it is contemplated that activin-A neutralisation will benefit patients with COPD.

In the control Ad-GFP groups, there were only minor inflammatory changes consisting essentially of inflammatory cell occupation of the adventitia of large pulmonary arteries and arterioles (Figure 6). These changes did not extend into the alveolar spaces or correlate with the spatial distribution of virus infected cells. Although they persisted in some mice up to day 56, they were not associated with any functional or structural alterations in the adjacent parenchyma even in mice that received five-fold higher Ad-GFP viral-loads (Figure 6).

### Example 4: Activin-A-induced lung injury is associated with acute alveolar cell death, sustained up-regulation of HMGB1 and inflammation in a murine model

To delineate if the mechanism responsible for alveolar wall damage involved apoptotic cell-death, tissue sections were analysed by TUNEL staining. A dramatic wave of TUNEL⁺ signal that peaked 48 hours after Ad-ActA-instillation was observed, while the level of activin-A in the BAL fluids was approximately 5 ng/ml (Figure 7). Thereafter, despite further increase of activin-A to approximately 28 ng/ml, the signal rapidly declined and TUNEL⁺ material decreased to very low levels (Figure 7C). Notably, a transient apoptotic response has been observed after over-expression of TGFβ in the mouse lung as well (Lee, Cho et al. 2004). Interestingly, few TUNEL⁺ cells exhibited typical apoptotic cell morphology. The majority of the signal involved apparently fragmented cell-debris that was either attached to the basement membrane of alveolar-septa or was present in clumps in the airspaces (Figure 7B). This staining pattern could stem from a rapid transition of cells undergoing apoptosis into secondary necrosis and cellular rupture.

Co-staining for CD45, T1α, SpC, and TUNEL demonstrated that TUNEL⁺ material in the airspaces was admixed mainly with T1α positive fragments (Figure 8) suggesting that it was derived from Type-I pneumonocyte wall fragmentation. Occasionally, some TUNEL⁺ Type-II pneumonocytes were also detected in the affected areas. No TUNEL⁺ material was ever detected at any time point in the lungs of untreated or Ad-GFP-treated mice (Figure 7C). Rupture of damaged cells in the lung tissue could lead to release of damage-associated molecular patterns (DAMPs) which are well recognised in experimental animal models and human diseases as key pro-inflammatory "drivers" (Bianchi 2007). Therefore, applicants investigated if over-expression of activin-A in the airways was associated with alterations in the level or pattern of expression of HMGB1, the prototypical DAMP molecule. Indeed, Ad-ActA treated mice demonstrated substantial increase of HMGB1 immuno-staining (Figure 7D) that was apparent already on day 2, reached maximum levels at approximately day 15 and persisted until approximately day 56.

### Example 5: Activin-A over-expression leads to the development of a dynamically evolving inflammatory response in a mammal (murine) model

Activin-A over-expression triggered a dynamically evolving inflammatory response (Figure 9) that involved neutrophils and macrophages around days 0 to 4, macrophages, neutrophils and lymphocytes around days 5 to 15 and macrophages and lymphocytes around days 15 to 56. In the interstitium, macrophages (CD68⁺ cells) constituted approximately 30% of the CD45⁺ cells on day 7; however, by day 24 they constituted the vast majority of the CD45⁺ infiltrates. Quantitation of the mRNA levels for a series of cytokines and chemokines demonstrated that activin-A over-expression produced a "cytokine storm"-like response in the lung. A substantial increase in the levels of MCP1/CCL2 and IL6 mRNA (maximum levels already on day 2) were followed by a cocktail of TH1, TH2 and TH17 cytokines that included IL-1β, IL-4, KC/IL-8, IFNγ, TNFα and IL-17 reaching maximal levels around day 7, and IL-5 and IL-10 reaching maximal levels around day 15. The third inflammatory wave (days 15 to 56) was characterised by rapid decline of cytokine/chemokine mediators with the exception of a second wave of KC/IL-8 expression and a further increase in the levels of IL-17α. The substantial increase in the expression of Arg-1 around day 15 is consistent with a shift of the macrophage population towards the "alternatively activated" phenotype. The expression of selected cytokines, namely IL-6, MCP-1/CCL2, TNFα and IL-5, was also analysed in BAL samples at the protein level confirming their up-regulation in Ad-ActA treated mice with kinetics similar to those at the mRNA levels (Figure 10). Only minor inflammatory changes consisting essentially of inflammatory cell occupation of the adventitia of pulmonary arteries and arterioles were evident in the Ad-GFP-treated mice (Figure 6). These changes did not extend into the alveolar spaces or correlate with the spatial distribution of virus-infected cells. Although they persisted in some mice up to day 56, they were not associated with any functional or structural alterations in adjacent parenchyma even in mice that received five-fold higher Ad-GFP viral loads (Figure 6B). Furthermore, intra-tracheal instillation of Ad-GFP control-viruses did not lead to any significant changes in cytokine mRNA (Figure 9B) or protein level (Figure 10).

### Example 6: Activin-A alters the composition and function of lung epithelium in a murine model

Although Clara cells dominated the distil airways in the untreated and Ad-GFP-treated mice, the majority of airway epithelial cells in the Ad-ActA-treated mice between days 15 to 35 were ciliated. Immuno-staining with cell specific markers confirmed a dramatic reduction of CC10 immuno-staining and a shift in favour of the ciliated phenotype (Figure 11A). In the alveolar compartment, numbers of Type-I pneumonocytes gradually declined (Figure 11B) and clusters of detached T1α⁺ pneumonocytes were found in the airspaces. These detached T1α⁺ cells were TUNEL⁻ and, thus, apoptosis as a mechanism of Type-I pneumonocyte reduction in the chronic phase of the activin-A-induced response was deemed unlikely.

Type-II pneumonocytes were also drastically affected by activin-A. As shown in Figure 12, SpC immuno-staining declined rapidly, reached the lowest levels already on day 7 and remained very low until day 15 (Figure 11A). Thereafter, SpC⁺ cells re-appeared in clusters and usually in regions neighbouring those severely affected. Even on day 56, SpC expression was markedly heterogeneous with certain areas of the lung containing high numbers of SpC⁺ cells and others being completely devoid (Figure 11A).

Surfactant-protein mRNA levels of SpC, SpB and SpA were severely down-regulated in Ad-ActA-treated mice (Figure 11B). They reached the lowest around days 7 to 15, being reduced by approximately 70 to 80%, compared with Ad-GFP-treated and untreated controls. The inhibitory effect of activin-A was selective as SpD levels were not reduced at any time point but rather demonstrated statistically significant increase between approximately days 24 to 34.

The down-regulation of SpC and SpB mRNA levels stems from direct action of activin-A on the alveolar epithelial cells as it could be recapitulated *in vitro* by treating the epithelial cells lines MLE12 (Figure 11C) and MLE15 (data not shown) with 10 ng/ml recombinant activin-A.

Given the fact that similar alterations in lung airway epithelial composition has been observed in severe asthma (e.g. presence of ciliated epithelial cells ascending to the terminal bronchiole level) it is contemplated that activin neutralisers, as described herein, will be useful therapeutic agents for the prophylaxis and / or therapy of said disease.

### Example 7: Activin-A over-expression leads to a systemic pro-coagulant state in a murine model

Local inflammatory processes in the lung can promote a pro-coagulant state (Yoo and Juliano 2000). Therefore, applicants investigated the effect of activin-A over-expression on the haemostatic balance using the "tail bleeding time" assay (Figure 13A). Bleeding time in the Ad-ActA-treated mice, 7 days post-viral-instillation, was reduced almost 75% compared with the control groups. Bleeding time gradually improved, reaching normal levels at 4 months. The kinetics of tail bleeding time reduction correlated with an increase in mRNA levels for tissue factor (TF), a key activator of the extrinsic coagulation pathway (Bastarache, Wang et al. 2007), suggesting that up-regulation of TF could be one of the mechanisms by which activin-A interferes with the coagulation cascade. Up-regulation of TF mRNA levels stems from_a direct action of activin-A on alveolar epithelial cells since it could be recapitulated *in vitro* by treating the epithelial cell lines MLE12 (Figure 13B) and MLE15 (data not shown) with 10 ng/ml recombinant activin-A (Figure 13B).

Given that: (a) establishment of a pro-coagulant state characterises many pathologies such as autoimmunity (including, but not limited to: rheumatoid arthritis, systemic lupus erythematosous, multiple sclerosis, inflammatory bowel disease, vasculitis and anti-phospholipis syndrome), acute responses against infectious agents such as highly pathogenic viruses (e.g. influenza A viruses H5N1 and H1N1, coronaviruses SARS and human rhinoviruses C and D) and acute injury of tissues inflicted by infection, toxic substances, trauma, heart infarction or surgery; and (b) the pro-coagulant state associated with the above mentioned pathologies contributes to their pathophysiology, applicants contemplate that activin neutralisers, as described herein, will be useful therapeutic agents for the prophylaxis and / or therapy of said diseases.

### Example 8: Increased levels of activin-A are detected in BAL fluid of human ARDS patients; correlation between survival and activin-A-expression pattern

Although, in isolation, the structural and functional changes, the cytokine profile and kinetics observed in the Ad-ActA-treated mice are seen in different human respiratory diseases, taken together, they resembled in many aspects those described in ARDS (Tsushima, King et al. 2009). Therefore, applicants analysed activin-A and follistatin protein levels in BAL fluids obtained from ARDS patients. Levels of activin-A in the ARDS patient group were significantly higher than the control group (Figure 14A). Considering the significant dilution of BAL fluids during collection (approximately twenty-five to one hundred-fold, estimated from protein concentrations in BAL and serum), BAL activin-A levels of 2 to 3 ng/ml suggests that local concentration of this molecule in affected areas could reach, and even exceed, the levels of ectopically expressed activin-A in applicants experimental mice, i.e. 5 to 28 ng/ml.

Follistatin was also elevated in the ARDS group although, pair-wise comparison of activin-A and follistatin levels in the same patient revealed that in all but one patient, the concentration of activin-A exceeded that of follistatin (Figure 14B). For some of the ARDS patients BAL cells were also available as frozen samples and mRNA levels of activin-A, activin-B and TGFβ1 were analysed (Figure 14C). In comparison with PBMCs from healthy controls, BAL cells from ARDS patients contained approximately 40 times more activin-A mRNA. This increase appears to be selective since the same samples contained marginally elevated activin-B and only five to six-fold higher TGFβ1 mRNA.

In a small group of patients BAL samples were collected twice; the first, immediately upon admission to the intensive care unit and, the second, four days later. Measurement of activin-A levels in the BAL samples demonstrated that patients that had reduced levels of activin-A in their second BAL sample (as compared with the first BAL sample) had much higher chances of survival. In contrast, patients that had either similar or increased levels of activin-A in their second BAL sample (as compared with the first BAL sample) had much higher chances to succumb to ARDS.

These findings, in human subjects, demonstrate the involvement of activin-A in ARDS pathophysiology and leads to the conclusion that activin neutralisers, as described herein, will be useful therapeutic agents for the prophylaxis and / or therapy of said disease.

### Example 9: Therapeutic neutralisation of activin-A protects from activin-A-induced pathology in a murine model

To validate activin-A as a therapeutic target, applicants used a fusion protein composed of the extracellular portion of activin-A's type-II receptor (ActRIIB) fused to the Fc-portion of the human IgG1. Although all Ad-ActA-treated groups contained similar levels of activin-A mRNA in their lungs (Figure 15C) those treated either prophylactically or therapeutically exhibited dramatic normalisation of lung-compliance, tail bleeding time (Figure 15A), histology and mRNA levels of SpC, IL-6, Collagen I &III, TF and Arg-1 (Figure 15B & C). The "therapeutic" reversal of activin-A-induced physiological and histochemical changes indicates that, even after infliction of tissue damage and development of inflammation, the induced processes are highly dependent on the continuous presence of activin-A and thus pharmacological normalisation of activin-A levels was beneficial.

These findings demonstrate the therapeutic value of employing an activin neutraliser *in* vivo in a mammal (murine) activin-A induced disease model.

### Example 10: Neutralisation of endogenous activin-A attenuates LPS-induced Acute-Lung-Injury in a murine model

To further validate the role of activin-A in ALI/ARDS pathophysiology, applicants utilised an established murine model in which only endogenous activin-A production is involved, namely, the model of intra-tracheal instillation of Lipopolysacharide (LPS). Mice were treated with 2.5 mg/kg of LPS intra-trachealy and analysed 4 days later. LPS-treated mice had increased levels of activin-A in the BAL (approximately 3 to 4 ng/ml BAL) and exhibited a dramatic, predominantly neutrophil inflammatory response, substantial reduction in lung-compliance, reduction in SpC immuno-staining, systemic pro-coagulant state and severe tissue injury as illustrated by the abundance of TUNEL immuno-staining. The TUNEL staining was phenotypically similar to the one observed in Ad-ActA-treated mice. Furthermore, the involvement of the endothelium was much more prominent because many of the medium size blood vessels in the affected areas were lined with TUNEL⁺ signal.

Prophylactic and therapeutic neutralisation of the endogenous activin-A with ActRIIB-Fc protein similarly improved lung-compliance and tail bleeding time, reduced dramatically TUNEL immuno-staining and up-regulated SpC immuno-staining. The beneficial effect of activin-neutralisation in a model that depends on endogenous activin-A activity supports the conclusion that this cytokine plays a key role in the pathophysiology of ALI/ARDS.

### Example 11: Effect of ActRIIB-Fc treatment on the inflammatory response induced by intra-tracheal instillation of LPS in a murine model

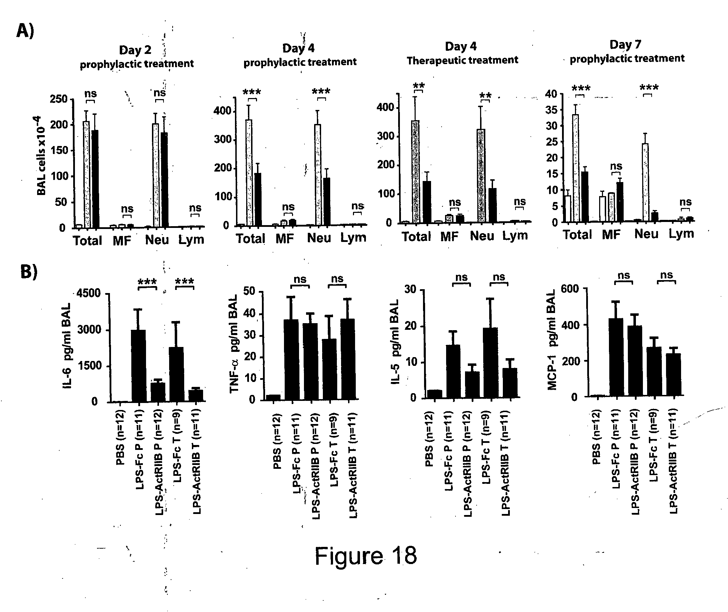

The effect of activin-A neutralisation on the LPS-induced inflammatory response was analysed in more detail. After intra-tracheal instillation of LPS, mice were treated prophylactically with ActRIIB-Fc and analysed 2, 4 or 7 days later. An additional group of mice were treated therapeutically with ActRIIB-Fc and analysed 4 days after LPS-instillation. Analysis of BAL samples demonstrated that LPS-instillation induced a strong, primarily neutrophilic, inflammatory response that reached maximum on day 4 and declined by day 7. Prophylactic and therapeutic delivery of ActRIIB-Fc modified, significantly, the inflammatory response of the LPS-treated mice. Although two days after LPS-instillation the ActRIIB-Fc-treated mice possessed similar levels of inflammatory cells with (as) the LPS-treated control group; the inflammatory response of the ActRIIB-Fc-treated groups resolved faster. Thus, four days after LPS-instillation the ActRIIB-Fc-treated mice possessed significantly reduced numbers of neutrophils and IL-6 protein levels in their BAL (Figure 18A & B) and seven days after LPS-instillation, while significant numbers of neutrophils were still present in the BAL samples of the LPS control group, the neutrophil levels in ActRIIB-Fc-treated mice were reduced to baseline (Figure 18B).

Interestingly, whereas IL-6 protein levels were significantly affected by the ActRIIB-Fc treatment four days after LPS-instillation, the levels of TNF-α, IL-5 and MCP-1 were not affected significantly (Figure 18B).

These discoveries further strengthen the conclusions derived from Example 9 and 10 by demonstrating the therapeutic value of activin-A neutralisers in a mammal (murine) disease model driven by endogenous activin-A production. Furthermore, they demonstrate that the key biological response for the beneficial therapeutic effect of the activin neutraliser is not its anti-inflammatory effects (as demonstrated in the Figure 18 an activin neutraliser does not affect the inflammatory response during the acute early phase of the response and the partial attenuation of inflammatory components is evident only at later stages, as a faster resolution of inflammation). It is evident that the beneficial effects of activin neutralisers derive from their capacity to normalise the coagulation system, to normalise surfactant expression and to prevent tissue injury (e.g. cell death). Therefore, activin neutralisers are not the appropriate anti-inflammatory therapeutic agents but, rather, therapeutic agents for normalisation of pathogenic activation of the coagulation system, for normalising suppression of surfactant expression and preventing acute cell death in stressed tissues.

### Pharmaceutical compositions and methods of administration

In accordance with the description of the invention provided above, specific preferred pharmaceutical compositions of the present invention may be prepared and examples of which are provided below. The following specific compositions are to be construed as merely illustrative examples of compositions and not as a limitation of the scope of the present invention in any way.

### Example 12: Composition for inhalation administration

For an aerosol container with a capacity of 20 to 30 ml; a mixture of 10 to 50 mg of ActRIIB-Fc with 0.5 to 1.0% by weight of a lubricating agent, such as polysorbate 85 or oleic acid, was dispersed in a suitable propellant, such as Freon, and placed into an appropriate aerosol container for either intranasal or oral inhalation administration.

### Example 13: Composition for parenteral administration

A pharmaceutical composition of the present invention for intramuscular injection could be prepared to contain 1 ml sterile isotonic saline and 0.5 to 2.0 mg ActRIIB-Fc. Similarly, a Pharmaceutical composition for intravenous infusion may comprise 250 ml of sterile Ringer's solution and 1 to 5 mg of ActRIIB-Fc.

### Example 14: Capsule composition

A pharmaceutical composition of the present invention in the form of a capsule may be prepared by filling a standard two-piece hard gelatin capsule with 0.5 to 5.0 mg of SB-505124 hydrochloride hydrate (Sigma-Aldrich, Product No S4696) in powdered form, 180 mg of lactose, 65 mg of talc and 12 mg of magnesium stearate.

### Example 15: Injectable parenteral composition

A pharmaceutical composition of the present invention in a form suitable for administration by injection may be prepared by mixing 0.1% by weight of ActRIIB-Fc in 12% by volume propylene glycol and isotonic saline. The solution is sterilised by filtration.

While specific embodiments of the subject matter have been discussed, the above specification is illustrative and not restrictive. Many variations will become apparent to those skilled in the art upon review of this specification and the claims below.

### BIBLIOGRAPHY

Abraham, E. (2000). "Coagulation abnormalities in acute lung injury and sepsis." Am J Respir Cell Mol Biol 22(4): 401-404.
Agresti, A. and M. E. Bianchi (2003). "HMGB proteins and gene expression." Curr Opin Genet Dev 13(2): 170-178.
Andreakos, E., U. Rauchhaus, et al. (2009). "Amphoteric liposomes enable systemic antigen-presenting cell-directed delivery of CD40 antisense and are therapeutically effective in experimental arthritis." Arthritis Rheum 60(4): 994-1005.
Antoine, D. J., D. P. Williams, et al. (2010). "Diet restriction inhibits apoptosis and HMGB1 oxidation and promotes inflammatory cell recruitment during acetaminophen hepatotoxicity." Mol Med 16(11-12): 479-490.
Anzueto, A., R. P. Baughman, et al. (1996). "Aerosolized surfactant in adults with sepsis-induced acute respiratory distress syndrome. Exosurf Acute Respiratory Distress Syndrome Sepsis Study Group." N Engl J Med 334(22): 1417-1421.
Aoki, F., M. Kurabayashi, et al. (2005). "Attenuation of bleomycin-induced pulmonary fibrosis by follistatin." Am J Respir Crit Care Med 172(6): 713-720.
Ashitani, J., H. Mukae, et al. (2002). "Elevated plasma procoagulant and fibrinolytic markers in patients with chronic obstructive pulmonary disease." Intern Med 41(3): 181-185.
Bader, A. G., D. Brown, et al. (2010). "The promise of microRNA replacement therapy." Cancer Res 70(18): 7027-7030.
Barany et al., Int. J. Pep. ProteinRes., 30:705-739 (1987)
Barnes, P. J. (2008). "Immunology of asthma and chronic obstructive pulmonary disease." Nat Rev Immunol 8(3): 183-192.
Barnes, P. J. (2011). "Glucocorticosteroids: current and future directions." Br J Pharmacol 163(1): 29-43.
Bartel, D. P. (2004). "MicroRNAs: genomics, biogenesis, mechanism, and function." Cell 116(2): 281-297.
Bastarache, J. A., L. Wang, et al. (2007). "The alveolar epithelium can initiate the extrinsic coagulation cascade through expression of tissue factor." Thorax 62(7): 608-616.
Bates, J. H., T. F. Schuessler, et al. (1997). "Temporal dynamics of acute isovolume bronchoconstriction in the rat." J Appl Physio 82(1): 55-62.
Bernard, D. J., K. B. Lee, et al. (2006). "Activin B can signal through both ALK4 and ALK7 in gonadotrope cells." Reprod Biol Endocrinol 4: 52.
Bernard, G. (2002). "The International Sepsis Forum's controversies in sepsis: corticosteroids should not be routinely used to treat septic shock." Crit Care 6(5): 384-386.
Bernard, G. R., A. Artigas, et al. (1994). "The American-European Consensus Conference on ARDS. Definitions, mechanisms, relevant outcomes, and clinical trial coordination." Am J Respir Crit Care Med 149(3 Pt 1): 818-824.
Bernard, G. R., J. L. Vincent, et al. (2001). "Efficacy and safety of recombinant human activated protein C for severe sepsis." N Engl J Med 344(10): 699-709.
Bertolino, P., R. Holmberg, et al. (2008). "Activin B receptor ALK7 is a negative regulator of pancreatic beta-cell function." Proc Natl Acad Sci U S A 105(20): 7246-7251.
Beutler, B. (2007). "Neo-ligands for innate immune receptors and the etiology of sterile inflammatory disease." Immunol Rev 220: 113-128.
Bevilacqua, M. P., J. S. Pober, et al. (1986). "Recombinant tumor necrosis factor induces procoagulant activity in cultured human vascular endothelium: characterization and comparison with the actions of interleukin 1." Proc Natl Acad Sci U S A 83(12): 4533-4537.
Bianchi, M. E. (2007). "DAMPs, PAMPs and alarming: all we need to know about danger." J Leukoc Biol 81(1): 1-5.
Bianchi, M. E. (2009). "HMGB1 loves company." J Leukoc Biol 86(3): 573-576.
Bianchi, M. E. and A. A. Manfredi (2007). "High-mobility group box 1 (HMGB1) protein at the crossroads between innate and adaptive immunity." Immunol Rev 220: 35-46.
Bijsterbosch, M. K., M. Manoharan, et al. (2001). "Delivery of cholesteryl-conjugated phosphorothioate oligodeoxynucleotides to Kupffer cells by lactosylated low-density lipoprotein." Biochem Pharmacol 62(5): 627-633.
Bilezikjian, L. M., A. L. Blount, et al. (2001). "Actions of activins, inhibins and follistatins: implications in anterior pituitary function." Clin Exp Pharmacol Physiol 28(3): 244-248.
Bonaldi, T., F. Talamo, et al. (2003). "Monocytic cells hyperacetylate chromatin protein HMGB1 to redirect it towards secretion." EMBO J 22(20): 5551-5560.
Bone, R. C. (1996). "Immunologic dissonance: a continuing evolution in our understanding of the systemic inflammatory response syndrome (SIRS) and the multiple organ dysfunction syndrome (MODS)." Ann Intern Med 125(8): 680-687.
Bone, R. C. (1996). "Sir Isaac Newton, sepsis, SIRS, and CARS." Crit Care Med 24(7): 1125-1128.
Bone, R. C., C. J. Fisher, Jr., et al. (1987). "A controlled clinical trial of high-dose methylprednisolone in the treatment of severe sepsis and septic shock." N Engl J Med 317(11): 653-658.
Bonniaud, P., G. Martin, et al. (2004). "Connective tissue growth factor is crucial to inducing a profibrotic environment in "fibrosis-resistant" BALB/c mouse lungs." Am J Respir Cell Mol Biol 31(5): 510-516.
Borensztajn, K. S., J. H. von der Thusen, et al. (2011). "The role of coagulation in chronic inflammatory disorders: a jack of all trades." Curr Pharm Des 17(1): 9-16.
Boulikas, T. (1996). "Liposome DNA delivery and uptake by cells (review)." Oncol Rep 3(6): 989-995.
Bours, M. J., E. L. Swennen, et al. (2006). "Adenosine 5'-triphosphate and adenosine as endogenous signaling molecules in immunity and inflammation." Pharmacol Ther 112(2): 358-404.
Brosh, N., D. Sternberg, et al. (1995). "The plasmacytoma growth inhibitor restrictin-P is an antagonist of interleukin 6 and interleukin 11. Identification as a stroma-derived activin A." J Biol Chem 270(49): 29594-29600.
Broze, G. J., Jr., Z. F. Yin, et al. (2001). "A tail vein bleeding time model and delayed bleeding in hemophiliac mice." Thromb Haemost 85(4): 747-748.
Byfield et al., (SB-505124 Is a selective inhibitor of Transforming Growth Factor-β type I receptors ALK4, ALK5 and ALK7. Mol. Pharmacol., 65(3):744-752 (2004))
Callahan, J. F., J. L. Burgess, et al. (2002). "Identification of novel inhibitors of the transforming growth factor betal (TGF-beta1) type 1 receptor (ALK5)." J Med Chem 45(5): 999-1001.
Caniggia, I., S. J. Lye, et al. (1997). "Activin is a local regulator of human cytotrophoblast cell differentiation." Endocrinology 138(9): 3976-3986.
Casey, B. P. and P. M. Glazer (2001). "Gene targeting via triple-helix formation." Prog Nucleic Acid Res Mol Biol 67: 163-192.
Cepkova, M. and M. A. Matthay (2006). "Pharmacotherapy of acute lung injury and the acute respiratory distress syndrome." J Intensive Care Med 21(3): 119-143.
Chen, G. Y. and G. Nunez (2010). "Sterile inflammation: sensing and reacting to damage." Nat Rev Immunol 10(12): 826-837.
Cheng, T., D. Liu, et al. (2003). "Activated protein C blocks p53-mediated apoptosis in ischemic human brain endothelium and is neuroprotective." Nat Med 9(3): 338-342.
Cho, S. H., Z. Yao, et al. (2003). "Regulation of activin A expression in mast cells and asthma: its effect on the proliferation of human airway smooth muscle cells." J Immunol 170(8): 4045-4052.
Collins, R. A., R. C. Gualano, et al. (2005). "Hyperresponsiveness to inhaled but not intravenous methacholine during acute respiratory syncytial virus infection in mice." Respir Res 6: 142.
Costa, E. L., 1. A. Schettino, et al. (2006). "The lung in sepsis: guilty or innocent?" Endocr Metab Immune Disord Drug Targets 6(2): 213-216.
Deans, K. J., M. Haley, et al. (2005). "Novel therapies for sepsis: a review." J Trauma 58(4): 867-874.
de Gouville AC, Boullay V, Krysa G, Pilot J, Brusq JM, Loriolle F, Gauthier JM, Papworth SA, Laroze A, Gellibert F, Huet S.(2005). Inhibition of TGF-beta signaling by an ALK5 inhibitor protects rats from dimethylnitrosamine-induced liver fibrosis. Br J Pharmacol. 145:166-77.
Dohi, T., C. Ejima, et al. (2005). "Therapeutic potential of follistatin for colonic inflammation in mice." Gastroenterology 128(2): 411-423.
Enhorning, G., L. C. Duffy, et al. (1995). "Pulmonary surfactant maintains patency of conducting airways in the rat." Am J Respir Crit Care Med 151(2 Pt 1): 554-556.
Eppstein, D. A., Y. V. Marsh, et al. (1985). "Biological activity of liposome-encapsulated murine interferon gamma is mediated by a cell membrane receptor." Proc Natl Acad Sci U S A 82(11): 3688-3692.
Esmon, C. T. (2004). "The impact of the inflammatory response on coagulation." Thromb Res 114(5-6): 321-327.
Ferhani, N., S. Letuve, et al. (2010). "Expression of high-mobility group box 1 and of receptor for advanced glycation end products in chronic obstructive pulmonary disease." Am J Respir Crit Care Med 181(9): 917-927.
Fisher, C. J., Jr., J. M. Agosti, et al. (1996). "Treatment of septic shock with the tumor necrosis factor receptor:Fc fusion protein. The Soluble TNF Receptor Sepsis Study Group." N Engl J Med 334(26): 1697-1702.
Fujita, M., J. M. Shannon, et al. (2003). "Overexpression of tumor necrosis factor-alpha diminishes pulmonary fibrosis induced by bleomycin or transforming growth factor-beta." Am J Respir Cell Mol Biol 29(6): 669-676.
Gardella, S., C. Andrei, et al. (2002). "The nuclear protein HMGB1 is secreted by monocytes via a non-classical, vesicle-mediated secretory pathway." EMBO Rep 3(10): 995-1001.
Gerard, C. (2003). "Complement C5a in the sepsis syndrome--too much of a good thing?" N Engl_ J Med 348(2): 167-169.
Glasser, S. W., E. A. Detmer, et al. (2003). "Pneumonitis and emphysema in sp-C gene targeted mice." J Biol Chem 278(16): 14291-14298.
Gordon, S. (2002). "Pattern recognition receptors: doubling up for the innate immune response." Cell 111(7): 927-930.
Greene, K. E., J. R. Wright, et al. (1999). "Serial changes in surfactant-associated proteins in lung and serum before and after onset of ARDS." Am J Respir Crit Care Med 160(6): 1843-1850.
Gregory, T. J., K. P. Steinberg, et al. (1997). "Bovine surfactant therapy for patients with acute respiratory distress syndrome." Am J Respir Crit Care Med 155(4): 1309-1315.
Gribi, R., T. Tanaka, et al. (2001). "Expression of activin A in inflammatory arthropathies." Mol Cell Endocrinol 180(1-2): 163-167.
Grygielko, E. T., W. M. Martin, et al. (2005). "Inhibition of gene markers of fibrosis with a novel inhibitor of transforming growth factor-beta type I receptor kinase in puromycin-induced nephritis." J Pharmacol Exp Ther 313(3): 943-951.
Gunther, A., P. Mosavi, et al. (2000). "Alveolar fibrin formation caused by enhanced procoagulant and depressed fibrinolytic capacities in severe pneumonia. Comparison with the acute respiratory distress syndrome." Am J Respir Crit Care Med 161(2 Pt 1): 454-462.
Hale, K. A., S. L. Ewing, et al. (1984). "Lung disease in long-term cigarette smokers with and without chronic air-flow obstruction." Am Rev Respir Dis 130(5): 716-721.
Halpin, D. M. and M. Miravitlles (2006). "Chronic obstructive pulmonary disease: the disease and its burden to society." Proc Am Thorac Soc 3(7): 619-623.
Han, M. K. (2011). "Update in chronic obstructive pulmonary disease in 2010." Am J Respir Crit Care Med 183(10): 1311-1315.
Hardy, C. L., A. E. O'Connor, et al. (2006). "Follistatin is a candidate endogenous negative regulator of activin A in experimental allergic asthma." Clin Exp Allergy 36(7): 941-950.
Harrison, C. A., P. C. Gray, et al. (2005). "Antagonists of activin signaling: mechanisms and potential biological applications." Trends Endocrinol Metab 16(2): 73-78.
Hasselhoff (1988) Nature 334:585.
Hawlisch, H., Y. Belkaid, et al. (2005). "C5a negatively regulates toll-like receptor 4-induced immune responses." Immunity 22(4): 415-426.
Hedger, M. P., A. E. Drummond, et al. (1989). "Inhibin and activin regulate [3H]thymidine uptake by rat thymocytes and 3T3 cells in vitro." Mol Cell Endocrinol 61(1): 133-138.
Helene, C. (1991). "The anti-gene strategy: control of gene expression by triplex-forming-oligonucleotides." Anticancer Drug Des 6(6): 569-584.
Hemmi, H., O. Takeuchi, et al. (2000). "A Toll-like receptor recognizes bacterial DNA." Nature 408(6813): 740-745.
Herridge, M. S. and D. C. Angus (2005). "Acute lung injury--affecting many lives." N Engl J Med 353(16): 1736-1738.
Hobbs, S., S. Jitrapakdee, et al. (1998). "Development of a bicistronic vector driven by the human polypeptide chain elongation factor 1alpha promoter for creation of stable mammalian cell lines that express very high levels of recombinant proteins." Biochem Biophys Res Commun 252(2): 368-372.
Huber, S., F. R. Stahl, et al. (2009). "Activin a promotes the TGF-beta-induced conversion of CD4+CD25- T cells into Foxp3+ induced regulatory T cells." J Immunol 182(8): 4633-4640.
Hubner, G., C. Alzheimer, et al. (1999). "Activin: a novel player in tissue repair processes." Histol Histopathol 14(1): 295-304.
Inman, G. J., F. J. Nicolas, et al. (2002). "SB-431542 is a potent and specific inhibitor of transforming growth factor-beta superfamily type I activin receptor-like kinase (ALK) receptors ALK4, ALK5, and ALK7." Mol Pharmacol 62(1): 65-74.
Inouye, S., Y. Guo, et al. (1991). "Recombinant expression of human follistatin with 315 and 288 amino acids: chemical and biological comparison with native porcine follistatin." Endocrinology 129(2): 815-822.
Inouye, S., N. Ling, et al. (1992). "Localization of the heparin binding site of follistatin." Mol Cell Endocrinol 90(1): 1-6.
Iwasaki, A. and R. Medzhitov (2010). "Regulation of adaptive immunity by the innate immune system." Science 327(5963): 291-295.
Jobe, A. H. (1993). "Pulmonary surfactant therapy." N Engl J Med 328(12): 861-868.
Jones, K. L., A. Mansell, et al. (2007). "Activin A is a critical component of the inflammatory response, and its binding protein, follistatin, reduces mortality in endotoxemia." Proc Natl Acad Sci U S A 104(41): 16239-16244.
Kalai, M., G. Van Loo, et al. (2002). "Tipping the balance between necrosis and apoptosis in human and murine cells treated with interferon and dsRNA." Cell Death Differ 9(9): 981-994.
Kambas, K., M. M. Markiewski, et al. (2008). "C5a and TNF-alpha up-regulate the expression of tissue factor in intra-alveolar neutrophils of patients with the acute respiratory distress syndrome." J Immunol 180(11): 7368-7375.
Kanazawa, H., Y. Tochino, et al. (2011). "Validity of HMGB1 measurement in epithelial lining fluid in patients with COPD." Eur J Clin Invest.
Karagiannidis, C., G. Hense, et al. (2006). "Activin A is an acute allergen-responsive cytokine and provides a link to TGF-beta-mediated airway remodeling in asthma." J Allergy Clin Immunol 117(1): 111-118.
Keutmann, H. T., A. L. Schneyer, et al. (2004). "The role of follistatin domains in follistatin biological action." Mol Endocrinol 18(1): 228-240.
Khadaroo, R. G. and J. C. Marshall (2002). "ARDS and the multiple organ dysfunction syndrome. Common mechanisms of a common systemic process." Crit Care Clin 18(1): 127-141.
Kildsgaard, J., T. J. Hollmann, et al. (2000). "Cutting edge: targeted disruption of the C3a receptor gene demonstrates a novel protective anti-inflammatory role for C3a in endotoxin-shock." J Immunol 165(10): 5406-5409.
Kojima, I., H. Mogami, et al. (1993). "Modulation of growth of vascular smooth muscle cells by activin A." Exp Cell Res 206(1): 152-156.
Kokkola, R., A. Andersson, et al. (2005). "RAGE is the major receptor for the proinflammatory activity of HMGB1 in rodent macrophages." Scand J Immunol 61(1): 1-9.
Kokkola, R., J. Li, et al. (2003). "Successful treatment of collagen-induced arthritis in mice and rats by targeting extracellular high mobility group box chromosomal protein 1 activity." Arthritis Rheum 48(7): 2052-2058.
Kolb, M., P. J. Margetts, et al. (2001). "Transient expression of IL-1beta induces acute lung injury and chronic repair leading to pulmonary fibrosis." J Clin Invest 107(12): 1529-1536.
Kohler, G. and C. Milstein (1975). "Continuous cultures of fused cells secreting antibody of predefined specificity." Nature 256(5517): 495-497.
Kono, H., C. J. Chen, et al. (2010). "Uric acid promotes an acute inflammatory response to sterile cell death in mice." J Clin Invest 120(6): 1939-1949.
Kono, H. and K. L. Rock (2008). "How dying cells alert the immune system to danger." Nat Rev Immunol 8(4): 279-289.
Kox, W. J., T. Volk, et al. (2000). "Immunomodulatory therapies in sepsis." Intensive Care Med 26 Suppl 1: S124-128.
Krieg, A. M. (2002). "CpG motifs in bacterial DNA and their immune effects." Annu Rev Immunol 20: 709-760.
Kroemer, G., L. Galluzzi, et al. (2009). "Classification of cell death: recommendations of the Nomenclature Committee on Cell Death 2009." Cell Death Differ 16(1): 3-11.
Krummen, L. A., T. K. Woodruff, et al. (1993). "Identification and characterization of binding proteins for inhibin and activin in human serum and follicular fluids." Endocrinology 132(1): 431-443.
Labiris, N. R. and M. B. Dolovich (2003). "Pulmonary drug delivery. Part II: the role of inhalant delivery devices and drug formulations in therapeutic effectiveness of aerosolized medications." Br J Clin Pharmacol 56(6): 600-612.
Langer et al. (1981). J. Biomed. Mater. Res., 15:167-277.
Langer et al. (1982). Chem. Tech., 12:98-105.
Lee, C. G., S. J. Cho, et al. (2004). "Early growth response gene 1-mediated apoptosis is essential for transforming growth factor beta1-induced pulmonary fibrosis." J Exp Med 200(3): 377-389.
Levi, M. and H. Ten Cate (1999). "Disseminated intravascular coagulation." N Engl J Med 341(8): 586-592.
Liliensiek, B., M. A. Weigand, et al. (2004). "Receptor for advanced glycation end products (RAGE) regulates sepsis but not the adaptive immune response." J Clin Invest 113(11): 1641-1650.
Ling, N., S. Y. Ying, et al. (1986). "Pituitary FSH is released by a heterodimer of the beta-subunits from the two forms of inhibin." Nature 321(6072): 779-782.
Livak, K. J. and T. D. Schmittgen (2001). "Analysis of relative gene expression data using real-time quantitative PCR and the 2(-Delta Delta C(T)) Method." Methods 25(4): 402-408.
Lundblad, L. K., J. Thompson-Figueroa, et al. (2005). "Tumor necrosis factor-alpha overexpression in lung disease: a single cause behind a complex phenotype." Am J Respir Crit Care Med 171(12): 1363-1370.
Maher et al. (1991). Antisense Res. and Dev. 1:227
Manoharan, M., G. B. Inamati, et al. (2002). "Improving antisense oligonucleotide binding to human serum albumin: dramatic effect of ibuprofen conjugation." Chembiochem 3(12): 1257-1260.
Marc, M. M., P. Korosec, et al. (2004). "Complement factors c3a, c4a, and c5a in chronic obstructive pulmonary disease and asthma." Am J Respir Cell Mol Biol 31(2): 216-219.
Marc, M. M., S. S. Kristan, et al. (2010). "Complement factor C5a in acute exacerbation of Chronic Obstructive Pulmonary Disease." Scand J Immunol 71(5): 386-391.
Massague, J. (1990). "The transforming growth factor-beta family." Annu Rev Cell Biol 6: 597-641.
Matsuse, T., Y. Fukuchi, et al. (1995). "Expression of immunoreactive and bioactive activin A protein in adult murine lung after bleomycin treatment." Am J Respir Cell Mol Biol 13(1): 17-24.
Matthay, M. A. and R. L. Zemans (2011). "The acute respiratory distress syndrome: pathogenesis and treatment." Annu Rev Pathol 6: 147-163.
Matuschak, G. M. and A. J. Lechner (2010). "Acute lung injury and the acute respiratory distress syndrome: pathophysiology and treatment." Mo Med 107(4): 252-258.
Matzinger, P. (2002). "The danger model: a renewed sense of self." Science 296(5566): 301-305.
Meduri, G. U. (1996). "The role of the host defence response in the progression and outcome of ARDS: pathophysiological correlations and response to glucocorticoid treatment." Eur Respir J 9(12): 2650-2670.
Meduri, G. U. and S. Kanangat (1998). "Glucocorticoid treatment of sepsis and acute respiratory distress syndrome: time for a critical reappraisal." Crit Care Med 26(4): 630-633.
Meduri, G. U., P. E. Marik, et al. (2007). "Corticosteroids in ARDS: a counterpoint." Chest 132(3): 1093-1094; author reply 1094.
Medzhitov, R. (2009). "Approaching the asymptote: 20 years later." Immunity 30(6): 766-775.
Medzhitov, R. and C. A. Janeway, Jr. (2002). "Decoding the patterns of self and nonself by the innate immune system." Science 296(5566): 298-300.
Merrifield, B. (1986). "Solid phase synthesis." Science 232(4748): 341-347.
Morimoto, K., W. J. Janssen, et al. (2006). "Lovastatin enhances clearance of apoptotic cells (efferocytosis) with implications for chronic obstructive pulmonary disease." J Immunol 176(12): 7657-7665.
Moustakas, A. and C. H. Heldin (2009). "The regulation of TGFbeta signal transduction." Development 136(22): 3699-3714.
Muller, S., L. Ronfani, et al. (2004). "Regulated expression and subcellular localization of HMGB1, a chromatin protein with a cytokine function." J Intern Med 255(3): 332-343.
Munz, B., G. Hubner, et al. (1999). "A novel role of activin in inflammation and repair." J Endocrinol 161(2): 187-193.
Nakae, H., S. Endo, et al. (1994). "Serum complement levels and severity of sepsis." Res Commun Chem Pathol Pharmacol 84(2): 189-195.
Nielsen, P. E. (2000). "Peptide nucleic acids: on the road to new gene therapeutic drugs." Pharmacol Toxicol 86(1): 3-7.
Nogee, L. M., A. E. Dunbar, 3rd, et al. (2001). "A mutation in the surfactant protein C gene associated with familial interstitial lung disease." N Engl J Med 344(8): 573-579.
Nusing, R. M. and J. Barsig (1999). "Induction of prostanoid, nitric oxide, and cytokine formation in rat bone marrow derived macrophages by activin A." Br J Pharmacol 127(4): 919-926.
Ogawa, K., M. Funaba, et al. (2006). "Activin A functions as a Th2 cytokine in the promotion of the alternative activation of macrophages." J Immunol 177(10): 6787-6794.
Ogawa, K., M. Funaba, et al. (2008). "A dual role of activin A in regulating immunoglobulin production of B cells." J Leukoc Biol 83(6): 1451-1458.
Ohga, E., T. Matsuse, et al. (2000). "Activin receptors are expressed on human lung fibroblast and activin A facilitates fibroblast-mediated collagen gel contraction." Life Sci 66(17): 1603-1613.
Ohguchi, M., K. Yamato, et al. (1998). "Activin A regulates the production of mature interleukin-1beta and interleukin-1 receptor antagonist in human monocytic cells." J Interferon Cytokine Res 18(7): 491-498.
Panopoulou, E., C. Murphy, et al. (2005). "Activin A suppresses neuroblastoma xenograft tumor growth via antimitotic and antiangiogenic mechanisms." Cancer Res 65(5): 1877-1886.
Park, J. S., F. Gamboni-Robertson, et al. (2006). "High mobility group box 1 protein interacts with multiple Toll-like receptors." Am J Physiol Cell Physiol 290(3): C917-924.
Park, J. S., D. Svetkauskaite, et al. (2004). "Involvement of toll-like receptors 2 and 4 in cellular activation by high mobility group box 1 protein." J Biol Chem 279(9): 7370-7377.
Phillips, D. J., D. M. de Kretser, et al. (2009). "Activin and related proteins in inflammation: not just interested bystanders." Cytokine Growth Factor Rev 20(2): 153-164.
Phillips, D. J., K. L. Jones, et al. (2005). "Activin A: from sometime reproductive factor to genuine cytokine." Vet Immunol Immunopathol 108(1-2): 23-27.
Phillips, D. J., K. L. Jones, et al. (2001). "Evidence for activin A and follistatin involvement in the systemic inflammatory response." Mol Cell Endocrinol 180(1-2): 155-162.
Piek, E., C. H. Heldin, et al. (1999). "Specificity, diversity, and regulation in TGF-beta superfamily signaling." FASEB J 13(15): 2105-2124.
Pillow, J. J., T. R. Korfhagen, et al. (2001). "Overexpression of TGF-alpha increases lung tissue hysteresivity in transgenic mice." J Appl Physiol 91(6): 2730-2734.
Pooga, M., T. Land, et al. (2001). "PNA oligomers as tools for specific modulation of gene expression." Biomol Eng 17(6): 183-192.
Popovic, K., M. Ek, et al. (2005). "Increased expression of the novel proinflammatory cytokine high mobility group box chromosomal protein 1 in skin lesions of patients with lupus erythematosus." Arthritis Rheum 52(11): 3639-3645.
Qin, S., H. Wang, et al. (2006). "Role of HMGB1 in apoptosis-mediated sepsis lethality." J Exp Med 203(7): 1637-1642.
Quintana, F. J. and I. R. Cohen (2005). "Heat shock proteins as endogenous adjuvants in sterile and septic inflammation." J Immunol 175(5): 2777-2782.
Rice, T. W. and G. R. Bernard (2005). "Therapeutic intervention and targets for sepsis." Annu Rev Med 56: 225-248.
Riedemann, N. C., R. F. Guo, et al. (2004). "Regulatory role of C5a on macrophage migration inhibitory factor release from neutrophils." J Immunol 173(2): 1355-1359.
Risbridger, G. P., J. F. Schmitt, et al. (2001). "Activins and inhibins in endocrine and other tumors." Endocr Rev 22(6): 836-858.
Ritis, K., M. Doumas, et al. (2006). "A novel C5a receptor-tissue factor cross-talk in neutroplils links innate immunity to coagulation pathways." J Immunol 177(7): 4794-4802.
Rittirsch, D., M. A. Flierl, et al. (2008). "Functional roles for C5a receptors in sepsis." Nat Med 14(5): 551-557.
Rittirsch, D., M. A. Flierl, et al. (2008). "Harmful molecular mechanisms in sepsis." Nat Rev Immunol 8(10): 776-787.
Robson, N. C., H. Wei, et al. (2009). "Activin-A attenuates several human natural killer cell functions." Blood 113(14): 3218-3225.
Rosendahl, A., D. Checchin, et al. (2001). "Activation of the TGF-beta/activin-Smad2 pathway during allergic airway inflammation." Am J Respir Cell Mol Biol 25(1): 60-68.
Russell, J. A. (2006). "Management of sepsis." N Engl J Med 355(16): 1699-1713.
Scaffidi, P., T. Misteli, et al. (2002). "Release of chromatin protein HMGB1 by necrotic cells triggers inflammation." Nature 418(6894): 191-195.
Schnittgen, T. D. and K. J. Livak (2008). "Analyzing real-time PCR data by the comparative C(T) method." Nat Protoc 3(6): 1101-1108.
Schneyer, A., A. Schoen, et al. (2003). "Differential binding and neutralization of activins A and B by follistatin and follistatin like-3 (FSTL-3/FSRP/FLRG)." Endocrinology 144(5): 1671-1674.
Schneyer, A., Y. Sidis, et al. (2004). "Differential actions of follistatin and follistatin-like 3." Mol Cell Endocrinol 225(1-2): 25-28.
Schneyer, A., D. Tortoriello, et al. (2001). "Follistatin-related protein (FSRP): a new member of the follistatin gene family." Mol Cell Endocrinol 180(1-2): 33-38.
Schreiber, H., D. Rittirsch, et al. (2006). "Complement activation during sepsis in humans." Adv Exp Med Biol 586: 217-226.
Schultz, M. J., J. Millo, et al. (2004). "Local activation of coagulation and inhibition of fibrinolysis in the lung during ventilator associated pneumonia." Thorax 59(2): 130-135.
Segerer, S. E., N. Muller, et al. (2008). "The glycoprotein-hormones activin A and inhibin A interfere with dendritic cell maturation." Reprod Biol Endocrinol 6: 17.
Semino, C., J. Ceccarelli, et al. (2007). "The maturation potential of NK cell clones toward autologous dendritic cells correlates with HMGB1 secretion." J Leukoc Biol 81(1): 92-99.
Semitekolou, M., T. Alissafi, et al. (2009). "Activin-A induces regulatory T cells that suppress T helper cell immune responses and protect from allergic airway disease." J Exp Med 206(8): 1769-1785.
Seong, S. Y. and P. Matzinger (2004). "Hydrophobicity: an ancient damage-associated molecular pattern that initiates innate immune responses." Nat Rev Immunol 4(6): 469-478.
Sidis, Y., A. L. Schneyer, et al. (2005). "Heparin and activin-binding determinants in follistatin and FSTL3." Endocrinology 146(1): 130-136.
Sidis, Y., D. V. Tortoriello, et al. (2002). "Follistatin-related protein and follistatin differentially neutralize endogenous vs. exogenous activin." Endocrinology 143(5): 1613-1624.
Sidman, K. R., W. D. Steber, et al. (1983). "Controlled release of macromolecules and pharmaceuticals from synthetic polypeptides based on glutamic acid." Biopolymers 22(1): 547-556.
Silasi-Mansat, R., H. Zhu, et al. (2010). "Complement inhibition decreases the procoagulant response and confers organ protection in a baboon model of Escherichia coli sepsis." Blood 116(6): 1002-1010.
Silva, M. T., A. do Vale, et al. (2008). "Secondary necrosis in multicellular animals: an outcome of apoptosis with pathogenic implications." Apoptosis 13(4): 463-482.
Sime, P. J., Z. Xing, et al. (1997). "Transient gene transfer and expression in the lung." Chest 111(6 Suppl): 89S-94S.
Sime, P. J., Z. Xing, et al. (1997). "Adenovector-mediated gene transfer of active transforming growth factor-beta1 induces prolonged severe fibrosis in rat lung." J Clin Invest 100(4): 768-776.
Sitia, G., M. Iannacone, et al. (2007). "Treatment with HMGB1 inhibitors diminishes CTL-induced liver disease in HBV transgenic mice." J Leukoc Biol 81(1): 100-107.
Spragg, R. G., N. Gilliard, et al. (1994). "Acute effects of a single dose of porcine surfactant on patients with the adult respiratory distress syndrome." Chest 105(1): 195-202.
Spragg, R. G., J. F. Lewis, et al. (2004). "Effect of recombinant surfactant protein C-based surfactant on the acute respiratory distress syndrome." N Engl J Med 351(9): 884-892.
Spragg, R. G., J. F. Lewis, et al. (2003). "Treatment of acute respiratory distress syndrome with recombinant surfactant protein C surfactant." Am J Respir Crit Care Med 167(11): 1562-1566.
Stouthard, J. M., M. Levi, et al. (1996). "Interleukin-6 stimulates coagulation, not fibrinolysis, in humans." Thromb Haemost 76(5): 738-742.
Sulyok, S., M. Wankell, et al. (2004). "Activin: an important regulator of wound repair, fibrosis, and neuroprotection." Mol Cell Endocrinol 225(1-2): 127-132.
Sumitomo, S., S. Inouye, et al. (1995). "The heparin binding site of follistatin is involved in its interaction with activin." Biochem Biophys Res Commun 208(1): 1-9.
Taguchi, A., D. C. Blood, et al. (2000). "Blockade of RAGE-amphoterin signalling suppresses tumour growth and metastases." Nature 405(6784): 354-360.
Tam et al. (1983). J. Am. Chem. Soc., 105:6442-6455.
Taniguchi, N., K. Kawahara, et al. (2003). "High mobility group box chromosomal protein 1 plays a role in the pathogenesis of rheumatoid arthritis as a novel cytokine." Arthritis Rheum 48(4): 971-981.
Thomsen, G., T. Woolf, et al. (1990). "Activins are expressed early in Xenopus embryogenesis and can induce axial mesoderm and anterior structure." Cell 63(3): 485-493.
Thorp, E. and I. Tabas (2009). "Mechanisms and consequences of efferocytosis in advanced atherosclerosis." J Leukoc Biol 86(5): 1089-1095.
Tomioka, S., J. H. Bates, et al. (2002). "Airway and tissue mechanics in a murine model of asthma: alveolar capsule vs. forced oscillations." J Appl Physiol 93(1): 263-270.
Tortoriello, D. V., Y. Sidis, et al. (2001). "Human follistatin-related protein: a structural homologue of follistatin with nuclear localization." Endocrinology 142(8): 3426-3434.
Tsushima, K., L. S. King, et al. (2009). "Acute lung injury review." Intern Med 48(9): 621-630.
Vale, W., J. Rivier, et al. (1986). "Purification and characterization of an FSH releasing protein from porcine ovarian follicular fluid." Nature 321(6072): 776-779.
Vale, W., E. Wiater, et al. (2004). "Activins and inhibins and their signaling." Ann N Y Acad Sci 1038: 142-147.
Walmrath, D., A. Gunther, et al. (1996). "Bronchoscopic surfactant administration in patients with severe adult respiratory distress syndrome and sepsis." Am J Respir Crit Care Med 154(1): 57-62.
Wang, H., O. Bloom, et al. (1999). "HMG-1 as a late mediator of endotoxin lethality in mice." Science 285(5425): 248-251.
Wang, H., S. Zhu, et al. (2008). "Therapeutic potential of HMGB1-targeting agents in sepsis." Expert Rev Mol Med 10: e32.
Wang, Q., H. T. Keutmann, et al. (2000). "Analysis of human follistatin structure: identification of two discontinuous N-terminal sequences coding for activin A binding and structural consequences of activin binding to native proteins." Endocrinology 141(9): 3183-3193.
Wang, S. Y., G. X. Tai, et al. (2008). "Inhibitory effect of activin A on activation of lipopolysaccharide-stimulated mouse macrophage RAW264.7 cells." Cytokine 42(1): 85-91.
Ware, L. B. (2006). "Pathophysiology of acute lung injury and the acute respiratory distress syndrome." Semin Respir Crit Care Med 27(4): 337-349.
Weintraub (1990). Scientific American; 262:40.
Weisman, S., D. Hirsch-Lerner, et al. (2004). "Nanostructure of cationic lipid-oligonucleotide complexes." Biophys J 87(1): 609-614.
Welt, C., Y. Sidis, et al. (2002). "Activins, inhibins, and follistatins: from endocrinology to signaling. A paradigm for the new millennium." Exp Biol Med (Maywood) 227(9): 724-752.
Werner, S. and C. Alzheimer (2006). "Roles of activin in tissue repair, fibrosis, and inflammatory disease." Cytokine Growth Factor Rev 17(3): 157-171.
Wiswell, T. E., R. M. Smith, et al. (1999). "Bronchopulmonary segmental lavage with Surfaxin (KL(4)-surfactant) for acute respiratory distress syndrome." Am J Respir Crit Care Med 160(4): 1188-1195.
Woodruff, T. K. and J. P. Mather (1995). "Inhibin, activin and the female reproductive axis." Annu Rev Physiol 57: 219-244.
Wouters, E. F., K. H. Groenewegen, et al. (2007). "Systemic inflammation in chronic obstructive pulmonary disease: the role of exacerbations." Proc Am Thorac Soc 4(8): 626-634.
Wynn, T. A. (2007). "Common and unique mechanisms regulate fibrosis in various fibroproliferative diseases." J Clin Invest 117(3): 524-529.
Xing, Z., Y. Ohkawara, et al. (1997). "Adenoviral vector-mediated interleukin-10 expression in vivo: intramuscular gene transfer inhibits cytokine responses in endotoxemia." Gene Ther 4(2): 140-149.
Yang, H., M. Ochani, et al. (2004). "Reversing established sepsis with antagonists of endogenous high-mobility group box 1." Proc Natl Acad Sci U S A 101(1): 296-301.
Yoo, H. and R. L. Juliano (2000). "Enhanced delivery of antisense oligonucleotides with fluorophore-conjugated PAMAM dendrimers." Nucleic Acids Res 28(21): 4225-4231.
Yoshinaga, K., K. Mimori, et al. (2008). "Activin A enhances MMP-7 activity via the transcription factor AP-1 in an esophageal squamous cell carcinoma cell line." Int J Oncol 33(3): 453-459.
Yu, J. and K. E. Dolter (1997). "Production of activin A and its roles in inflammation and hematopoiesis." Cytokines Cell Mol Ther 3(3): 169-177.
Zosky, G. R., C. von Garnier, et al. (2004). "The pattern of methacholine responsiveness in mice is dependent on antigen challenge dose." Respir Res 5: 15.

SEQUENCE LISTING

<160> NUMBER OF SEQ ID NOS: 62

<210> SEQ ID NO: 1
<211> LENGTH: 20
<212> TYPE: DNA
<213> ORGANISM: Artificial Sequence
<220> FEATURE:
<223> OTHER INFORMATION: oligonucleotide

<400> SEQUENCE: 1

agcagacctc ggagatcatc 20

<210> SEQ ID NO: 2
<211> LENGTH: 21
<212> TYPE: DNA
<213> ORGANISM: Artificial Sequence
<220> FEATURE:
<223> OTHER INFORMATION: oligonucleotide

<400> SEQUENCE: 2

ttggggactt ttaggaagag c 21

<210> SEQ ID NO: 3
<211> LENGTH: 22
<212> TYPE: DNA
<213> ORGANISM: Artificial Sequence
<220> FEATURE:
<223> OTHER INFORMATION: oligonucleotide

<400> SEQUENCE: 3

accactacct gagcacccag tc 22

<210> SEQ ID NO: 4
<211> LENGTH: 20
<212> TYPE: DNA
<213> ORGANISM: Artificial Sequence
<220> FEATURE:
<223> OTHER INFORMATION: oligonucleotide

<400> SEQUENCE: 4

gtccatgccg agagtgatcc 20

<210> SEQ ID NO: 5
<211> LENGTH: 22
<212> TYPE: DNA
<213> ORGANISM: Artificial Sequence
<220> FEATURE:
<223> OTHER INFORMATION: oligonucleotide

<400> SEQUENCE: 5

tgacgtcact ggagttgtac gg 22

<210> SEQ ID NO: 6
<211> LENGTH: 21
<212> TYPE: DNA
<213> ORGANISM: Artificial Sequence
<220> FEATURE:
<223> OTHER INFORMATION: oligonucleotide

<400> SEQUENCE: 6

ggttcatgtc atggatggtg c 21

<210> SEQ ID NO: 7
<211> LENGTH: 25
<212> TYPE: DNA
<213> ORGANISM: Artificial Sequence
<220> FEATURE:
<223> OTHER INFORMATION: oligonucleotide

<400> SEQUENCE: 7

caaccaacaa gtgatattct ccatg 25

<210> SEQ ID NO: 8
<211> LENGTH: 20
<212> TYPE: DNA
<213> ORGANISM: Artificial Sequence
<220> FEATURE:
<223> OTHER INFORMATION: oligonucleotide

<400> SEQUENCE: 8

gatccacact ctccagctgc 20

<210> SEQ ID NO: 9
<211> LENGTH: 19
<212> TYPE: DNA
<213> ORGANISM: Artificial Sequence
<220> FEATURE:
<223> OTHER INFORMATION: oligonucleotide

<400> SEQUENCE: 9

acaggagaag ggacgccat 19

<210> SEQ ID NO: 10
<211> LENGTH: 21
<212> TYPE: DNA
<213> ORGANISM: Artificial Sequence
<220> FEATURE:
<223> OTHER INFORMATION: oligonucleotide

<400> SEQUENCE: 10

gaagccctac agacgagctc a                                    21


<210> SEQ ID NO: 11
<211> LENGTH: 23
<212> TYPE: DNA
<213> ORGANISM: Artificial Sequence
<220> FEATURE:
<223> OTHER INFORMATION: oligonucleotide

<400> SEQUENCE: 11

agcacagtgg tgaaagagac ctt                                  23


<210> SEQ ID NO: 12
<211> LENGTH: 22
<212> TYPE: DNA
<213> ORGANISM: Artificial Sequence
<220> FEATURE:
<223> OTHER INFORMATION: oligonucleotide

<400> SEQUENCE: 12

ccaatgcat agctggtgat tt                                    22


<210> SEQ ID NO: 13
<211> LENGTH: 23
<212> TYPE: DNA
<213> ORGANISM: Artificial Sequence
<220> FEATURE:
<223> OTHER INFORMATION: oligonucleotide

<400> SEQUENCE: 13

gaggatacca ctcccaacag acc                                  23


<210> SEQ ID NO: 14
<211> LENGTH: 24
<212> TYPE: DNA
<213> ORGANISM: Artificial Sequence
<220> FEATURE:
<223> OTHER INFORMATION: oligonucleotide

<400> SEQUENCE: 14

aagtgcatca tcgttgttca taca                                 24

<210> SEQ ID NO: 15
<211> LENGTH: 23
<212> TYPE: DNA
<213> ORGANISM: Artificial Sequence
<220> FEATURE:
<223> OTHER INFORMATION: oligonucleotide

<400> SEQUENCE: 15

attgtccaaa agatgctaaa agg 23

<210> SEQ ID NO: 16
<211> LENGTH: 21
<212> TYPE: DNA
<213> ORGANISM: Artificial Sequence
<220> FEATURE:
<223> OTHER INFORMATION: oligonucleotide

<400> SEQUENCE: 16

tgtatagtgt tgtcagaagc c 21

<210> SEQ ID NO: 17
<211> LENGTH: 20
<212> TYPE: DNA
<213> ORGANISM: Artificial Sequence
<220> FEATURE:
<223> OTHER INFORMATION: oligonucleotide

<400> SEQUENCE: 17

ggttgccaag ccttatcgga 20

<210> SEQ ID NO: 18
<211> LENGTH: 20
<212> TYPE: DNA
<213> ORGANISM: Artificial Sequence
<220> FEATURE:
<223> OTHER INFORMATION: oligonucleotide

<400> SEQUENCE: 18

acctgctcca ctgccttgct 20

<210> SEQ ID NO: 19
<211> LENGTH: 20
<212> TYPE: DNA
<213> ORGANISM: Artificial Sequence
<220> FEATURE:
<223> OTHER INFORMATION: oligonucleotide

<400> SEQUENCE: 19

agaccagact cccctgtgca 20

<210> SEQ ID NO: 20
<211> LENGTH: 21
<212> TYPE: DNA
<213> ORGANISM: Artificial Sequence
<220> FEATURE:
<223> OTHER INFORMATION: oligonucleotide

<400> SEQUENCE: 20

tgggtcctgt agatggcatt g                                                21

<210> SEQ ID NO: 21
<211> LENGTH: 19
<212> TYPE: DNA
<213> ORGANISM: Artificial Sequence
<220> FEATURE:
<223> OTHER INFORMATION: oligonucleotide

<400> SEQUENCE: 21

gctccagaag gcccctcaga                                                  19

<210> SEQ ID NO: 22
<211> LENGTH: 19
<212> TYPE: DNA
<213> ORGANISM: Artificial Sequence
<220> FEATURE:
<223> OTHER INFORMATION: oligonucleotide

<400> SEQUENCE: 22

agctttccct ccgcattga                                                   19

<210> SEQ ID NO: 23
<211> LENGTH: 25
<212> TYPE: DNA
<213> ORGANISM: Artificial Sequence
<220> FEATURE:
<223> OTHER INFORMATION: oligonucleotide

<400> SEQUENCE: 23

catcttctca aaattcgagt gacaa                                            25

<210> SEQ ID NO: 24
<211> LENGTH: 23
<212> TYPE: DNA
<213> ORGANISM: Artificial Sequence
<220> FEATURE:
<223> OTHER INFORMATION: oligonucleotide

<400> SEQUENCE: 24

tgggagtaga caaggtacaa ccc                                              23

<210> SEQ ID NO: 25
<211> LENGTH: 24
<212> TYPE: DNA
<213> ORGANISM: Artificial Sequence
<220> FEATURE:
<223> OTHER INFORMATION: oligonucleotide

<400> SEQUENCE: 25

tcaagtggca tagatgtgga agaa 24

<210> SEQ ID NO: 26
<211> LENGTH: 21
<212> TYPE: DNA
<213> ORGANISM: Artificial Sequence
<220> FEATURE:
<223> OTHER INFORMATION: oligonucleotide

<400> SEQUENCE: 26

tggctctgca ggattttcat g 21

<210> SEQ ID NO: 27
<211> LENGTH: 22
<212> TYPE: DNA
<213> ORGANISM: Artificial Sequence
<220> FEATURE:
<223> OTHER INFORMATION: oligonucleotide

<400> SEQUENCE: 27

cgacaatact ctcttcaagt gg 22

<210> SEQ ID NO: 28
<211> LENGTH: 24
<212> TYPE: DNA
<213> ORGANISM: Artificial Sequence
<220> FEATURE:
<223> OTHER INFORMATION: oligonucleotide

<400> SEQUENCE: 28

gggtttattc ttcttgttca ttcg 24

<210> SEQ ID NO: 29
<211> LENGTH: 21
<212> TYPE: DNA
<213> ORGANISM: Artificial Sequence
<220> FEATURE:
<223> OTHER INFORMATION: oligonucleotide

<400> SEQUENCE: 29

cactgacacc atagacattg c 21

<210> SEQ ID NO: 30
<211> LENGTH: 21
<212> TYPE: DNA
<213> ORGANISM: Artificial Sequence
<220> FEATURE:
<223> OTHER INFORMATION: oligonucleotide

<400> SEQUENCE: 30

gattgtctcg tcaaggactt c                                                21

<210> SEQ ID NO: 31
<211> LENGTH: 20
<212> TYPE: DNA
<213> ORGANISM: Artificial Sequence
<220> FEATURE:
<223> OTHER INFORMATION: oligonucleotide

<400> SEQUENCE: 31

gctccactgg catcgttgtg                                                  20

<210> SEQ ID NO: 32
<211> LENGTH: 21
<212> TYPE: DNA
<213> ORGANISM: Artificial Sequence
<220> FEATURE:
<223> OTHER INFORMATION: oligonucleotide

<400> SEQUENCE: 32

gcgaaagcct caagactagg g                                                21

<210> SEQ ID NO: 33
<211> LENGTH: 18
<212> TYPE: DNA
<213> ORGANISM: Artificial Sequence
<220> FEATURE:
<223> OTHER INFORMATION: oligonucleotide

<400> SEQUENCE: 33

ccttgtcctc cgatgttc                                                    18

<210> SEQ ID NO: 34
<211> LENGTH: 18
<212> TYPE: DNA
<213> ORGANISM: Artificial Sequence
<220> FEATURE:
<223> OTHER INFORMATION: oligonucleotide

<400> SEQUENCE: 34

cctgttcact ggtgttcc                                                    18

<210> SEQ ID NO: 35
<211> LENGTH: 20
<212> TYPE: DNA
<213> ORGANISM: Artificial Sequence
<220> FEATURE:
<223> OTHER INFORMATION: oligonucleotide

<400> SEQUENCE: 35

caaacaatgg gagtcctcag 20

<210> SEQ ID NO: 36
<211> LENGTH: 24
<212> TYPE: DNA
<213> ORGANISM: Artificial Sequence
<220> FEATURE:
<223> OTHER INFORMATION: oligonucleotide

<400> SEQUENCE: 36

cacatctctc taatggtatc aaag 24

<210> SEQ ID NO: 37
<211> LENGTH: 19
<212> TYPE: DNA
<213> ORGANISM: Artificial Sequence
<220> FEATURE:
<223> OTHER INFORMATION: oligonucleotide

<400> SEQUENCE: 37

aggaatcaaa ggtgaaagc 19

<210> SEQ ID NO: 38
<211> LENGTH: 18
<212> TYPE: DNA
<213> ORGANISM: Artificial Sequence
<220> FEATURE:
<223> OTHER INFORMATION: oligonucleotide

<400> SEQUENCE: 38

ctgatagtgg gagaaggc 18

<210> SEQ ID NO: 39
<211> LENGTH: 20
<212> TYPE: DNA
<213> ORGANISM: Artificial Sequence
<220> FEATURE:
<223> OTHER INFORMATION: oligonucleotide

<400> SEQUENCE: 39

cagaagaatg gaagagtcag 20

<210> SEQ ID NO: 40
<211> LENGTH: 20
<212> TYPE: DNA
<213> ORGANISM: Artificial Sequence
<220> FEATURE:
<223> OTHER INFORMATION: oligonucleotide

<400> SEQUENCE: 40

cagatatgca gggagtcacc 20

210> SEQ ID NO: 41
<211> LENGTH: 20
<212> TYPE: DNA
<213> ORGANISM: Artificial Sequence
<220> FEATURE:
<223> OTHER INFORMATION: oligonucleotide

<400> SEQUENCE: 41

gagcggagag tactggatcg 20

210> SEQ ID NO: 42
<211> LENGTH: 20
<212> TYPE: DNA
<213> ORGANISM: Artificial Sequence
<220> FEATURE:
<223> OTHER INFORMATION: oligonucleotide

<400> SEQUENCE: 42

gttcgggctg atgtaccagt 20

210> SEQ ID NO: 43
<211> LENGTH: 20
<212> TYPE: DNA
<213> ORGANISM: Artificial Sequence
<220> FEATURE:
<223> OTHER INFORMATION: oligonucleotide

<400> SEQUENCE: 43

aggctgaagg aaacagcaaa 20

210> SEQ ID NO: 44
<211> LENGTH: 20
<212> TYPE: DNA
<213> ORGANISM: Artificial Sequence
<220> FEATURE:
<223> OTHER INFORMATION: oligonucleotide

<400> SEQUENCE: 44

tagtctcatt gccttgcgtg 20

210> SEQ ID NO: 45
<211> LENGTH: 18
<212> TYPE: DNA
<213> ORGANISM: Artificial Sequence
<220> FEATURE:
<223> OTHER INFORMATION: oligonucleotide

<400> SEQUENCE: 45

ctctggcatc tggcatcc 18

210> SEQ ID NO: 46
<211> LENGTH: 20
<212> TYPE: DNA
<213> ORGANISM: Artificial Sequence
<220> FEATURE:
<223> OTHER INFORMATION: oligonucleotide

<400> SEQUENCE: 46

gctggtataa ggtggtctcg 20

210> SEQ ID NO: 47
<211> LENGTH: 19
<212> TYPE: DNA
<213> ORGANISM: Artificial Sequence
<220> FEATURE:
<223> OTHER INFORMATION: oligonucleotide

<400> SEQUENCE: 47

agaaggagat ggcaagatg 19

210> SEQ ID NO: 48
<211> LENGTH: 18
<212> TYPE: DNA
<213> ORGANISM: Artificial Sequence
<220> FEATURE:
<223> OTHER INFORMATION: oligonucleotide

<400> SEQUENCE: 48

ggaggagatg tagcaagg 18

210> SEQ ID NO: 49
<211> LENGTH: 22
<212> TYPE: DNA
<213> ORGANISM: Artificial Sequence
<220> FEATURE:
<223> OTHER INFORMATION: oligonucleotide

<400> SEQUENCE: 49

ttgagaagga tggcaagtat gg 22

210> SEQ ID NO: 50
<211> LENGTH: 18
<212> TYPE: DNA
<213> ORGANISM: Artificial Sequence
<220> FEATURE:
<223> OTHER INFORMATION: oligonucleotide

<400> SEQUENCE: 50

tggaagcgga acgggaac 18

210> SEQ ID NO: 51
<211> LENGTH: 24
<212> TYPE: DNA
<213> ORGANISM: Artificial Sequence
<220> FEATURE:
<223> OTHER INFORMATION: oligonucleotide

<400> SEQUENCE: 51

tatctgtatg gtcgtggctc tag 24

210> SEQ ID NO: 52
<211> LENGTH: 18
<212> TYPE: DNA
<213> ORGANISM: Artificial Sequence
<220> FEATURE:
<223> OTHER INFORMATION: oligonucleotide

<400> SEQUENCE: 52

tgctgtcggc tgtggttc 18

<210> SEQ ID NO: 53
<211> LENGTH: 22
<212> TYPE: DNA
<213> ORGANISM: Artificial Sequence
<220> FEATURE:
<223> OTHER INFORMATION: oligonucleotide

<400> SEQUENCE: 53

accatttaca aacgccccaa ag 22

<210> SEQ ID NO: 54
<211> LENGTH: 18
<212> TYPE: DNA
<213> ORGANISM: Artificial Sequence
<220> FEATURE:
<223> OTHER INFORMATION: oligonucleotide

<400> SEQUENCE: 54

acttgccgca gggtgagg 18

<210> SEQ ID NO: 55
<211> LENGTH: 20
<212> TYPE: DNA
<213> ORGANISM: Artificial Sequence
<220> FEATURE:
<223> OTHER INFORMATION: oligonucleotide

<400> SEQUENCE: 55

agcctcaaac tccaaacacc 20

<210> SEQ ID NO: 56
<211> LENGTH: 18
<212> TYPE: DNA
<213> ORGANISM: Artificial Sequence
<220> FEATURE:
<223> OTHER INFORMATION: oligonucleotide

<400> SEQUENCE: 56

caacagggat ttgaccac 18

<210> SEQ ID NO: 57
<211> LENGTH: 20
<212> TYPE: DNA
<213> ORGANISM: Artificial Sequence
<220> FEATURE:
<223> OTHER INFORMATION: oligonucleotide

<400> SEQUENCE: 57

ccagtatgac tccactcacg 20

<210> SEQ ID NO: 58
<211> LENGTH: 20
<212> TYPE: DNA
<213> ORGANISM: Artificial Sequence
<220> FEATURE:
<223> OTHER INFORMATION: oligonucleotide

<400> SEQUENCE: 58

ctcctggaag atggtgatgg 20

<210> SEQ ID NO: 59
<211> LENGTH: 26
<212> TYPE: DNA
<213> ORGANISM: Artificial Sequence
<220> FEATURE:
<223> OTHER INFORMATION: oligonucleotide

<400> SEQUENCE: 59

ggactagtaa catgacggcg ccctgg 26

210> SEQ ID NO: 60
<211> LENGTH: 24
<212> TYPE: DNA
<213> ORGANISM: Artificial Sequence
<220> FEATURE:
<223> OTHER INFORMATION: oligonucleotide

<400> SEQUENCE: 60

ccagatcgcg gtgggggctg tcgg 24

<210> SEQ ID NO: 61
<211> LENGTH: 42
<212> TYPE: DNA
<213> ORGANISM: Artificial Sequence
<220> FEATURE:
<223> OTHER INFORMATION: oligonucleotide

<400> SEQUENCE: 61

gcagatctaa tcgaaggtcg tggtgatccc aaatcttgtg ac 42

<210> SEQ ID NO: 62
<211> LENGTH: 48
<212> TYPE: DNA
<213> ORGANISM: Artificial Sequence
<220> FEATURE:
<223> OTHER INFORMATION: oligonucleotide

<400> SEQUENCE: 62

tccctgtctc cgggtaaaca ccatcaccat caccattgag cgccgctt 48

## Claims

1. Use of an activin neutraliser or a mixture thereof for the manufacture of a medicament for the prophylaxis and / or therapy of diseases associated with aberrant Host Defence Response activation in a subject.

2. Use of the activin neutraliser as in claim **1,** wherein said medicament is administered via local and / or systemic circulation.

3. Use of the activin neutraliser as in claim **1** or **2,** wherein said activin is selected from the group consisting of: activin-A, activin-B and activin-AB.

4. Use of the activin neutraliser as in claim **1, 2 or 3,** wherein said diseases are selected from the group comprising: (a) acute injury of tissues inflicted by infection, toxic substances or trauma, wound healing pursuant to surgery, severe bums or other tissue injury, meningitis, appendicitis, renal tubular necrosis, traumatic brain injury and sepsis; (b) autoimmune diseases including, but not limited to, Rheumatoid arthritis, multiple sclerosis, inflammatory bowel disease, vasculitis, anti-phospholipid Syndrome, scleroderma, Systemic lupus erythematosous and osteoarthritis; and (c) respiratory diseases including, but not limited to organising peumonia, non-specific interstitial pneumonia, sarcoidosis, bronchiolitis obliterans, pulmonary hypertension, pneumonia, ALI/ARDS, COPD, acute responses against infectious agents such as pathogenic viruses including, but not limited to, influenza A viruses H5N1 and H1N1, coronaviruses SARS, and human rhinoviruses C and D.

5. Use of the activin neutraliser as in claim **1, 2, 3 or 4,** wherein said activin neutraliser is co-administered with at least one additional therapeutic agent.

6. Use of the activin neutraliser as in claim **4,** wherein said diseases are selected from the group consisting of: ALI/ARDS, sepsis and COPD.

7. Use of the activin neutraliser as in claim **1, 2, 3, 4, 5 or 6,** wherein said activin neutraliser is a soluble ActRII polypeptide, or a fragment(s) or analogue thereof.

8. Use of the activin neutraliser as in claim 7, wherein said soluble ActRII polypeptide, or a fragment(s) or analogue is administered *via* injection or inhalation routes.

9. Use of the activin neutraliser as in claim **7** or **8,** wherein said soluble ActRII polypeptide fragment is ActRIIB-Fc.

10. Use of the activin neutraliser as in claim **1, 2, 3, 4,** or **5,** wherein said activin neutraliser is a low molecular weight organic compound, or a pharmaceutically acceptable salt thereof.

11. Use of the activin neutraliser as in claim **10,** wherein said low molecular weight organic compound, or a pharmaceutically acceptable salt thereof, is administered *via* oral, injection or inhalation routes.

12. Use of the activin neutraliser as in claim **5,** wherein said at least one additional therapeutic agent is an anti-inflammatory therapeutic agent.

13. Use of the activin neutraliser as in claim **12,** wherein said anti-inflammatory therapeutic agent is a corticosteroid.

14. Use of the activin neutraliser as in claim **5,** wherein said at least one additional therapeutic agent is a neutraliser of pro-inflammatory and pro-fibrotic cytokines.

15. Use of the activin neutraliser as in claim **14,** wherein said neutraliser of pro-inflammatory and pro-fibrotic cytokines is selected from the group comprising: IL-4, IL-5, IL-6, TNF-α, IL-17 and MCP-1.

16. Use of the activin neutraliser as in claim **5,** wherein said at least one additional therapeutic agent is a neutraliser of DAMPs.

17. Use of the activin neutraliser as in claim **16,** wherein said neutraliser of DAMPs is selected from the group comprising: HMGB-1 and TREM-1.

18. Use of the activin neutraliser as in claim **5,** wherein said at least one additional therapeutic agent is a neutraliser of the coagulation system.

19. Use of the activin neutraliser as in claim **18,** wherein said neutraliser of the coagulation system is selected from the group comprising: activated protein C (APC), active site inhibited factor VIIa (ASIS), tissue factor pathway inhibitor (TFPI), anti-thrombin (AT), low-molecular weight heparin, vitamin K antagonists and acetylsalicylic acid.

20. Use of the activin neutraliser as in claim **5,** wherein said at least one additional therapeutic agent is a neutraliser of the complement system.

21. Use of the activin neutraliser as in claim **20,** wherein said neutraliser of the complement system is selected from the group comprising: endogenous soluble complement inhibitors and agents which neutralise key proteins of the complement cascade reaction.

22. Use of the activin neutraliser as in claim **5,** wherein said at least one additional therapeutic agent is a surfactant replacement agent.

23. Use of the activin neutraliser as in claim **5,** wherein said at least one additional therapeutic agent is an agent targeting infectious agents.

24. Use of the activin neutraliser as in claim **23,** wherein said agent targeting infectious agents is selected from the group comprising: an antibiotic agent, an anti-fungal agent and an anti-viral agent.
